# EUROPEAN PATENT APPLICATION

(11) **EP 4 488 385 A2**
(43) Date of publication of application: **08.01.2025**
(21) Application number: 24200814.2
(22) Date of filing: 25.01.2022
(51) Int. Cl.: C12Q 1/682

(54) **NUCLEIC ACID ANALOG PROBES FOR IN SITU ANALYSIS**

(30) Priority: 26.01.2021 US 202163141908 P
(62) Divisional of application: 22703809.8
(71) Applicant: 10x Genomics, Inc., Pleasanton, CA 94588-3260 (US)
(72) Inventor: COSTA, Justin, Pleasanton, CA 94588-3260 (US)
(74) Representative: J A Kemp LLP

(57) **Abstract**

The present disclosure relates in some aspects to proteins and nucleic acids for probing analytes in a biological sample. In some embodiments, disclosed herein is a method in which detection probes (e.g., fluorescently labeled detection probes) may comprise a sequence of nucleobases on a synthetic backbone, the detection probes therefore generating a greater signal than reference detection probes having the same sequence of nucleobases on a sugar-phosphate backbone.

## Description

### CROSS REFERENCE TO RELATED APPLICATION

This application claims priority from U.S. Provisional Application No. 63/141,908 filed January 26, 2021, entitled "NUCLEIC ACID ANALOG PROBES FOR IN SITU ANALYSIS," the contents of which are incorporated herein by reference in their entireties for all purposes.

### FIELD

The present disclosure relates in some aspects to methods for *in situ* analysis of nucleic acid molecules as analytes or reporters for other analytes in a biological sample.

### BACKGROUND

Methods are available for analyzing nucleic acids present in a biological sample, such as a cell or a tissue. Current methods for analyzing nucleic acids present in a biological sample, for example for *in situ* analysis, can have low sensitivity and specificity, limited plexity, biased, time-consuming, labor-intensive, and/or error-prone. Improved methods for analyzing nucleic acids present in a biological sample are needed. Provided herein are methods, polynucleotides, sets of polynucleotides, compositions, and kits that meet such and other needs.

### BRIEF SUMMARY

In some aspects, provided herein is a method of a method of analyzing a biological sample, comprising: binding a plurality of detection probes directly or indirectly to a DNA concatemer in the biological sample, wherein a detection probe of the plurality of detection probes comprises a sequence of nucleobases on a synthetic backbone, and the detection probe directly or indirectly binds to a target sequence in the DNA concatemer, and detecting signals from said plurality of bound detection probes, where the signal generated is greater than a plurality of reference detection probes having the same sequence of nucleobases on a sugar-phosphate backbone. In some embodiments, at least some of the plurality of detection probes comprise a sequence of nucleobases on a synthetic backbone. In some embodiments, each of the plurality of detection probes comprises a sequence of nucleobases on a synthetic backbone. In some embodiments, all of the plurality of detection probes comprise a sequence of nucleobases on a synthetic backbone. In some embodiments, at least some, each, or all of the plurality of detection probes are morpholino-based probes.

In some embodiments, the detection probe can be less electrostatically negative than the reference detection probe.

In any of the preceding embodiments, the detection probe can be directly or indirectly coupled to a detectable label. In some instances, the detectable label comprises a fluorescent label. In some embodiments, the detection probe is covalently coupled (e.g., via a bond or via a linker moiety) to a detectable label, e.g., a fluorescent label. In some embodiments, the detection probe is noncovalently coupled to a detectable label, e.g., a fluorescent label, for instance, via noncovalent interaction between a binding pair (e.g., biotin coupled to the detection probe and streptavidin coupled to the detectable label, or vice versa). In some embodiments, the detection probe is not directly coupled (e.g., covalently or noncovalently) to a detectable label, and the detection probe can be an intermediate probe that directly or indirectly binds to a detectably-labeled probe that is directly or indirectly coupled (e.g., covalently or noncovalently) to a detectable label. In some embodiments, the intermediate probe hybridizes to a detectably-labeled probe that is covalently coupled to a detectable label. In any of the preceding embodiments, the intermediate probe and/or the detectably-labeled probe can be a nucleic acid analog probe comprising a sequence of nucleobases on a synthetic backbone in which the sugar in the backbone of a naturally occurring nucleic acid is replaced by a morpholine (e.g., methylene morpholino) ring, and the methylenemorpholine rings are linked by phosphorodiamidate moieties. In some embodiments, a nucleic acid analog probe disclosed herein has neutral charge along the molecule, and binds to complementary sequences of target nucleic acid (e.g., DNA or RNA, such as a DNA concatemer) by Watson-Crick base pairing. In some embodiments, a nucleic acid analog probe disclosed herein has a pitch of about 3.4 angstroms between bases. In some embodiments, a nucleic acid analog probe comprises the same sequence of nucleobases on a synthetic backbone as a reference probe comprising nucleobases on a sugar-phosphate backbone.

In any of the preceding embodiments, the detection probe can directly hybridize to the target sequence. In any of the preceding embodiments, the detection probe can hybridize to an intermediate probe which directly or indirectly binds to the target sequence. In any of the preceding embodiments, the detection probe can directly hybridize to an intermediate probe that directly hybridizes to the target sequence.

In any of the preceding embodiments, the detection probe can comprise a single-stranded region and optionally a double-stranded region.

In any of the preceding embodiments, the sequence of nucleobases on the synthetic backbone can be a first probe sequence, and the detection probe can further comprise a second probe sequence comprising nucleobases on a sugar-phosphate backbone. In any of the preceding embodiments, the first probe sequence can be 5' or 3' to the second probe sequence In any of the preceding embodiments, the detection probe can comprise one or more first probe sequences and/or one or more second probe sequences. In any of the preceding embodiments, the second probe sequence can be a DNA sequence, an RNA sequence, or a combination thereof.

In any of the preceding embodiments, the detection probe can comprise nucleobases A, T, C, G, or U, or variants thereof, in any suitable order and combination.

In any of the preceding embodiments, the detection probe can be between about 5 and about 50 nucleobases in length. In some instances, the detection probe can be between about 10 and about 20, between about 20 and about 30, or between about 30 and about 40 nucleobases in length.

In any of the preceding embodiments, the detection probe can comprise phosphorodiamidate linkages in the synthetic backbone. In any of the preceding embodiments, the detection probe can comprise morpholine rings in the synthetic backbone. In any of the preceding embodiments, the detection probe can be a morpholino.

In any of the preceding embodiments, the detection probe can be uncharged or positively charged.

In any of the preceding embodiments, the DNA concatemer can comprise one or more barcode sequences, optionally wherein the target sequence can comprise a barcode sequence corresponding to an analyte in the biological sample.

In some embodiments, the DNA concatemer is a product generated *in situ.* In some embodiments, the DNA concatemer is a product generated *in situ* in a cell, cell sample, or tissue sample, such as a tissue section. In any of the preceding embodiments, the DNA concatemer can be a product (e.g., an extension product (e.g., by a DNA or RNA polymerase), a replication product, a reverse transcription product, and/or an amplification product such as a rolling circle amplification (RCA) product) of an endogenous nucleic acid molecule in the biological sample, optionally wherein the endogenous nucleic acid molecule can be a viral or cellular DNA or RNA, such as genomic DNA/RNA, mRNA, or cDNA. In any of the preceding embodiments, the DNA concatemer can be an extension product (e.g., by a DNA or RNA polymerase), a replication product, a reverse transcription product, and/or an amplification product. In some embodiments, the DNA concatemer is a product generated from rolling circle amplification (RCA). In some embodiments, the DNA concatemer is a product generated from rolling circle amplification (RCA) *in situ* in a cell, cell sample, or tissue sample, such as a tissue section. In some embodiments, the product is generated using or is associated with an endogenous nucleic acid molecule in the biological sample. In some instances, the endogenous nucleic acid molecule is a viral or cellular DNA or RNA, such as genomic DNA/RNA, mRNA, or cDNA.

In any of the preceding embodiments, the DNA concatemer can be a product (e.g., an extension product (e.g., by a DNA or RNA polymerase), a replication product, a reverse transcription product, and/or an amplification product such as a rolling circle amplification (RCA) product) of a labelling agent that can directly or indirectly bind to an analyte in the biological sample. In any of the preceding embodiments, the labelling agent can comprise a reporter oligonucleotide, optionally wherein the reporter oligonucleotide can comprise one or more barcode sequences and the DNA concatemer comprises one or a plurality of copies of the one or more barcode sequences. In some instances, the product is generated using a DNA polymerase and/or an RNA polymerase.

In any of the preceding embodiments, the DNA concatemer can be a rolling circle amplification (RCA) product of a circular or circularizable (e.g., padlock) probe or probe set that hybridizes to a DNA (e.g., a cDNA of an mRNA) or RNA molecule in the biological sample. In any of the preceding embodiments, DNA concatemers generated from a plurality of different mRNA and/or cDNA molecules can be analyzed, a barcode sequence in a particular circular or circularizable (e.g., padlock) probe or probe set can uniquely correspond to a particular mRNA or cDNA molecule, and the particular circular or circularizable (e.g., padlock) probe or probe set can further comprise an anchor sequence that is common among circular or circularizable (e.g., padlock) probes or probe sets for a subset of the plurality of different mRNA and/or cDNA molecules. In any of the preceding embodiments, the labelling agent can comprise a binding moiety that can directly or indirectly bind to a non-nucleic acid analyte in the biological sample, e.g., an analyte comprising a peptide, a protein, a carbohydrate, and/or a lipid, and the reporter oligonucleotide in the labelling agent can identify the binding moiety and/or the non-nucleic acid analyte. In any of the preceding embodiments, the binding moiety of the labelling agent can comprise an antibody or antigen binding fragment thereof that can directly or indirectly bind to a protein analyte, and the DNA concatemer can be a rolling circle amplification (RCA) product of a circular or circularizable (e.g., padlock) probe or probe set that can hybridize to a reporter oligonucleotide of the labelling agent.

In any of the preceding embodiments, the DNA concatemer can be in the form of a nanoball having a diameter of between about 0.1 µm and about 3 µm, e.g., between about 0.1 µm and about 0.5 µm, between about 0.5 µm and about 1 µm, or between about 1 µm and about 1.5 µm.

In any of the preceding embodiments, the DNA concatemer can be between about 1 and about 15 kilobases, between about 15 and about 25 kilobases, between about 25 and about 35 kilobases, between about 35 and about 45 kilobases, between about 45 and about 55 kilobases, between about 55 and about 65 kilobases, between about 65 and about 75 kilobases, or more than 75 kilobases in length, e.g., between about 45 and about 70 kilobases.

In any of the preceding embodiments, the DNA concatemer can comprise between about 10 and about 100, between about 100 and about 1,000, between about 1,000 and about 5,000, between about 5,000 and about 10,000, or more than 10,000 copies of the target sequence.

In any of the preceding embodiments, at least about 10%, at least about 25%, at least about 50%, at least about 75%, or at least about 90% of the copies of the target sequence in the DNA concatemer can be bound directly or indirectly to one or more molecules of the plurality of detection probes.

In any of the preceding embodiments, the DNA concatemer can be generated *in situ.* In any of the preceding embodiments, the DNA concatemer can be immobilized in the biological sample. In any of the preceding embodiments, the DNA concatemer can be crosslinked to one or more other molecules (e.g., a cellular molecule or an extracellular molecule) in the biological sample.

In any of the preceding embodiments, a complex comprising multiple molecules of the detection probe can be detected *in situ* in the biological sample. In any of the preceding embodiments, the method can comprise imaging the biological sample to detect the complex, e.g., using fluorescent microscopy.

In any of the preceding embodiments, a sequence (e.g., the target sequence) of the DNA concatemer molecule can be analyzed *in situ* in the biological sample. In any of the preceding embodiments, the detection probe can be for analyzing a sequence (e.g., the target sequence) of the DNA concatemer by sequential hybridization, sequencing by hybridization, sequencing by ligation, sequencing by synthesis, sequencing by binding, or a combination thereof. In any of the preceding embodiments, the sequence to be analyzed can comprise a barcode sequence, e.g., a barcode sequence corresponding to an analyte or a portion thereof or a labelling agent for an analyte or portion thereof in the biological sample

In any of the preceding embodiments, the method can further comprise removing one or more molecules of the detection probe that have not bound to the target sequence.

In any of the preceding embodiments, the detection probe of the plurality of detection probes can be a first detection probe and the method can further comprise removing molecules of the first detection probe bound to the target sequence, prior to contacting the biological sample with a plurality of second detection probes comprising a sequence of nucleobases on a synthetic backbone. In any of the preceding embodiments, the target sequence can be a first target sequence, and a second detection probe of the plurality of second detection probes can directly or indirectly bind to a second target sequence in the DNA concatemer, wherein the first and second target sequences can be the same or different.

In any of the preceding embodiments, the detection probe can be a first detection probe and can hybridize to a first intermediate probe which can hybridize to the target sequence, optionally wherein the first intermediate probe can comprise a sequence of nucleobases on a synthetic backbone. In any of the preceding embodiments, the method can further comprise removing molecules of the first detection probe and/or the first intermediate probe bound to the target sequence. In any of the preceding embodiments, the method can further comprise contacting the biological sample with a plurality of second detection probes comprising a sequence of nucleobases on a synthetic backbone and a second intermediate probe, wherein the plurality of second detection probes can hybridize to the second intermediate probe which can hybridize to the target sequence, optionally wherein the first intermediate probe can comprise a sequence of nucleobases on a synthetic backbone.

In any of the preceding embodiments, the biological sample can be a processed or cleared biological sample. In any of the preceding embodiments, the biological sample can be a tissue sample. In any of the preceding embodiments, the tissue sample can be a tissue slice between about 1 µm and about 50 µm in thickness, optionally wherein the tissue slice can be between about 5 µm and about 35 µm in thickness.

In any of the preceding embodiments, the tissue sample can be embedded in a hydrogel. In any of the preceding embodiments, the analyte or the DNA concatemer can be crosslinked to the hydrogel. In any of the preceding embodiments, the analyte or the DNA concatemer can comprise one or more functional groups for attachment to the hydrogel. In any of the preceding embodiments, the analytes can comprise biomolecules and/or products thereof from a tissue sample.

In some aspects, provided herein is a method of analyzing a biological sample, comprising: (a) contacting a biological sample with a plurality of fluorescently labeled detection probes, wherein: the biological sample comprises a rolling circle amplification (RCA) product immobilized therein, wherein the RCA product comprises multiple copies of a target sequence, a fluorescently labeled detection probe of the plurality of fluorescently labeled detection probes comprises a sequence of nucleobases on a synthetic backbone which hybridizes to the target sequence or an intermediate probe that hybridizes to the target sequence, (b) optionally removing molecules of the fluorescently labeled detection probe and/or the intermediate probe that have not hybridized to the RCA product; and (c) detecting molecules of the fluorescently labeled detection probe bound to the RCA product in the biological sample, and the signals generated by the fluorescently labeled detection probes hybridized to the RCA product is greater than a plurality of reference detection probes having the same fluorescent label and sequence of nucleobases on a sugar-phosphate backbone. In some embodiments, at least some of the plurality of fluorescently labeled detection probes comprise a sequence of nucleobases on a synthetic backbone e. In some embodiments, each of the plurality of fluorescently labeled detection probes comprises a sequence of nucleobases on a synthetic backbone. In some embodiments, all of the plurality of fluorescently labeled detection probes comprise a sequence of nucleobases on a synthetic backbone. In some embodiments, at least some, each, or all of the plurality of fluorescently labeled detection probes are morpholino-based probes.

In any of the preceding embodiments, the method can further comprise prior to step (a): contacting the biological sample with a circular or circularizable (e.g., padlock) probe or probe set that can directly or indirectly bind to an analyte in the biological sample, and generating the RCA product using the circular or circularizable probe or probe set as template.

In any of the preceding embodiments, the fluorescently labeled detection probe can be uncharged or less electrostatically negative than a reference detection probe having the same fluorescent label and sequence of nucleobases on a sugar-phosphate backbone.

In any of the preceding embodiments, the circular or circularizable probe or probe set can hybridize to a nucleic acid sequence of the analyte. In any of the preceding embodiments, the circular or circularizable probe or probe set can hybridize to a reporter oligonucleotide of a labelling agent, wherein the labelling agent can further comprise a binding moiety that can directly or indirectly bind to the analyte, wherein the reporter oligonucleotide can correspond to the binding moiety and/or the analyte.

In any of the preceding embodiments, the intermediate probe can comprise a sequence of nucleobases on a synthetic backbone which can hybridize to the target sequence, optionally wherein there can be more molecules of the intermediate probe hybridized in the interior of the RCA product than a reference intermediate probe having the same sequence of nucleobases on a sugar-phosphate backbone.

In any of the preceding embodiments, the fluorescently labeled detection probe and/or the intermediate probe can be morpholino.

In any of the preceding embodiments, the RCA product can have a diameter of less than about 1.5 µm. In some instances, the RCA product can have a diameter of between about 0.1 µm and about 0.5 µm, between about 0.5 µm and about 1 µm, or between about 1 µm and about 1.5 µm, optionally less than about 1 µm . In any of the preceding embodiments, the RCA product can have a diameter of about 100 nm, about 200 nm, about 300 nm, about 400 nm, about 500 nm, about 600 nm, about 700 nm, about 800 nm, about 900 nm, about 1,000 nm, about 1,100 nm, about 1,200 nm, about 1,300 nm, about 1,400 nm, or about 1,500 nm.

In any of the preceding embodiments, in the contacting step, the number of molecules of the fluorescently labeled detection probe can be no more than 5, no more than 4, no more than 3, no more than 2, or no more than 1.5 times the number of copies of the target sequence in the RCA product.

In any of the preceding embodiments, at least about 10%, at least about 25%, at least about 50%, at least about 75%, or at least about 90% of the copies of the target sequence in the RCA product can be bound directly or indirectly to one or more molecules of the intermediate probe and/or the fluorescently labeled detection probe.

In any of the preceding embodiments, the biological sample can be contacted with multiple molecules of the reference detection probe, and more molecules of the fluorescently labeled detection probe than the reference detection probe can bind to the RCA product in the biological sample.

In any of the preceding embodiments, the method can further comprise removing molecules of the fluorescently labeled detection probe and the intermediate probe bound to the RCA product in the biological sample.

In any of the preceding embodiments, the fluorescently labeled detection probe can be a first fluorescently labeled detection probe and the intermediate probe can be a first intermediate probe, and the method can further comprise contacting the biological sample with a second fluorescently labeled detection probe, wherein: the second fluorescently labeled detection probe can comprise a sequence of nucleobases on a synthetic backbone which hybridizes to a second intermediate probe that can hybridize to the target sequence, and molecules of the second fluorescently labeled detection probe bound to the RCA product in the biological sample can be detected. In any of the preceding embodiments, the second intermediate probe can comprise a sequence of nucleobases on a synthetic backbone which can hybridize to the target sequence, optionally wherein there can be more molecules of the second intermediate probe hybridized in the interior of the RCA product than a reference intermediate probe having the same sequence of nucleobases on a sugar-phosphate backbone.

In some aspects, provided herein is a composition, comprising: a DNA concatemer comprising multiple copies of a target sequence, a plurality of intermediate probes that hybridize to the target sequence, and a plurality of fluorescently labeled detection probes each comprising a sequence of nucleobases on a synthetic backbone, wherein the DNA concatemer, at least one molecule of the intermediate probe and at least one molecule of the fluorescently labeled detection probe form a hybridization complex.

In any of the preceding embodiments, the DNA concatemer of the composition can be a rolling circle amplification (RCA) product. In any of the preceding embodiments, the RCA product of the composition can be generated *in situ* in a biological sample, using a circular or circularizable (e.g., padlock) probe or probe set that directly or indirectly binds to an analyte in the biological sample as template.

In any of the preceding embodiments, the fluorescently labeled detection probe of the composition can be uncharged or less electrostatically negative than a reference detection probe having the same fluorescent label and sequence of nucleobases on a sugar-phosphate backbone. In any of the preceding embodiments, the intermediate probe of the composition can comprise a sequence of nucleobases on a synthetic backbone which can hybridize to the target sequence, optionally wherein the intermediate probe can be uncharged or less electrostatically negative than a reference intermediate probe having the same sequence of nucleobases on a sugar-phosphate backbone. In any of the preceding embodiments, at least a portion of the fluorescently labeled detection probe of the composition can hybridize to the intermediate probe.

In any of the preceding embodiments, the fluorescently labeled detection probe and/or the intermediate probe of the composition can be morpholino.

In any of the preceding embodiments, the DNA concatemer of the composition can have a diameter of less than about 1.5 µm. In some instances, the RCA product can have a diameter of between about 0.1 µm and about 0.5 µm, between about 0.5 µm and about 1 µm, or between about 1 µm and about 1.5 µm, optionally less than about 1 µm. In any of the preceding embodiments, the DNA concatemer can have a diameter of about 100 nm, about 200 nm, about 300 nm, about 400 nm, about 500 nm, about 600 nm, about 700 nm, about 800 nm, about 900 nm, about 1,000 nm, about 1,100 nm, about 1,200 nm, about 1,300 nm, about 1,400 nm, or about 1,500 nm.

In any of the preceding embodiments, at least about 10%, at least about 25%, at least about 50%, at least about 75%, or at least about 90% of the copies of the target sequence in the DNA concatemer of the composition can be bound directly or indirectly to one or more molecules of the intermediate probe and/or the fluorescently labeled detection probe.

In some aspects, provided herein is a kit, comprising: a fluorescently labeled detection probe comprising a sequence of nucleobases on a synthetic backbone, wherein at least a portion of the fluorescently labeled detection probe hybridizes to an intermediate probe, and the intermediate probe which comprises a sequence of nucleobases on a synthetic backbone that hybridizes to a target sequence.

In any of the preceding embodiments, the fluorescently labeled detection probe and the intermediate probe of the kit can be morpholino. In any of the preceding embodiments, the kit can further comprise a circular or circularizable (e.g., padlock) probe or probe set comprising the target sequence or a complement thereof, wherein the probe or probe set can be capable of directly or indirectly binding to an analyte in a biological sample and generating a rolling circle amplification product using the probe or probe set as template.

In any of the preceding embodiments, the binding of the plurality of detection probes can occur in the presence of a hybridization buffer. In any of the preceding embodiments, the hybridization buffer can comprise a reagent that increases the stability of A:T base pairs to approximately that of G:C pairs. In any of the preceding embodiments, the reagent can comprise ammonium ions. In any of the preceding embodiments, the reagent can be tetramethylammonium chloride.

### BRIEF DESCRIPTION OF THE DRAWINGS

**FIG. 1A** shows an example of a morpholino structure.
**FIG. 1B** shows an exemplary nucleic acid analog based on a morpholino backbone. The nucleic acid analog may comprise a detectable label for use as a detection probe, e.g., for binding to a rolling circle amplification (RCA) product or a probe directly or indirectly bound to the RCA product.
**FIG. 2A** shows limited binding capacity of exemplary standard detection probes (e.g., with a sequence of nucleobases on a sugar-phosphate backbone) to a DNA concatemer (e.g., an RCA product) due to the charge properties of the DNA concatemer. In some embodiments, the charge field and/or other barriers related to the physical chemistry of the DNA concatemer may prevent a significant number of standard detection probes from penetrating the DNA concatemer due to electrostatic repulsion.

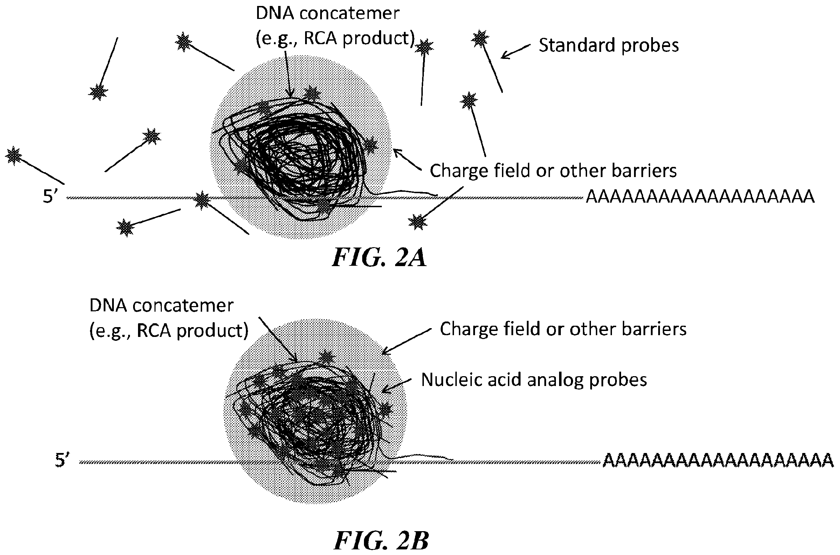

**FIG. 2B** shows an increased binding capacity of exemplary nucleic acid analog detection probes (e.g., with a sequence of nucleobases on a synthetic backbone) to a DNA concatemer (e.g., an RCA product) due to their decreased sensitivity to the charge properties of the DNA concatemer. In some embodiments, the nucleic acid analog detection probes are insensitive to properties of the DNA concatemer (e.g., electrostatic effects), allowing more nucleic acid analog detection probes to dock on and/or within the DNA concatemer, compared to standard detection probes having the same nucleobase sequences. Nucleic acid analytes with poly A sequences are shown in FIGS. 2A-2B as examples, and it should be appreciated that the nucleic acid analytes can be any nucleic acids such as genomic DNA, coding or noncoding RNA (e.g., mRNA), or cDNA.
**FIG. 3** shows exemplary probes for detecting a DNA concatemer (e.g., an RCA product), where the intermediate probe or the detection probe (e.g., a fluorescently labeled detection probe), or both can be nucleic acid analog probes. The RCA product can be produced using any suitable primary probe or probe set on any suitable target (e.g., a circularized SNAIL probe set on an RNA molecule).
**FIG. 4** shows an exemplary SNAIL probe set bound to a nucleic acid molecule (e.g., an mRNA).
**FIG. 5** shows results of the number of DNA concatemer detected (left panel) and the signal intensity of DNA concatemers above background (right panel) from an exemplary *in situ* detection experiment using morpholino detection probes or DNA control detection probes.

### DETAILED DESCRIPTION

All publications, comprising patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. Overview

A concatemer such as rolling circle amplification (RCA) products produced *in situ* or in bulk may display limited accessibility for probe hybridization, such as for binding of detection oligonucleotides. For example, analysis of DNA concatemer (e.g., RCA product or amplicon) size shows that increasing sizes of amplicons do not correlate to increasing brightness in *in situ* detection of the amplicons using fluorescently labeled detection oligonucleotides. In some embodiments, the detection probes are only able to hybridize to exposed sites, e.g., in the periphery of the DNA concatemer, and are less able to freely diffuse into the center of the structure because of steric or electrostatic effects. In some embodiments, only a small fraction of the potential docking sites on the DNA concatemer can be utilized. In some embodiments, maximizing the ability of detection probes to hybridize to an increased number of available docking sites in the DNA concatemer would significantly increase in the brightness of each amplicon, thereby increasing assay signal and/or sensitivity, e.g., for *in situ* sequencing of barcode sequences in DNA concatemers.

In one aspect, the low efficiency of detection probes hybridizing to all available docking sites in the DNA concatemer may be attributed to properties related to the physical chemistry of the DNA concatemer such as steric hindrance, wherein the DNA concatemers are coiled so tightly that the exclusion of the detection probe is primarily caused by base-stacking and other intra-polymeric interactions. In another aspect, negative phosphate charges along the DNA concatemer backbone impose a "swelling" of the amplicon structure through internal coulombic forces; thereby generating a smaller DNA polymer that exists in a looser configuration and larger topological size. In some embodiments, the large DNA concatemer sizes (e.g., ~1.3 µm in diameter) observed *in situ* present an anionic charge field sufficient to inhibit detection probe hybridization towards the center of the DNA concatemer. As more detection probes hybridize, the anionic charge field increases. Evidence that the observed lack of correlation between DNA concatemer size and brightness due to electrostatics is further bolstered by the observation that during molecular crowding events using large numbers of genes in a panel, the mean size of the DNA concatemers decreases while simultaneously increasing in brightness. The electrostatic or other effects of the DNA concatemer on detection probe hybridization accessibility may be decreased by using fluorescently labeled detection oligonucleotides with a synthetic backbone (e.g., morpholinos) as a substitute for the standard probes (e.g., intermediate probes such as secondary probes, and/or detection probes).

In one aspect, provided herein is a method in which a biological sample comprising a DNA concatemer is contacted with a probe (e.g., detection probe and/or intermediate probe), wherein the probe comprises a sequence of nucleobases on a synthetic backbone, and the probe directly or indirectly hybridizes to a target sequence in the DNA concatemer, and wherein there are more probes hybridized to the DNA concatemer than a reference probe having the same sequence of nucleobases on a sugar-phosphate backbone. In some examples, there are more probes having a sequence of nucleobases on a synthetic backbone hybridized in the interior of the DNA concatemer compared to reference probes having the same sequence of nucleobases on a sugar-phosphate backbone. In some embodiments, signals generated from a plurality of detection probes bound to a DNA concatemer having a sequence of nucleobases on a synthetic backbone is greater than signals generated by a plurality of reference detection probes bound to the DNA concatemer having the same sequence of nucleobases on a sugar-phosphate backbone. In some embodiments, the probe (e.g., a morpholino-based detection or intermediate probe) is less electrostatically negative than a reference probe having the same sequence of nucleobases on a sugar-phosphate backbone.

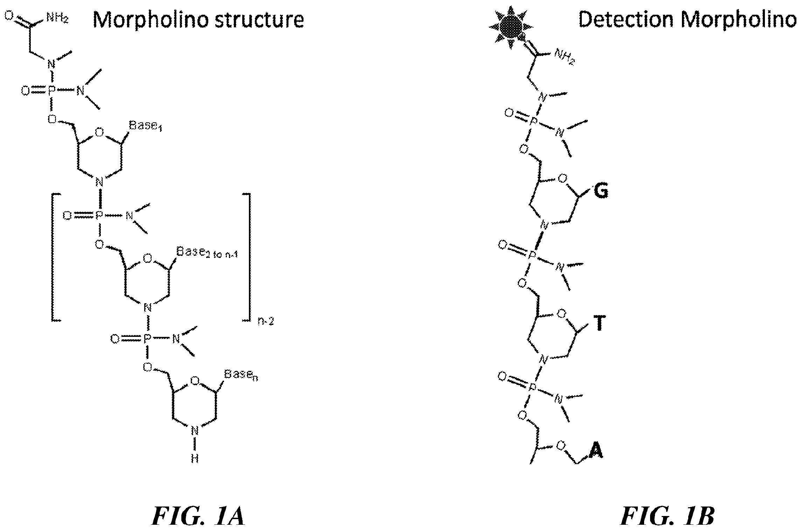

Morpholinos are a class of nucleic acid analogs that have morpholino (e.g., methylenemorpholine) rings in their backbones (FIG. 1A), which replace the deoxyribose (or ribose) rings characteristic of DNA and RNA oligonucleotides. In some embodiments, morpholinos contain uncharged phosphorodiamidate inter-subunit linkages instead of the anionic phosphodiester linkage found in natural nucleic acids. In some embodiments, the morpholine rings carry A, C, G or T, or other natural or synthetic bases suitably arranged for Watson-Crick base pairing and are commercially available. In some embodiments, morpholinos are uncharged molecules which are insensitive to the anionic charge properties of the DNA concatemer. In some embodiments, an electrostatic effect is responsible for the non-correlation between DNA concatemer size and brightness, and a signal is increased with a morpholino detection oligonucleotide (**FIG. 1B**) compared to DNA detection oligonucleotide. Standard DNA probes (labeled or unlabeled with a detectable label) may be hindered from hybridization towards the center of a DNA concatemer (e.g., an RCA product) due to the anionic charge field of the concatemer **(****FIG. 2A****)**. In some aspects, nucleic acid analog probes such as morpholino-based probes (labeled or unlabeled with a detectable label) may show an increased binding capacity to the DNA concatemer due to their decreased sensitivity to the charge properties of the concatemer **(****FIG. 2B****)**.

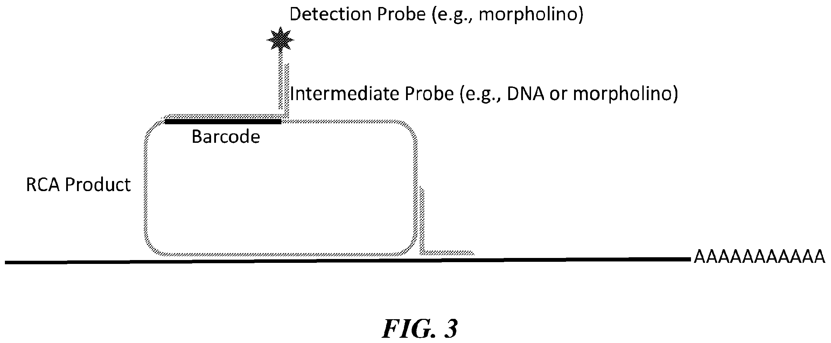

In one aspect, a method disclosed herein comprises using fluorescently labeled morpholinos as detection probes during the chemical/optical decoding cycles used during *in situ* analysis or sequencing. For example, in cases where a primary probe (e.g., padlock or circular probe) is used to generate an RCA, and an intermediate probe (e.g., secondary probe) binds to an RCA product and a detection probe binds to the secondary probe, the detection probe, any intermediate probes, or both can be nucleic acid analog probes such as morpholino-based probes (**FIG. 3**). Additional intermediate probe(s) may be used to bridge the binding of detectably labeled probe(s) to the RCA product, and any one or more of the detectably labeled probe(s) and the intermediate probe(s) may be nucleic acid analog probes such as morpholino-based probes (e.g., comprising nucleobases bases linked through phosphorodiamidate groups).

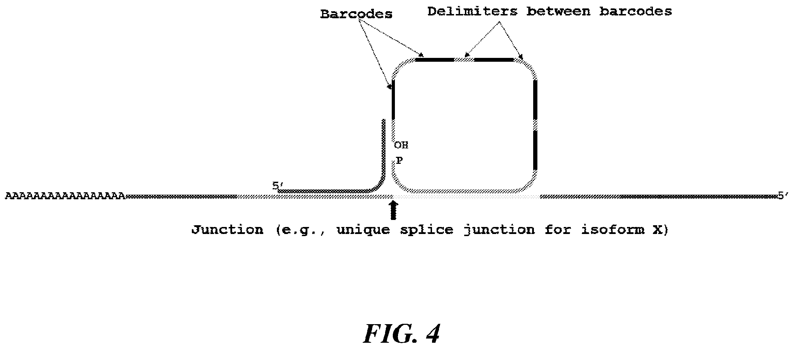

In some embodiments, using nucleic acid analog probes such as morpholino-based probes (e.g., as the detection probe and/or intermediate probe) allows less amplification time and smaller DNA concatemer (e.g., in the sub-micron range), while retaining an equivalent or even higher degree of signal brightness. In some embodiments, brighter and smaller amplicons decrease optical crowding, allowing for more genes to be analyzed in their positional context during a single experiment. In some embodiments, a method described herein permits using fewer probes (e.g., primary and/or secondary probes) to achieve adequate signal detection. In some embodiments, a method described herein also permits using fewer padlock probes (e.g., a single padlock probe) per RNA target, rather than conventional designs comprising 4 probes per RNA target. In some embodiments, a method disclosed herein allows examining the positional location of individual isoforms of a gene of interest simultaneously, as single probes could be designed to straddle unique exon/exon junctions, for example, as shown in **FIG. 4**.

In some instances, a major advantage of using a single probe (e.g., a single circular or circularizable probe such as a padlock probe) per analyte (e.g., RNA) is that molecular crowding issues can be addressed simply by diluting the probes, due to a one to one mapping of analytes (e.g., transcripts) to probes. Thus, decreasing DNA concatemer (e.g., RCA amplicon) size while increasing brightness of the DNA concatemer may be achieved if there are electrostatic and/or other properties of the DNA concatemer that to some degree prohibit hybridization of a large percentage of the available docking sites (e.g., barcode regions) in the DNA concatemer. In some instances, provided herein are uncharged oligonucleotide analogs, such as morpholino-based probes, that would overcome or reduce the prohibition of hybridization and afford a variety of advantages over the current standard nucleic acid probes, such as DNA probes.

### II. Samples, Analytes, and Target Sequences

### A. Samples

A sample disclosed herein can be or derived from any biological sample. Methods and compositions disclosed herein may be used for analyzing a biological sample, which may be obtained from a subject using any of a variety of techniques including, but not limited to, biopsy, surgery, and laser capture microscopy (LCM), and generally includes cells and/or other biological material from the subject. In addition to the subjects described above, a biological sample can be obtained from a prokaryote such as a bacterium, an archaea, a virus, or a viroid. A biological sample can also be obtained from non-mammalian organisms (e.g., a plant, an insect, an arachnid, a nematode, a fungus, or an amphibian). A biological sample can also be obtained from a eukaryote, such as a tissue sample, a patient derived organoid (PDO) or patient derived xenograft (PDX). A biological sample from an organism may comprise one or more other organisms or components therefrom. For example, a mammalian tissue section may comprise a prion, a viroid, a virus, a bacterium, a fungus, or components from other organisms, in addition to mammalian cells and non-cellular tissue components. Subjects from which biological samples can be obtained can be healthy or asymptomatic individuals, individuals that have or are suspected of having a disease (e.g., a patient with a disease such as cancer) or a pre-disposition to a disease, and/or individuals in need of therapy or suspected of needing therapy.

The biological sample can include any number of macromolecules, for example, cellular macromolecules and organelles (e.g., mitochondria and nuclei). The biological sample can be a nucleic acid sample and/or protein sample. The biological sample can be a carbohydrate sample or a lipid sample. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions. In some embodiments, the biological sample may comprise cells which are deposited on a surface.

Cell-free biological samples can include extracellular polynucleotides. Extracellular polynucleotides can be isolated from a bodily sample, e.g., blood, plasma, serum, urine, saliva, mucosal excretions, sputum, stool, and tears.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells. Biological samples can also include fetal cells and immune cells.

Biological samples can include analytes (e.g., protein, RNA, and/or DNA) embedded in a 3D matrix. In some embodiments, amplicons (e.g., rolling circle amplification products) derived from or associated with analytes (e.g., protein, RNA, and/or DNA) can be embedded in a 3D matrix. In some embodiments, a 3D matrix may comprise a network of natural molecules and/or synthetic molecules that are chemically and/or enzymatically linked, e.g., by crosslinking. In some embodiments, a 3D matrix may comprise a synthetic polymer. In some embodiments, a 3D matrix comprises a hydrogel.

In some embodiments, a substrate herein can be any support that is insoluble in aqueous liquid and which allows for positioning of biological samples, analytes, features, and/or reagents (e.g., probes) on the support. In some embodiments, a biological sample can be attached to a substrate. Attachment of the biological sample can be irreversible or reversible, depending upon the nature of the sample and subsequent steps in the analytical method. In certain embodiments, the sample can be attached to the substrate reversibly by applying a suitable polymer coating to the substrate, and contacting the sample to the polymer coating. The sample can then be detached from the substrate, e.g., using an organic solvent that at least partially dissolves the polymer coating. Hydrogels are examples of polymers that are suitable for this purpose.

In some embodiments, the substrate can be coated or functionalized with one or more substances to facilitate attachment of the sample to the substrate. Suitable substances that can be used to coat or functionalize the substrate include, but are not limited to, lectins, poly-lysine, antibodies, and polysaccharides.

A variety of steps can be performed to prepare or process a biological sample for and/or during an assay. Except where indicated otherwise, the preparative or processing steps described below can generally be combined in any manner and in any order to appropriately prepare or process a particular sample for and/or analysis.

### (i) Tissue Sectioning

A biological sample can be harvested from a subject (e.g., via surgical biopsy, whole subject sectioning) or grown in vitro on a growth substrate or culture dish as a population of cells, and prepared for analysis as a tissue slice or tissue section. Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section can be prepared by applying a touch imprint of a biological sample to a suitable substrate material.

The thickness of the tissue section can be a fraction of (e.g., less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, e.g., 10-20 µm thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 1.5, 2, 3, 4, 5, 6, 7, 8, 9, 10, 12, 13, 14, 15, 20, 30, 40, or 50 µm. Thicker sections can also be used if desired or convenient, e.g., at least 70, 80, 90, or 100 µm or more. Typically, the thickness of a tissue section is between 1-100 µm, 1-50 µm, 1-30 µm, 1-25 µm, 1-20 µm, 1-15 µm, 1-10 µm, 2-8 µm, 3-7 µm, or 4-6 µm, but as mentioned above, sections with thicknesses larger or smaller than these ranges can also be analysed.

Multiple sections can also be obtained from a single biological sample. For example, multiple tissue sections can be obtained from a surgical biopsy sample by performing serial sectioning of the biopsy sample using a sectioning blade. Spatial information among the serial sections can be preserved in this manner, and the sections can be analysed successively to obtain three-dimensional information about the biological sample.

### (ii) Freezing

In some embodiments, the biological sample (e.g., a tissue section as described above) can be prepared by deep freezing at a temperature suitable to maintain or preserve the integrity (e.g., the physical characteristics) of the tissue structure. The frozen tissue sample can be sectioned, e.g., thinly sliced, onto a substrate surface using any number of suitable methods. For example, a tissue sample can be prepared using a chilled microtome (e.g., a cryostat) set at a temperature suitable to maintain both the structural integrity of the tissue sample and the chemical properties of the nucleic acids in the sample. Such a temperature can be, e.g., less than -15°C, less than -20°C, or less than -25°C.

### (iii) Fixation and Postfixation

In some embodiments, the biological sample can be prepared using formalin-fixation and paraffin-embedding (FFPE), which are established methods. In some embodiments, cell suspensions and other non-tissue samples can be prepared using formalin-fixation and paraffin-embedding. Following fixation of the sample and embedding in a paraffin or resin block, the sample can be sectioned as described above. Prior to analysis, the paraffin-embedding material can be removed from the tissue section (e.g., deparaffinization) by incubating the tissue section in an appropriate solvent (e.g., xylene) followed by a rinse (e.g., 99.5% ethanol for 2 minutes, 96% ethanol for 2 minutes, and 70% ethanol for 2 minutes).

As an alternative to formalin fixation described above, a biological sample can be fixed in any of a variety of other fixatives to preserve the biological structure of the sample prior to analysis. For example, a sample can be fixed via immersion in ethanol, methanol, acetone, paraformaldehyde (PFA)-Triton, and combinations thereof.

In some embodiments, acetone fixation is used with fresh frozen samples, which can include, but are not limited to, cortex tissue, mouse olfactory bulb, human brain tumor, human post-mortem brain, and breast cancer samples. When acetone fixation is performed, pre- permeabilization steps (described below) may not be performed. Alternatively, acetone fixation can be performed in conjunction with permeabilization steps.

In some aspects, the methods comprise fixing an intact tissue. In some aspects, the methods are performed on fixed intact tissue. Generally, fixing or fixation involves preserving biological material (e.g., tissues, cells, organelles, molecules, etc.) from decay and/or degradation. Fixation may be accomplished using any convenient protocol. Fixation can comprise contacting the sample with a fixation reagent (e.g., a reagent that contains at least one fixative). Samples can be contacted by a fixation reagent for a wide range of times, which can depend on the temperature, the nature of the sample, and on the fixative(s). For example, a sample can be contacted by a fixation reagent for 24 or less hours, 18 or less hours, 12 or less hours, 8 or less hours, 6 or less hours, 4 or less hours, 2 or less hours, 60 or less minutes, 45 or less minutes, 30 or less minutes, 25 or less minutes, 20 or less minutes, 15 or less minutes, 10 or less minutes, 5 or less minutes, or 2 or less minutes.

A sample can be contacted by a fixation reagent at various temperatures, depending on the protocol and the reagent used. For example, in some instances a sample can be contacted by a fixation reagent at a temperature ranging from -22°C to 55°C, where specific ranges of interest comprise, but are not limited to 50 to 54°C, 40 to 44°C, 35 to 39°C, 28 to 32°C, 20 to 26°C, 0 to 6°C, and -18 to -22°C. In some instances, a sample can be contacted by a fixation reagent at a temperature of -20°C, 4°C, room temperature (22-25°C), 30°C, 37°C, 42°C, or 52°C.

Any convenient fixation reagent can be used. Common fixation reagents comprise crosslinking fixatives, precipitating fixatives, oxidizing fixatives, mercurials, and the like. Crosslinking fixatives chemically join two or more molecules by a covalent bond and a wide range of cross-linking reagents can be used. Examples of suitable cross liking fixatives comprise but are not limited to aldehydes (e.g., formaldehyde, also commonly referred to as "paraformaldehyde" and "formalin"; glutaraldehyde; etc.), imidoesters, NHS (N- Hydroxysuccinimide) esters, and the like. Examples of suitable precipitating fixatives comprise but are not limited to alcohols (e.g., methanol, ethanol, etc.), acetone, acetic acid, etc. In some embodiments, the fixative is formaldehyde (e.g., paraformaldehyde or formalin). A suitable final concentration of formaldehyde in a fixation reagent is 0.1 to 10%, 1-8%, 1-4%, 1-2%, 3-5%, or 3.5-4.5%, comprising about 1.6% for 10 minutes. In some embodiments, the sample is fixed in a final concentration of 4% formaldehyde (as diluted from a more concentrated stock solution, e.g., 38%, 37%, 36%, 20%, 18%, 16%, 14%, 10%, 8%, 6%, etc.). In some embodiments, the sample is fixed in a final concentration of 10% formaldehyde. In some embodiments, the sample is fixed in a final concentration of 1% formaldehyde. In some embodiments, the fixative is glutaraldehyde. A suitable concentration of glutaraldehyde in a fixation reagent is 0.1 to 1%. A fixation reagent can contain more than one fixative in any combination. For example, in some embodiments, the sample is contacted with a fixation reagent containing both formaldehyde and glutaraldehyde.

In some embodiments, the methods provided herein comprises one or more post-fixing (also referred to as postfixation) steps. In some embodiments, one or more post-fixing step is performed after contacting a sample with a polynucleotide disclosed herein, e.g., one or more probes such as a circular or padlock probe. In some embodiments, one or more post-fixing step is performed after a hybridization complex comprising a probe and a target is formed in a sample. In some embodiments, one or more post-fixing step is performed prior to a ligation reaction disclosed herein, such as the ligation to circularize a padlock probe.

In some embodiments, one or more post-fixing step is performed after contacting a sample with a binding or labelling agent (e.g., an antibody or antigen binding fragment thereof) for a non-nucleic acid analyte such as a protein analyte. The labelling agent can comprise a nucleic acid molecule (e.g., reporter oligonucleotide) comprising a sequence corresponding to the labelling agent and therefore corresponds to (e.g., uniquely identifies) the analyte. In some embodiments, the labelling agent can comprise a reporter oligonucleotide comprising one or more barcode sequences.

A post-fixing step may be performed using any suitable fixation reagent disclosed herein, for example, 3% (w/v) paraformaldehyde in DEPC-PBS.

### (iv) Embedding

As an alternative to paraffin embedding described above, a biological sample can be embedded in any of a variety of other embedding materials to provide structural substrate to the sample prior to sectioning and other handling steps. In some cases, the embedding material can be removed, e.g., prior to analysis of tissue sections obtained from the sample. In some aspects, the embedding material can be applied to the sample one or more times. Suitable embedding materials include, but are not limited to, waxes, resins (e.g., methacrylate resins), epoxies, and agar.

In some embodiments, the biological sample can be embedded in a matrix (e.g., a hydrogel matrix). Embedding the sample in this manner typically involves contacting the biological sample with a hydrogel such that the biological sample becomes surrounded by the hydrogel. For example, the sample can be embedded by contacting the sample with a suitable polymer material, and activating the polymer material to form a hydrogel. In some embodiments, the hydrogel is formed such that the hydrogel is internalized within the biological sample.

In some embodiments, the biological sample is immobilized in the hydrogel via cross-linking of the polymer material that forms the hydrogel. Cross-linking can be performed chemically and/or photochemically, or alternatively by any other hydrogel-formation method known in the art.

The composition and application of the hydrogel-matrix to a biological sample typically depends on the nature and preparation of the biological sample (e.g., sectioned, non-sectioned, type of fixation). As one example, where the biological sample is a tissue section, the hydrogel-matrix can include a monomer solution and an ammonium persulfate (APS) initiator/tetramethylethylenediamine (TEMED) accelerator solution. As another example, where the biological sample consists of cells (e.g., cultured cells or cells disassociated from a tissue sample), the cells can be incubated with the monomer solution and APS/TEMED solutions. For cells, hydrogel-matrix gels are formed in compartments, including but not limited to devices used to culture, maintain, or transport the cells. For example, hydrogel-matrices can be formed with monomer solution plus APS/TEMED added to the compartment to a depth ranging from about 0.1 µm to about 2 mm.

Additional methods and aspects of hydrogel embedding of biological samples are described for example in Chen et al., Science 347(6221):543-548, 2015, the entire contents of which are incorporated herein by reference.

In some embodiments, the methods disclosed comprise embedding the sample in a matrix-forming material, e.g., in a manner to substantially retain the relative three-dimensional spatial relationship of a plurality of nucleic acids in the sample. In some embodiments, the methods disclosed comprise making a three dimensional matrix of nucleic acids. In some embodiments, nucleic acids are covalently bound into a matrix or into or to a matrix material. The nucleic acids may be co-polymerized with the matrix material or cross-linked to the matrix material or both. According to one aspect, a plurality of nucleic acid sequences of certain length, such as DNA or RNA sequences are part of a three-dimensional copolymer. The nucleic acids may then be amplified and detected and/or analyzed (e.g., sequenced) *in situ,* e.g., within the matrix. The three-dimensional matrix of nucleic acids provides, in a certain aspect, an information storage medium where the nucleic acids, e.g., a sequence of one or more nucleotides, represent stored information which can be read within the three-dimensional matrix. Matrix forming materials may comprise polyacrylamide, cellulose, alginate, polyamide, cross-linked agarose, cross-linked dextran, cross-linked polyethylene glycol, disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin, e.g., as described in US Patent No. 10,138,509, herein specifically incorporated by reference. The matrix forming materials can form a matrix by polymerization and/or cross-linking of the matrix forming materials using methods specific for the matrix forming materials and known methods, reagents and conditions

In some aspects, the matrix is sufficiently optically transparent or otherwise has optical properties suitable for detection, imaging and/or sequencing, for example, as described herein and/or using standard high-throughput sequencing chemistries and deep three dimensional imaging for high throughput information readout. Exemplary high-throughput sequencing chemistries that utilize fluorescence imaging comprise ABI SoLiD (Life Technologies), in which a sequencing primer on a template is ligated to a library of fluorescently labeled nonamers with a cleavable terminator. After ligation, the beads are then imaged using four color channels (FITC, Cy3, Texas Red and Cy5). The terminator is then cleaved off leaving a free-end to engage in the next ligation-extension cycle. After all dinucleotide combinations have been determined, the images are mapped to the color code space to determine the specific base calls per template. The workflow is achieved using an automated fluidics and imaging device (e.g., SoLiD 5500 W Genome Analyzer, ABI Life Technologies). Another sequencing platform uses sequencing by synthesis, in which a pool of single nucleotide with a cleavable terminator is incorporated using DNA polymerase. After imaging, the terminator is cleaved and the cycle is repeated. The fluorescence images are then analyzed to call bases for each DNA amplicons within the flow cell (HiSeq, Illumina).

In some embodiments, the methods disclosed comprise preparing a biological tissue specimen for microscopic analysis, e.g., a process that maintains the 3-D integrity of the tissue by embedding it in a hydrogel, and making molecules of interest accessible for optical probing and molecular labeling while allowing undesired molecules such as lipids to be washed away. See Chung et al., "CLARITY for mapping the nervous system," Nature Methods 10 (2013) and US Patent No. 10,545,075, herein specifically incorporated by reference. In some embodiments, the methods disclosed herein comprise fixing a biological tissue specimen obtained from a mammal by contacting the biological tissue specimen with a fixation agent and a plurality of hydrogel subunits, thereby cross-linking the hydrogel subunits to biomolecules within the biological tissue specimen to produce biomolecule-bound hydrogel subunits. In some embodiments, the methods further comprise polymerizing the biomolecule-bound hydrogel subunits to form a hydrogel-embedded biological tissue specimen. In some embodiments, the methods further comprise electrophoresing the hydrogel-embedded biological tissue specimen to remove a plurality of cellular components from the specimen and form a cleared hydrogel-embedded biological tissue specimen.

In some embodiments, the methods disclosed comprise embedding the sample in a hydrogel. The hydrogel-tissue chemistry described comprises covalently attaching nucleic acids to in situ synthesized hydrogel for tissue clearing, enzyme diffusion, and multiple-cycle sequencing. In some embodiments, a hydrogel or hydrogel network comprises a network of polymer chains that are water-insoluble, sometimes found as a colloidal gel in which water is the dispersion medium. In other words, hydrogels are a class of polymeric materials that can absorb large amounts of water without dissolving. Hydrogels can contain over 99% water and may comprise natural or synthetic polymers, or a combination thereof.

Hydrogels also possess a degree of flexibility very similar to natural tissue, due to their significant water content. A detailed description of suitable hydrogels may be found in published U.S. Patent Application 20100055733, herein specifically incorporated by reference. As used herein, the terms "hydrogel subunits" or "hydrogel precursors" mean hydrophilic monomers, prepolymers, or polymers that can be crosslinked, or "polymerized", to form a three-dimensional (3D) hydrogel network. Without being bound by any scientific theory, it is believed that this fixation of the biological specimen in the presence of hydrogel subunits crosslinks the components of the specimen to the hydrogel subunits, thereby securing molecular components in place, preserving the tissue architecture and cell morphology.

In some embodiments, the embedding comprises copolymerizing the one or more amplicons with acrylamide, for example, to form a copolymer which contains more than one type of subunit. A copolymer encompasses polymers which comprise two, three, four, five, or six types of subunits.

### (v) Staining and Immunohistochemistry (IHC)

To facilitate visualization, biological samples can be stained using a wide variety of stains and staining techniques. In some embodiments, for example, a sample can be stained using any number of stains, and/or immunohistochemical reagents. One or more staining steps may be performed to prepare or process a biological sample for an assay described herein or may be performed during and/or after an assay. In some embodiments, the sample can be contacted with one or more nucleic acid stains, membrane stains (e.g., cellular or nuclear membrane), cytological stains, or combinations thereof. In some examples, the stain may be specific to proteins, phospholipids, DNA (e.g., dsDNA, ssDNA), RNA, an organelle or compartment of the cell. The sample may be contacted with one or more labeled antibodies (e.g., a primary antibody specific for the analyte of interest and a labeled secondary antibody specific for the primary antibody). In some embodiments, cells in the sample can be segmented using one or more images taken of the stained sample. In some embodiments, the stain is performed using a lipophilic dye. In some examples, the staining is performed with a lipophilic carbocyanine or aminostyryl dye, or analogs thereof (e.g, DiI, DiO, DiR, DiD). Other cell membrane stains may include FM and RH dyes or immunohistochemical reagents specific for cell membrane proteins. In some examples, the stain may include but is not limited to, acridine orange, acid fuchsin, Bismarck brown, carmine, coomassie blue, cresyl violet, DAPI, eosin, ethidium bromide, acid fuchsine, haematoxylin, Hoechst stains, iodine, methyl green, methylene blue, neutral red, Nile blue, Nile red, osmium tetroxide, propidium iodide, rhodamine, or safranine.

The sample can be stained using hematoxylin and eosin (H&E) staining techniques, using Papanicolaou staining techniques, Masson's trichrome staining techniques, silver staining techniques, Sudan staining techniques, and/or using Periodic Acid Schiff (PAS) staining techniques. PAS staining is typically performed after formalin or acetone fixation. In some embodiments, the sample can be stained using Romanowsky stain, including Wright's stain, Jenner's stain, Can-Grunwald stain, Leishman stain, and Giemsa stain. The sample can also be subjected to staining of cell nuclei, such as by fluorescent dyes including DAPI, Hoechst 33258, olivomycin, and/or acriflavin and/or nonfluorescent staining.

In some embodiments, biological samples can be destained. Methods of destaining or discoloring a biological sample are known in the art, and generally depend on the nature of the stain(s) applied to the sample. For example, in some embodiments, one or more immunofluorescent stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905, the entire contents of each of which are incorporated herein by reference.

### (vi) Isometric Expansion

In some embodiments, a biological sample embedded in a matrix (e.g., a hydrogel) can be isometrically expanded. Isometric expansion methods that can be used include hydration, a preparative step in expansion microscopy, as described in Chen et al., Science 347(6221):543-548, 2015.

Isometric expansion can be performed by anchoring one or more components of a biological sample to a gel, followed by gel formation, proteolysis, and swelling. In some embodiments, analytes in the sample, products of the analytes, and/or probes associated with analytes in the sample can be anchored to the matrix (e.g., hydrogel). Isometric expansion of the biological sample can occur prior to immobilization of the biological sample on a substrate, or after the biological sample is immobilized to a substrate. In some embodiments, the isometrically expanded biological sample can be removed from the substrate prior to contacting the substrate with probes disclosed herein.

In general, the steps used to perform isometric expansion of the biological sample can depend on the characteristics of the sample (e.g., thickness of tissue section, fixation, cross-linking), and/or the analyte of interest (e.g., different conditions to anchor RNA, DNA, and protein to a gel).

In some embodiments, proteins in the biological sample are anchored to a swellable gel such as a polyelectrolyte gel. An antibody can be directed to the protein before, after, or in conjunction with being anchored to the swellable gel. DNA and/or RNA in a biological sample can also be anchored to the swellable gel via a suitable linker. Examples of such linkers include, but are not limited to, 6-((Acryloyl)amino) hexanoic acid (Acryloyl-X SE) (available from ThermoFisher, Waltham, MA), Label-IT Amine (available from MirusBio, Madison, WI) and Label X (described for example in Chen et al., Nat. Methods 13 :679-684, 2016, the entire contents of which are incorporated herein by reference).

Isometric expansion of the sample can increase the spatial resolution of the subsequent analysis of the sample. The increased resolution in spatial profiling can be determined by comparison of an isometrically expanded sample with a sample that has not been isometrically expanded.

In some embodiments, a biological sample is isometrically expanded to a size at least 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x, 4.1x, 4.2x, 4.3x, 4.4x, 4.5x, 4.6x, 4.7x, 4.8x, or 4.9x its non-expanded size. In some embodiments, the sample is isometrically expanded to at least 2x and less than 20x of its non-expanded size.

### (vii) Crosslinking and De-crosslinking

In some embodiments, the biological sample is reversibly cross-linked prior to or during an *in situ* assay round. In some aspects, the analytes, polynucleotides and/or amplification product (e.g., amplicon) of an analyte or a probe bound thereto can be anchored to a polymer matrix. For example, the polymer matrix can be a hydrogel. In some embodiments, one or more of the polynucleotide probe(s) and/or amplification product (e.g., amplicon) thereof can be modified to contain functional groups that can be used as an anchoring site to attach the polynucleotide probes and/or amplification product to a polymer matrix. In some embodiments, a modified probe comprising oligo dT may be used to bind to mRNA molecules of interest, followed by reversible crosslinking of the mRNA molecules.

In some embodiments, the biological sample is immobilized in a hydrogel via cross-linking of the polymer material that forms the hydrogel. Cross-linking can be performed chemically and/or photochemically, or alternatively by any other hydrogel-formation method known in the art. A hydrogel may include a macromolecular polymer gel including a network. Within the network, some polymer chains can optionally be cross-linked, although cross-linking does not always occur.

In some embodiments, a hydrogel can include hydrogel subunits, such as, but not limited to, acrylamide, bis-acrylamide, polyacrylamide and derivatives thereof, poly(ethylene glycol) and derivatives thereof (e.g. PEG-acrylate (PEG-DA), PEG-RGD), gelatin-methacryloyl (GelMA), methacrylated hyaluronic acid (MeHA), polyaliphatic polyurethanes, polyether polyurethanes, polyester polyurethanes, polyethylene copolymers, polyamides, polyvinyl alcohols, polypropylene glycol, polytetramethylene oxide, polyvinyl pyrrolidone, polyacrylamide, poly(hydroxyethyl acrylate), and poly(hydroxyethyl methacrylate), collagen, hyaluronic acid, chitosan, dextran, agarose, gelatin, alginate, protein polymers, methylcellulose, and the like, and combinations thereof.

In some embodiments, a hydrogel includes a hybrid material, e.g., the hydrogel material includes elements of both synthetic and natural polymers. Examples of suitable hydrogels are described, for example, in U.S. Patent Nos. 6,391,937, 9,512,422, and 9,889,422, and in U.S. Patent Application Publication Nos. 2017/0253918, 2018/0052081 and 2010/0055733, the entire contents of each of which are incorporated herein by reference.

In some embodiments, the hydrogel can form the substrate. In some embodiments, the substrate includes a hydrogel and one or more second materials. In some embodiments, the hydrogel is placed on top of one or more second materials. For example, the hydrogel can be pre-formed and then placed on top of, underneath, or in any other configuration with one or more second materials. In some embodiments, hydrogel formation occurs after contacting one or more second materials during formation of the substrate. Hydrogel formation can also occur within a structure (e.g., wells, ridges, projections, and/or markings) located on a substrate

In some embodiments, hydrogel formation on a substrate occurs before, contemporaneously with, or after probes are provided to the sample. For example, hydrogel formation can be performed on the substrate already containing the probes.

In some embodiments, hydrogel formation occurs within a biological sample. In some embodiments, a biological sample (e.g., tissue section) is embedded in a hydrogel. In some embodiments, hydrogel subunits are infused into the biological sample, and polymerization of the hydrogel is initiated by an external or internal stimulus.

In embodiments in which a hydrogel is formed within a biological sample, functionalization chemistry can be used. In some embodiments, functionalization chemistry includes hydrogel-tissue chemistry (HTC). Any hydrogel-tissue backbone (e.g., synthetic or native) suitable for HTC can be used for anchoring biological macromolecules and modulating functionalization. Non-limiting examples of methods using HTC backbone variants include CLARITY, PACT, ExM, SWITCH and ePACT. In some embodiments, hydrogel formation within a biological sample is permanent. For example, biological macromolecules can permanently adhere to the hydrogel allowing multiple rounds of interrogation. In some embodiments, hydrogel formation within a biological sample is reversible.

In some embodiments, additional reagents are added to the hydrogel subunits before, contemporaneously with, and/or after polymerization. For example, additional reagents can include but are not limited to oligonucleotides (e.g., probes), endonucleases to fragment DNA, fragmentation buffer for DNA, DNA polymerase enzymes, dNTPs used to amplify the nucleic acid and to attach the barcode to the amplified fragments. Other enzymes can be used, including without limitation, RNA polymerase, transposase, ligase, proteinase K, and DNAse. Additional reagents can also include reverse transcriptase enzymes, including enzymes with terminal transferase activity, primers, and switch oligonucleotides. In some embodiments, optical labels are added to the hydrogel subunits before, contemporaneously with, and/or after polymerization.

In some embodiments, HTC reagents are added to the hydrogel before, contemporaneously with, and/or after polymerization. In some embodiments, a cell labelling agent is added to the hydrogel before, contemporaneously with, and/or after polymerization. In some embodiments, a cell-penetrating agent is added to the hydrogel before, contemporaneously with, and/or after polymerization.

Hydrogels embedded within biological samples can be cleared using any suitable method. For example, electrophoretic tissue clearing methods can be used to remove biological macromolecules from the hydrogel-embedded sample. In some embodiments, a hydrogel-embedded sample is stored before or after clearing of hydrogel, in a medium (e.g., a mounting medium, methylcellulose, or other semi-solid mediums).

In some embodiments, a method disclosed herein comprises de-crosslinking the reversibly cross-linked biological sample. The de-crosslinking does not need to be complete. In some embodiments, only a portion of crosslinked molecules in the reversibly cross-linked biological sample are de-crosslinked and allowed to migrate.

### (viii) Tissue Permeabilization and Treatment

In some embodiments, a biological sample can be permeabilized to facilitate transfer of analytes out of the sample, and/or to facilitate transfer of species (such as probes) into the sample If a sample is not permeabilized sufficiently, the amount of analyte captured from the sample may be too low to enable adequate analysis. Conversely, if the tissue sample is too permeable, the relative spatial relationship of the analytes within the tissue sample can be lost. Hence, a balance between permeabilizing the tissue sample enough to obtain good signal intensity while still maintaining the spatial resolution of the analyte distribution in the sample is desirable.

In general, a biological sample can be permeabilized by exposing the sample to one or more permeabilizing agents. Suitable agents for this purpose include, but are not limited to, organic solvents (e.g., acetone, ethanol, and methanol), cross-linking agents (e.g., paraformaldehyde), detergents (e.g., saponin, Triton X-100^{™} or Tween-20^{™}), and enzymes (e.g., trypsin, proteases). In some embodiments, the biological sample can be incubated with a cellular permeabilizing agent to facilitate permeabilization of the sample. Additional methods for sample permeabilization are described, for example, in Jamur et al., Method Mol. Biol. 588:63-66, 2010, the entire contents of which are incorporated herein by reference. Any suitable method for sample permeabilization can generally be used in connection with the samples described herein.

In some embodiments, where a diffusion-resistant medium is used to limit migration of analytes or other species during the analytical procedure, the diffusion-resistant medium can include at least one permeabilization reagent. For example, the diffusion-resistant medium can include wells (e.g., micro-, nano-, or picowells) containing a permeabilization buffer or reagents. In some embodiments, where the diffusion-resistant medium is a hydrogel, the hydrogel can include a permeabilization buffer. In some embodiments, the hydrogel is soaked in permeabilization buffer prior to contacting the hydrogel with a sample. In some embodiments, the hydrogel or other diffusion-resistant medium can contain dried reagents or monomers to deliver permeabilization reagents when the diffusion-resistant medium is applied to a biological sample. In some embodiments, the diffusion-resistant medium (e.g., hydrogel) is covalently attached to a solid substrate (e.g., an acrylated glass slide).

In some embodiments, permeabilization solution can be delivered to a sample through a porous membrane. In some embodiments, a porous membrane is used to limit diffusive analyte losses, while allowing permeabilization reagents to reach a sample. Membrane chemistry and pore size can be manipulated to minimize analyte loss. In some embodiments, the porous membrane may be made of glass, silicon, paper, hydrogel, polymer monoliths, or other material. In some embodiments, the material may be naturally porous. In some embodiments, the material may have pores or wells etched into solid material. In some embodiments, the permeabilization reagents are flowed through a microfluidic chamber or channel over the porous membrane. In some embodiments, the flow controls the sample's access to the permeabilization reagents. In some embodiments, the permeabilization reagents diffuse through the pores of the membrane and into the tissue.

In some embodiments, the biological sample can be permeabilized by adding one or more lysis reagents to the sample. Examples of suitable lysis agents include, but are not limited to, bioactive reagents such as lysis enzymes that are used for lysis of different cell types, e.g., gram positive or negative bacteria, plants, yeast, mammalian, such as lysozymes, achromopeptidase, lysostaphin, labiase, kitalase, lyticase, and a variety of other commercially available lysis enzymes.

Other lysis agents can additionally or alternatively be added to the biological sample to facilitate permeabilization. For example, surfactant-based lysis solutions can be used to lyse sample cells. Lysis solutions can include ionic surfactants such as, for example, sarcosyl and sodium dodecyl sulfate (SDS). More generally, chemical lysis agents can include, without limitation, organic solvents, chelating agents, detergents, surfactants, and chaotropic agents.

In some embodiments, the biological sample can be permeabilized by non-chemical permeabilization methods. Non-chemical permeabilization methods are known in the art. For example, non-chemical permeabilization methods that can be used include, but are not limited to, physical lysis techniques such as electroporation, mechanical permeabilization methods (e.g., bead beating using a homogenizer and grinding balls to mechanically disrupt sample tissue structures), acoustic permeabilization (e.g., sonication), and thermal lysis techniques such as heating to induce thermal permeabilization of the sample.

In some aspects, the methods provided herein comprise permeabilizing the tissue. In some embodiments, the methods are performed using permeabilized tissue. In some aspects, permeabilization comprises a process of rendering the cells (cell membranes etc.) of a sample permeable to experimental reagents such as nucleic acid probes, antibodies, chemical substrates, etc. Any convenient method and/or reagent for permeabilization can be used. Suitable permeabilization reagents comprise detergents (e.g., Saponin, Triton X-100, Tween-20, etc.), organic fixatives (e.g., acetone, methanol, ethanol, etc.), enzymes, etc. Detergents can be used at a range of concentrations. For example, 0.001%-1% detergent, 0.05%-0.5% detergent, or 0.1%- 0.3% detergent can be used for permeabilization (e.g., 0.1 % Saponin, 0.2% tween-20, 0.1-0.3% triton X-100, etc.). In some embodiments, methanol on ice for at least 10 minutes is used to permeabilize.

In some embodiments, a sample can be contacted by a permeabilization reagent for a wide range of times, which can depend on the temperature, the nature of the sample, and on the permeabilization reagent(s). For example, a sample can be contacted by a permeabilization reagent for 24 or more hours, 24 hours or less, 18 hours or less, 12 hours or less, 8 hours or less, 6 hours or less, 4 hours or less, 2 hours or less, 60 minutes or less, 45 minutes or less, 30 minutes or less, 25 minutes or less, 20 minutes or less, 15 minutes or less, 10 minutes or less, 5 minutes or less, or 2 minutes or less. In some embodiments, a sample can be contacted by a permeabilization reagent at various temperatures, depending on the protocol and the reagent used. For example, in some instances a sample can be contacted by a permeabilization reagent at a temperature ranging from -82°C to 55°C, where specific ranges of interest comprise, but are not limited to: 50 to 54°C, 40 to 44°C, 35 to 39°C, 28 to 32°C, 20 to 26°C, 0 to 6°C, -18 to -22 °C, and -78 to -82°C. In some instances a sample can be contacted by a permeabilization reagent at a temperature of - 80°C, -20°C, 4°C, room temperature (in some examples, less than 30°C, or 22-25°C), 30°C, 37°C, 42°C, or 52°C.

In some embodiments, a sample is contacted with an enzymatic permeabilization reagent. Enzymatic permeabilization reagents that permeabilize a sample by partially degrading extracellular matrix or surface proteins that hinder the permeation of the sample by assay reagents. Contact with an enzymatic permeabilization reagent can take place at any point after fixation and prior to target detection. In some instances, the enzymatic permeabilization reagent is proteinase K, a commercially available enzyme. In some cases, permeabilization is performed prior to providing the probe polynucleotides (e.g., the circular or padlock probe, primer, and anchor) to the sample.

In such cases, the sample is contacted with proteinase K prior to contact with a post fixation reagent. Proteinase K treatment (e.g., contact by proteinase K; also commonly referred to as "proteinase K digestion") can be performed over a range of times at a range of temperatures, over a range of enzyme concentrations that are empirically determined for each cell type or tissue type under investigation. For example, a sample can be contacted by proteinase K for 30 minutes or less, 25 minutes or less, 20 minutes or less, 15 minutes or less, 10 minutes or less, 5 minutes or less, or 2 minutes or less. A sample can be contacted by 1 pg/ml or less, 2 pg/ml or less, 4pg/ml or less, 8 pg/ml or less, 10 pg/ml or less, 20 pg/ml or less, 30 pg/ml or less, 50 pg/ml or less, or100 pg/ml or less proteinase K. In some embodiments, a sample can be contacted by proteinase K at a temperature ranging from 2°C to 55°C, where specific ranges of interest comprise, but are not limited to: 50 to 54°C, 40 to 44°C, 35 to 39°C, 28 to 32°C, 20 to 26°C, and 0 to 6°C. In some instances, a sample can be contacted by proteinase K at a temperature of 4°C, room temperature (in some examples, less than 30°C, or 22-25°C), 30°C, 37°C, 42°C, or 52°C. In some embodiments, a sample is not contacted with an enzymatic permeabilization reagent. In some embodiments, a sample is not contacted with proteinase K. In some aspects, contact of an intact tissue with at least a fixation reagent and a permeabilization reagent results in the production of a fixed and permeabilized tissue.

Additional reagents can be added to a biological sample to perform various functions prior to analysis of the sample. In some embodiments, DNase and RNase inactivating agents or inhibitors such as proteinase K, and/or chelating agents such as EDTA, can be added to the sample. For example, a method disclosed herein may comprise a step for increasing accessibility of a nucleic acid for binding, e.g., a denaturation step to opening up DNA in a cell for hybridization by a probe. For example, proteinase K treatment may be used to free up DNA with proteins bound thereto.

### (ix) Selective Enrichment of RNA Species

In some embodiments, where RNA is the analyte, one or more RNA analyte species of interest can be selectively enriched. For example, one or more species of RNA of interest can be selected by addition of one or more oligonucleotides to the sample. In some embodiments, the additional oligonucleotide is a sequence used for priming a reaction by an enzyme (e.g., a polymerase). For example, one or more primer sequences with sequence complementarity to one or more RNAs of interest can be used to amplify the one or more RNAs of interest, thereby selectively enriching these RNAs.

In some embodiments, one or more nucleic acid probes can be used to hybridize to a target nucleic acid (e.g., cDNA or RNA molecule, such as an mRNA) and ligated in a templated ligation reaction (e.g., RNA-templated ligation (RTL) or DNA-templated ligation (e.g., on cDNA)) to generate a product for analysis. In some aspects, when two or more analytes are analyzed, a first and second probe that is specific for (e.g., specifically hybridizes to) each RNA or cDNA analyte are used. For example, in some embodiments of the methods provided herein, templated ligation is used to detect gene expression in a biological sample. An analyte of interest (such as a protein), bound by a labelling agent or binding agent (e.g., an antibody or epitope binding fragment thereof), wherein the binding agent is conjugated or otherwise associated with a reporter oligonucleotide comprising a reporter sequence that identifies the binding agent, can be targeted for analysis. Probes may be hybridized to the reporter oligonucleotide and ligated in a templated ligation reaction to generate a product for analysis. In some embodiments, gaps between the probe oligonucleotides may first be filled prior to ligation, using, for example, Mu polymerase, DNA polymerase, RNA polymerase, reverse transcriptase, VENT polymerase, Taq polymerase, and/or any combinations, derivatives, and variants (e.g., engineered mutants) thereof. In some embodiments, the assay can further include amplification of templated ligation products (e.g., by multiplex PCR).

In some embodiments, an oligonucleotide with sequence complementarity to the complementary strand of captured RNA (e.g., cDNA) can bind to the cDNA. For example, biotinylated oligonucleotides with sequence complementary to one or more cDNA of interest binds to the cDNA and can be selected using biotinylation-strepavidin affinity using any of a variety of methods known to the field (e.g., streptavidin beads).

Alternatively, one or more species of RNA can be down-selected (e.g., removed) using any of a variety of methods. For example, probes can be administered to a sample that selectively hybridize to ribosomal RNA (rRNA), thereby reducing the pool and concentration of rRNA in the sample. Additionally and alternatively, duplex-specific nuclease (DSN) treatment can remove rRNA (see, e.g., Archer, et al, Selective and flexible depletion of problematic sequences from RNA-seq libraries at the cDNA stage, BMC Genomics, 15 401, (2014), the entire contents of which are incorporated herein by reference). Furthermore, hydroxyapatite chromatography can remove abundant species (e.g., rRNA) (see, e.g., Vandernoot, V.A., cDNA normalization by hydroxyapatite chromatography to enrich transcriptome diversity in RNA-seq applications, Biotechniques, 53(6) 373-80, (2012), the entire contents of which are incorporated herein by reference).

A biological sample may comprise one or a plurality of analytes of interest. Methods for performing multiplexed assays to analyze two or more different analytes in a single biological sample are provided.

### B. Analytes

The methods and compositions disclosed herein can be used to detect and analyze a wide variety of different analytes. In some aspects, an analyte can include any biological substance, structure, moiety, or component to be analyzed. In some aspects, a target disclosed herein may similarly include any analyte of interest. In some examples, a target or analyte can be directly or indirectly detected.

Analytes can be derived from a specific type of cell and/or a specific subcellular region. For example, analytes can be derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis, and/or allow access of one or more reagents (e.g., probes for analyte detection) to the analytes in the cell or cell compartment or organelle.

The analyte may include any biomolecule, macromolecule, or chemical compound, including a protein or peptide, a lipid or a nucleic acid molecule, or a small molecule, including organic or inorganic molecules. The analyte may be a cell or a microorganism, including a virus, or a fragment or product thereof. An analyte can be any substance or entity for which a specific binding partner (e.g. an affinity binding partner) can be developed. Such a specific binding partner may be a nucleic acid probe (for a nucleic acid analyte) and may lead directly to the generation of a RCA template (e.g. a padlock or other circularizable probe). Alternatively, the specific binding partner may be coupled to a nucleic acid, which may be detected using an RCA strategy, e.g. in an assay which uses or generates a circular nucleic acid molecule which can be the RCA template.

Analytes of particular interest may include nucleic acid molecules, such as DNA (e.g. genomic DNA, mitochondrial DNA, plastid DNA, viral DNA, etc.) and RNA (e.g. mRNA, microRNA, rRNA, snRNA, viral RNA, etc.), and synthetic and/or modified nucleic acid molecules, (e.g. including nucleic acid domains comprising or consisting of synthetic or modified nucleotides such as LNA, PNA, morpholino, etc.), proteinaceous molecules such as peptides, polypeptides, proteins or prions or any molecule which includes a protein or polypeptide component, etc., or fragments thereof, or a lipid or carbohydrate molecule, or any molecule which comprise a lipid or carbohydrate component. The analyte may be a single molecule or a complex that contains two or more molecular subunits, e.g. including but not limited to protein-DNA complexes, which may or may not be covalently bound to one another, and which may be the same or different. Thus in addition to cells or microorganisms, such a complex analyte may also be a protein complex or protein interaction. Such a complex or interaction may thus be a homo- or hetero-multimer. Aggregates of molecules, e.g. proteins may also be target analytes, for example aggregates of the same protein or different proteins. The analyte may also be a complex between proteins or peptides and nucleic acid molecules such as DNA or RNA, e.g. interactions between proteins and nucleic acids, e.g. regulatory factors, such as transcription factors, and DNA or RNA.

### (i) Endogenous Analytes

In some embodiments, an analyte herein is endogenous to a biological sample and can include nucleic acid analytes and non-nucleic acid analytes. Methods and compositions disclosed herein can be used to analyze nucleic acid analytes (e.g., using a nucleic acid probe or probe set that directly or indirectly hybridizes to a nucleic acid analyte) and/or non-nucleic acid analytes (e.g., using a labelling agent that comprises a reporter oligonucleotide and binds directly or indirectly to a non-nucleic acid analyte) in any suitable combination

Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral coat proteins, extracellular and intracellular proteins, antibodies, and antigen binding fragments. In some embodiments, the analyte is inside a cell or on a cell surface, such as a transmembrane analyte or one that is attached to the cell membrane. In some embodiments, the analyte can be an organelle (e.g., nuclei or mitochondria). In some embodiments, the analyte is an extracellular analyte, such as a secreted analyte. Exemplary analytes include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (e.g., phosphorylation, glycosylation, ubiquitination, nitrosylation, methylation, acetylation or lipidation) state of a cell surface protein, a gap junction, and an adherens junction.

Examples of nucleic acid analytes include DNA analytes such as single-stranded DNA (ssDNA), double-stranded DNA (dsDNA), genomic DNA, methylated DNA, specific methylated DNA sequences, fragmented DNA, mitochondrial DNA, *in situ* synthesized PCR products, and RNA/DNA hybrids. The DNA analyte can be a transcript of another nucleic acid molecule (e.g., DNA or RNA such as mRNA) present in a tissue sample.

Examples of nucleic acid analytes also include RNA analytes such as various types of coding and non-coding RNA. Examples of the different types of RNA analytes include messenger RNA (mRNA), including a nascent RNA, a pre-mRNA, a primary-transcript RNA, and a processed RNA, such as a capped mRNA (e.g., with a 5' 7-methyl guanosine cap), a polyadenylated mRNA (poly-A tail at the 3' end), and a spliced mRNA in which one or more introns have been removed. Also included in the analytes disclosed herein are non-capped mRNA, a non-polyadenylated mRNA, and a non-spliced mRNA. The RNA analyte can be a transcript of another nucleic acid molecule (e.g., DNA or RNA such as viral RNA) present in a tissue sample. Examples of a non-coding RNAs (ncRNA) that is not translated into a protein include transfer RNAs (tRNAs) and ribosomal RNAs (rRNAs), as well as small non-coding RNAs such as microRNA (miRNA), small interfering RNA (siRNA), Piwi-interacting RNA (piRNA), small nucleolar RNA (snoRNA), small nuclear RNA (snRNA), extracellular RNA (exRNA), small Cajal body-specific RNAs (scaRNAs), and the long ncRNAs such as Xist and HOTAIR. The RNA can be small (e.g., less than 200 nucleic acid bases in length) or large (e.g., RNA greater than 200 nucleic acid bases in length). Examples of small RNAs include 5.8S ribosomal RNA (rRNA), 5S rRNA, tRNA, miRNA, siRNA, snoRNAs, piRNA, tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16s rRNA or 23s rRNA).

In some embodiments described herein, an analyte may be a denatured nucleic acid, wherein the resulting denatured nucleic acid is single-stranded. The nucleic acid may be denatured, for example, optionally using formamide, heat, or both formamide and heat. In some embodiments, the nucleic acid is not denatured for use in a method disclosed herein.

In certain embodiments, an analyte can be extracted from a live cell. Processing conditions can be adjusted to ensure that a biological sample remains live during analysis, and analytes are extracted from (or released from) live cells of the sample. Live cell-derived analytes can be obtained only once from the sample, or can be obtained at intervals from a sample that continues to remain in viable condition.

Methods and compositions disclosed herein can be used to analyze any number of analytes. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000, at least about 100,000 or more different analytes present in a region of the sample or within an individual feature of the substrate.

In any embodiment described herein, the analyte comprises a target sequence. In some embodiments, the target sequence may be endogenous to the sample, generated in the sample, added to the sample, or associated with an analyte in the sample. In some embodiments, the target sequence is a single-stranded target sequence (e.g., a sequence in a rolling circle amplification product). In some embodiments, the analytes comprises one or more single-stranded target sequences. In one aspect, a first single-stranded target sequence is not identical to a second single-stranded target sequence. In another aspect, a first single-stranded target sequence is identical to one or more second single-stranded target sequence. In some embodiments, the one or more second single-stranded target sequence is comprised in the same analyte (e.g., nucleic acid) as the first single-stranded target sequence. Alternatively, the one or more second single-stranded target sequence is comprised in a different analyte (e.g., nucleic acid) from the first single-stranded target sequence.

### (ii) Labelling Agents

In some embodiments, provided herein are methods and compositions for analyzing endogenous analytes (e.g., RNA, ssDNA, and cell surface or intracellular proteins and/or metabolites) in a sample using one or more labelling agents. In some embodiments, an analyte labelling agent may include an agent that interacts with an analyte (e.g., an endogenous analyte in a sample). In some embodiments, the labelling agents can comprise a reporter oligonucleotide that is indicative of the analyte or portion thereof interacting with the labelling agent. For example, the reporter oligonucleotide may comprise a barcode sequence that permits identification of the labelling agent. In some cases, the sample contacted by the labelling agent can be further contacted with a probe (e.g., a single-stranded probe sequence), that hybridizes to a reporter oligonucleotide of the labelling agent, in order to identify the analyte associated with the labelling agent. In some embodiments, the analyte labelling agent comprises an analyte binding moiety and a labelling agent barcode domain comprising one or more barcode sequences, e.g., a barcode sequence that corresponds to the analyte binding moiety and/or the analyte. An analyte binding moiety barcode includes to a barcode that is associated with or otherwise identifies the analyte binding moiety. In some embodiments, by identifying an analyte binding moiety by identifying its associated analyte binding moiety barcode, the analyte to which the analyte binding moiety binds can also be identified. An analyte binding moiety barcode can be a nucleic acid sequence of a given length and/or sequence that is associated with the analyte binding moiety. An analyte binding moiety barcode can generally include any of the variety of aspects of barcodes described herein.

In some embodiments, the method comprises one or more post-fixing (also referred to as post-fixation) steps after contacting the sample with one or more labelling agents.

In the methods and systems described herein, one or more labelling agents capable of binding to or otherwise coupling to one or more features may be used to characterize analytes, cells and/or cell features. In some instances, cell features include cell surface features. Analytes may include, but are not limited to, a protein, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features may include intracellular analytes, such as proteins, protein modifications (e.g., phosphorylation status or other post-translational modifications), nuclear proteins, nuclear membrane proteins, or any combination thereof.

In some embodiments, an analyte binding moiety may include any molecule or moiety capable of binding to an analyte (e.g., a biological analyte, e.g., a macromolecular constituent). A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have coupled thereto a first reporter oligonucleotide, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat. Pub. 20190367969, which are each incorporated by reference herein in their entirety.

In some embodiments, an analyte binding moiety includes one or more antibodies or antigen binding fragments thereof. The antibodies or antigen binding fragments including the analyte binding moiety can specifically bind to a target analyte. In some embodiments, the analyte is a protein (e.g., a protein on a surface of the biological sample (e.g., a cell) or an intracellular protein). In some embodiments, a plurality of analyte labelling agents comprising a plurality of analyte binding moieties bind a plurality of analytes present in a biological sample. In some embodiments, the plurality of analytes includes a single species of analyte (e.g., a single species of polypeptide). In some embodiments in which the plurality of analytes includes a single species of analyte, the analyte binding moieties of the plurality of analyte labelling agents are the same. In some embodiments in which the plurality of analytes includes a single species of analyte, the analyte binding moieties of the plurality of analyte labelling agents are the different (e.g., members of the plurality of analyte labelling agents can have two or more species of analyte binding moieties, wherein each of the two or more species of analyte binding moieties binds a single species of analyte, e.g., at different binding sites). In some embodiments, the plurality of analytes includes multiple different species of analyte (e.g., multiple different species of polypeptides).

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide.

In some aspects, these reporter oligonucleotides may comprise nucleic acid barcode sequences that permit identification of the labelling agent which the reporter oligonucleotide is coupled to. The selection of oligonucleotides as the reporter may provide advantages of being able to generate significant diversity in terms of sequence, while also being readily attachable to most biomolecules, e.g., antibodies, etc., as well as being readily detected, e.g., using sequencing or array technologies.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment) using chemical conjugation techniques (e.g., Lightning-Link^{®} antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715, which is entirely incorporated herein by reference for all purposes. Likewise, protein and peptide biotinylation techniques have been developed and are readily available. See, e.g., U.S. Pat. No. 6,265,552, which is entirely incorporated herein by reference for all purposes. Furthermore, click reaction chemistry may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the label agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein. In some instances, the reporter oligonucleotides described herein may include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer binding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to a first oligonucleotide that is complementary (e.g., hybridizes) to a sequence of the reporter oligonucleotide.

In some embodiments, multiple different species of analytes (e.g., polypeptides) from the biological sample can be subsequently associated with the one or more physical properties of the biological sample. For example, the multiple different species of analytes can be associated with locations of the analytes in the biological sample. Such information (e.g., proteomic information when the analyte binding moiety(ies) recognizes a polypeptide(s)) can be used in association with other spatial information (e.g., genetic information from the biological sample, such as DNA sequence information, transcriptome information (i.e., sequences of transcripts), or both). For example, a cell surface protein of a cell can be associated with one or more physical properties of the cell (e.g., a shape, size, activity, or a type of the cell). The one or more physical properties can be characterized by imaging the cell. The cell can be bound by an analyte labelling agent comprising an analyte binding moiety that binds to the cell surface protein and an analyte binding moiety barcode that identifies that analyte binding moiety. Results of protein analysis in a sample (e.g., a tissue sample or a cell) can be associated with DNA and/or RNA analysis in the sample.

### (iii) Products of Endogenous Analyte and/or Labelling Agent

In some embodiments, provided herein are methods and compositions for analyzing one or more products of an endogenous analyte and/or a labelling agent in a biological sample. In some embodiments, an endogenous analyte (e.g., a viral or cellular DNA or RNA) or a product (e.g., a hybridization product, a ligation product, an extension product (e.g., by a DNA or RNA polymerase), a replication product, a transcription/reverse transcription product, and/or an amplification product such as a rolling circle amplification (RCA) product) thereof is analyzed. In some embodiments, a labelling agent that directly or indirectly binds to an analyte in the biological sample is analyzed. In some embodiments, a product (e.g., a hybridization product, a ligation product, an extension product (e.g., by a DNA or RNA polymerase), a replication product, a transcription/reverse transcription product, and/or an amplification product such as a rolling circle amplification (RCA) product) of a labelling agent that directly or indirectly binds to an analyte in the biological sample is analyzed.

### a. Hybridization

In some embodiments, a product of an endogenous analyte and/or a labelling agent is a hybridization product comprising the pairing of substantially complementary or complementary nucleic acid sequences within two different molecules, one of which is the endogenous analyte or the labelling agent (e.g., reporter oligonucleotide attached thereto). The other molecule can be another endogenous molecule or an exogenous molecule such as a probe. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

Various probes and probe sets can be hybridized to an endogenous analyte and/or a labelling agent and each probe may comprise one or more barcode sequences Exemplary barcoded probes or probe sets may be based on a padlock probe, a gapped padlock probe, a SNAIL (Splint Nucleotide Assisted Intramolecular Ligation) probe set, a PLAYR (Proximity Ligation Assay for RNA) probe set, a PLISH (Proximity Ligation *in situ* Hybridization) probe set, and RNA-templated ligation probes. The specific probe or probe set design can vary.

### b. Ligation

In some embodiments, a product of an endogenous analyte and/or a labelling agent is a ligation product. In some embodiments, the ligation product is formed between two or more endogenous analytes. In some embodiments, the ligation product is formed between an endogenous analyte and a labelling agent. In some embodiments, the ligation product is formed between two or more labelling agent. In some embodiments, the ligation product is an intramolecular ligation of an endogenous analyte. In some embodiments, the ligation product is an intramolecular ligation of a labelling agent or probe, for example, the circularization of a circularizable probe or probe set upon hybridization to a target sequence. The target sequence can be comprised in an endogenous analyte (e.g., nucleic acid such as genomic DNA or mRNA) or a product thereof (e.g., cDNA from a cellular mRNA transcript), or in a labelling agent (e.g., the reporter oligonucleotide) or a product thereof.

In some embodiments, provided herein is a probe or probe set capable of DNA-templated ligation, such as from a cDNA molecule. See, e.g., U.S. Pat. 8,551,710, which is hereby incorporated by reference in its entirety. In some embodiments, provided herein is a probe or probe set capable of RNA-templated ligation. See, e.g., U.S. Pat. Pub. 2020/0224244 which is hereby incorporated by reference in its entirety. In some embodiments, the probe set is a SNAIL probe set. See, e.g., U.S. Pat. Pub. 20190055594, which is hereby incorporated by reference in its entirety.

In some embodiments, provided herein is a multiplexed proximity ligation assay. See, e.g., U.S. Pat. Pub. 20140194311 which is hereby incorporated by reference in its entirety. In some embodiments, provided herein is a probe or probe set capable of proximity ligation, for instance a proximity ligation assay for RNA (e.g., PLAYR) probe set. See, e.g., U.S. Pat. Pub. 20160108458, which is hereby incorporated by reference in its entirety. In some embodiments, a circular probe can be indirectly hybridized to the target nucleic acid. In some embodiments, the circular construct is formed from a probe set capable of proximity ligation, for instance a proximity ligation *in situ* hybridization (PLISH) probe set. See, e.g., U.S. Pat. Pub. 2020/0224243 which is hereby incorporated by reference in its entirety.

In some embodiments, a probe such as a padlock probe may be used to analyze a reporter oligonucleotide, which may generated using proximity ligation or be subjected to proximity ligation. In some examples, the reporter oligonucleotide of a labelling agent that specifically recognizes a protein can be analyzed using *in situ* hybridization (e.g., sequential hybridization) and/or *in situ* sequencing (e.g., using padlock probes and rolling circle amplification of ligated padlock probes). Further, the reporter oligonucleotide of the labelling agent and/or a complement thereof and/or a product (e.g., a hybridization product, a ligation product, an extension product (e.g., by a DNA or RNA polymerase), a replication product, a transcription/reverse transcription product, and/or an amplification product) thereof can be recognized by another labelling agent and analyzed

In some embodiments, an analyte (a nucleic acid analyte or non-nucleic acid analyte) can be specifically bound by two labelling agents (e.g., antibodies) each of which is attached to a reporter oligonucleotide (e.g., DNA) that can participate in ligation, replication, and sequence decoding reactions, e.g., using a probe or probe set (e.g. a padlock probe, a SNAIL probe set, a circular probe, or a padlock probe and a connector). In some embodiments, the probe set may comprise two or more probe oligonucleotides, each comprising a region that is complementary to each other. For example, a proximity ligation reaction can include reporter oligonucleotides attached to pairs of antibodies that can be joined by ligation if the antibodies have been brought in proximity to each other, e.g., by binding the same target protein (complex), and the DNA ligation products that form are then used to template PCR amplification, as described for example in Söderberg et al., Methods. (2008), 45(3): 227-32, the entire contents of which are incorporated herein by reference. In some embodiments, a proximity ligation reaction can include reporter oligonucleotides attached to antibodies that each bind to one member of a binding pair or complex, for example, for analyzing a binding between members of the binding pair or complex. For detection of analytes using oligonucleotides in proximity, see, e.g., U.S. Patent Application Publication No. 2002/0051986, the entire contents of which are incorporated herein by reference. In some embodiments, two analytes in proximity can be specifically bound by two labelling agents (e.g., antibodies) each of which is attached to a reporter oligonucleotide (e.g., DNA) that can participate, when in proximity when bound to their respective targets, in ligation, replication, and/or sequence decoding reactions

In some embodiments, one or more reporter oligonucleotides (and optionally one or more other nucleic acid molecules such as a connector) aid in the ligation of the probe. Upon ligation, the probe may form a circularized probe. In some embodiments, one or more suitable probes can be used and ligated, wherein the one or more probes comprise a sequence that is complementary to the one or more reporter oligonucleotides (or portion thereof). The probe may comprise one or more barcode sequences. In some embodiments, the one or more reporter oligonucleotide may serve as a primer for rolling circle amplification (RCA) of the circularized probe. In some embodiments, a nucleic acid other than the one or more reporter oligonucleotide is used as a primer for rolling circle amplification (RCA) of the circularized probe. For example, a nucleic acid capable of hybridizing to the circularized probe at a sequence other than sequence(s) hybridizing to the one or more reporter oligonucleotide can be used as the primer for RCA. In other examples, the primer in a SNAIL probe set is used as the primer for RCA.

In some embodiments, one or more analytes can be specifically bound by two primary antibodies, each of which is in turn recognized by a secondary antibody each attached to a reporter oligonucleotide (e.g., DNA). Each nucleic acid molecule can aid in the ligation of the probe to form a circularized probe. In some instances, the probe can comprise one or more barcode sequences. Further, the reporter oligonucleotide may serve as a primer for rolling circle amplification of the circularized probe. The nucleic acid molecules, circularized probes, and RCA products can be analyzed using any suitable method disclosed herein for *in situ* analysis.

In some embodiments, the ligation involves chemical ligation. In some embodiments, the ligation involves template dependent ligation. In some embodiments, the ligation involves template independent ligation. In some embodiments, the ligation involves enzymatic ligation.

In some embodiments, the enzymatic ligation involves use of a ligase. In some aspects, the ligase used herein comprises an enzyme that is commonly used to join polynucleotides together or to join the ends of a single polynucleotide. An RNA ligase, a DNA ligase, or another variety of ligase can be used to ligate two nucleotide sequences together. Ligases comprise ATP-dependent double-strand polynucleotide ligases, NAD-i-dependent double-strand DNA or RNA ligases and single-strand polynucleotide ligases, for example any of the ligases described in EC 6.5.1.1 (ATP-dependent ligases), EC 6.5.1.2 (NAD+-dependent ligases), EC 6.5.1.3 (RNA ligases). Specific examples of ligases comprise bacterial ligases such as E. coli DNA ligase, Tth DNA ligase, Thermococcus sp. (strain 9° N) DNA ligase (9°N^{™} DNA ligase, New England Biolabs), Taq DNA ligase, Ampligase^{™} (Epicentre Biotechnologies) and phage ligases such as T3 DNA ligase, T4 DNA ligase and T7 DNA ligase and mutants thereof. In some embodiments, the ligase is a T4 RNA ligase. In some embodiments, the ligase is a splintR ligase. In some embodiments, the ligase is a single stranded DNA ligase. In some embodiments, the ligase is a T4 DNA ligase. In some embodiments, the ligase is a ligase that has an DNA-splinted DNA ligase activity. In some embodiments, the ligase is a ligase that has an RNA-splinted DNA ligase activity.

In some embodiments, the ligation herein is a direct ligation. In some embodiments, the ligation herein is an indirect ligation. "Direct ligation" means that the ends of the polynucleotides hybridize immediately adjacently to one another to form a substrate for a ligase enzyme resulting in their ligation to each other (intramolecular ligation). Alternatively, "indirect" means that the ends of the polynucleotides hybridize non-adjacently to one another, i.e., separated by one or more intervening nucleotides or "gaps". In some embodiments, said ends are not ligated directly to each other, but instead occurs either via the intermediacy of one or more intervening (so-called "gap" or "gap-filling" (oligo)nucleotides) or by the extension of the 3' end of a probe to "fill" the "gap" corresponding to said intervening nucleotides (intermolecular ligation). In some cases, the gap of one or more nucleotides between the hybridized ends of the polynucleotides may be "filled" by one or more "gap" (oligo)nucleotide(s) which are complementary to a splint, padlock probe, or target nucleic acid. The gap may be a gap of 1 to 60 nucleotides or a gap of 1 to 40 nucleotides or a gap of 3 to 40 nucleotides. In specific embodiments, the gap may be a gap of about 1, 2, 3, 4, 5, 6, 7, 8, 9 or 10 or more nucleotides, of any integer (or range of integers) of nucleotides in between the indicated values. In some embodiments, the gap between said terminal regions may be filled by a gap oligonucleotide or by extending the 3' end of a polynucleotide. In some cases, ligation involves ligating the ends of the probe to at least one gap (oligo)nucleotide, such that the gap (oligo)nucleotide becomes incorporated into the resulting polynucleotide. In some embodiments, the ligation herein is preceded by gap filling. In other embodiments, the ligation herein does not require gap filling.

In some embodiments, ligation of the polynucleotides produces polynucleotides with melting temperature higher than that of unligated polynucleotides. Thus, in some aspects, ligation stabilizes the hybridization complex containing the ligated polynucleotides prior to subsequent steps, comprising amplification and detection.

In some aspects, a high fidelity ligase, such as a thermostable DNA ligase (e.g., a *Taq* DNA ligase), is used. Thermostable DNA ligases are active at elevated temperatures, allowing further discrimination by incubating the ligation at a temperature near the melting temperature (Tₘ) of the DNA strands. This selectively reduces the concentration of annealed mismatched substrates (expected to have a slightly lower Tₘ around the mismatch) over annealed fully base-paired substrates. Thus, high-fidelity ligation can be achieved through a combination of the intrinsic selectivity of the ligase active site and balanced conditions to reduce the incidence of annealed mismatched dsDNA.

In some embodiments, the ligation herein is a proximity ligation of ligating two (or more) nucleic acid sequences that are in proximity with each other, e.g., through enzymatic means (e.g., a ligase). In some embodiments, proximity ligation can include a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Patent No. 7,264,929, the entire contents of which are incorporated herein by reference). A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation.

### c. Primer Extension and Amplification

In some embodiments, a product is a primer extension product of an analyte, a labelling agent, a probe or probe set bound to the analyte (e.g., a padlock probe bound to genomic DNA, mRNA, or cDNA), or a probe or probe set bound to the labelling agent (e.g., a padlock probe bound to one or more reporter oligonucleotides from the same or different labelling agents).

A primer is generally a single-stranded nucleic acid sequence having a 3' end that can be used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases. A primer, may in some cases, refer to a primer binding sequence. A primer extension reaction generally refers to any method where two nucleic acid sequences become linked (e.g., hybridized) by an overlap of their respective terminal complementary nucleic acid sequences (i.e., for example, 3' termini). Such linking can be followed by nucleic acid extension (e.g., an enzymatic extension) of one, or both termini using the other nucleic acid sequence as a template for extension. Enzymatic extension can be performed by an enzyme including, but not limited to, a polymerase and/or a reverse transcriptase.

In some embodiments, a product of an endogenous analyte and/or a labelling agent is an amplification product of one or more polynucleotides, for instance, a circular probe or circularizable probe or probe set. In some embodiments, the amplifying is achieved by performing rolling circle amplification (RCA). In other embodiments, a primer that hybridizes to the circular probe or circularized probe is added and used as such for amplification. In some embodiments, the RCA comprises a linear RCA, a branched RCA, a dendritic RCA, or any combination thereof.

In some embodiments, the amplification is performed at a temperature between or between about 20°C and about 60°C. In some embodiments, the amplification is performed at a temperature between or between about 30°C and about 40°C. In some aspects, the amplification step, such as the rolling circle amplification (RCA) is performed at a temperature between at or about 25°C and at or about 50°C, such as at or about 25°C, 27°C, 29°C, 31°C, 33°C, 35°C, 37°C, 39°C, 41°C, 43°C, 45°C, 47°C, or 49°C.

In some embodiments, upon addition of a DNA polymerase in the presence of appropriate dNTP precursors and other cofactors, a primer is elongated to produce multiple copies of the circular template. This amplification step can utilize isothermal amplification or non-isothermal amplification. In some embodiments, after the formation of the hybridization complex and association of the amplification probe, the hybridization complex is rolling-circle amplified to generate a cDNA nanoball (i.e., amplicon) containing multiple copies of the cDNA. Techniques for rolling circle amplification (RCA) are known in the art such as linear RCA, a branched RCA, a dendritic RCA, or any combination thereof. (See, e.g., Baner et al, Nucleic Acids Research, 26:5073-5078, 1998; Lizardi et al, Nature Genetics 19:226, 1998; Mohsen et al., Acc Chem Res. 2016 November 15; 49(11): 2540-2550; Schweitzer et al. Proc. Natl Acad. Sci. USA 97:101 13- 1 19, 2000; Faruqi et al, BMC Genomics 2:4, 2000; Nallur et al, Nucl. Acids Res. 29:el 18, 2001; Dean et al. Genome Res. 1 1 :l095- 1099, 2001; Schweitzer et al, Nature Biotech. 20:359-365, 2002; U.S. Patent Nos. 6,054,274, 6,291,187, 6,323,009, 6,344,329 and 6,368,801). Exemplary polymerases for use in RCA comprise DNA polymerase such phi29 (ϕ29) polymerase, Klenow fragment, *Bacillus stearothermophilus* DNA polymerase (BST), T4 DNA polymerase, T7 DNA polymerase, or DNA polymerase I. In some aspects, DNA polymerases that have been engineered or mutated to have desirable characteristics can be employed. In some embodiments, the polymerase is phi29 DNA polymerase.

In some aspects, during the amplification step, modified nucleotides can be added to the reaction to incorporate the modified nucleotides in the amplification product (e.g., nanoball). Exemplary of the modified nucleotides comprise amine-modified nucleotides. In some aspects of the methods, for example, for anchoring or cross-linking of the generated amplification product (e.g., nanoball) to a scaffold, to cellular structures and/or to other amplification products (e.g., other nanoballs). In some aspects, the amplification products comprises a modified nucleotide, such as an amine-modified nucleotide. In some embodiments, the amine-modified nucleotide comprises an acrylic acid N-hydroxysuccinimide moiety modification. Examples of other amine-modified nucleotides comprise, but are not limited to, a 5-Aminoallyl-dUTP moiety modification, a 5-Propargylamino-dCTP moiety modification, a N6-6-Aminohexyl-dATP moiety modification, or a 7-Deaza-7-Propargylamino-dATP moiety modification.

In some aspects, the polynucleotides and/or amplification product (e.g., amplicon) can be anchored to a polymer matrix. For example, the polymer matrix can be a hydrogel. In some embodiments, one or more of the polynucleotide probe(s) can be modified to contain functional groups that can be used as an anchoring site to attach the polynucleotide probes and/or amplification product to a polymer matrix. Exemplary modification and polymer matrix that can be employed in accordance with the provided embodiments comprise those described in, for example, US 2021/0324450, US 2021/0363579, US 2016/0024555, US 2018/0251833 and US 2017/0219465. In some examples, the scaffold also contains modifications or functional groups that can react with or incorporate the modifications or functional groups of the probe set or amplification product. In some examples, the scaffold can comprise oligonucleotides, polymers or chemical groups, to provide a matrix and/or support structures.

The amplification products may be immobilized within the matrix generally at the location of the nucleic acid being amplified, thereby creating a localized colony of amplicons. The amplification products may be immobilized within the matrix by steric factors. The amplification products may also be immobilized within the matrix by covalent or noncovalent bonding. In this manner, the amplification products may be considered to be attached to the matrix. By being immobilized to the matrix, such as by covalent bonding or cross-linking, the size and spatial relationship of the original amplicons is maintained. By being immobilized to the matrix, such as by covalent bonding or cross-linking, the amplification products are resistant to movement or unraveling under mechanical stress.

In some aspects, the amplification products are copolymerized and/or covalently attached to the surrounding matrix thereby preserving their spatial relationship and any information inherent thereto. For example, if the amplification products are those generated from DNA or RNA within a cell embedded in the matrix, the amplification products can also be functionalized to form covalent attachment to the matrix preserving their spatial information within the cell thereby providing a subcellular localization distribution pattern. In some embodiments, the provided methods involve embedding the one or more polynucleotide probe sets and/or the amplification products in the presence of hydrogel subunits to form one or more hydrogel-embedded amplification products. In some embodiments, the hydrogel-tissue chemistry described comprises covalently attaching nucleic acids to in situ synthesized hydrogel for tissue clearing, enzyme diffusion, and multiple-cycle sequencing while an existing hydrogel-tissue chemistry method cannot. In some embodiments, to enable amplification product embedding in the tissue-hydrogel setting, amine-modified nucleotides are comprised in the amplification step (e.g., RCA), functionalized with an acrylamide moiety using acrylic acid N-hydroxysuccinimide esters, and copolymerized with acrylamide monomers to form a hydrogel.

In some embodiments, the RCA template may comprise the target analyte, or a part thereof, where the target analyte is a nucleic acid, or it may be provided or generated as a proxy, or a marker, for the analyte. As noted above, many assays are known for the detection of numerous different analytes, which use a RCA-based detection system, e.g., where the signal is provided by generating a RCP from a circular RCA template which is provided or generated in the assay, and the RCP is detected to detect the analyte. The RCP may thus be regarded as a reporter which is detected to detect the target analyte. However, the RCA template may also be regarded as a reporter for the target analyte; the RCP is generated based on the RCA template, and comprises complementary copies of the RCA template. The RCA template determines the signal which is detected, and is thus indicative of the target analyte. As will be described in more detail below, the RCA template may be a probe, or a part or component of a probe, or may be generated from a probe, or it may be a component of a detection assay (i.e. a reagent in a detection assay), which is used as a reporter for the assay, or a part of a reporter, or signal-generation system. The RCA template used to generate the RCP may thus be a circular (e.g. circularized) reporter nucleic acid molecule, namely from any RCA-based detection assay which uses or generates a circular nucleic acid molecule as a reporter for the assay. Since the RCA template generates the RCP reporter, it may be viewed as part of the reporter system for the assay.

In some embodiments, a product herein includes a molecule or a complex generated in a series of reactions, e.g., hybridization, ligation, extension, replication, transcription/reverse transcription, and/or amplification (e.g., rolling circle amplification), in any suitable combination. For example, a product comprising a target sequence for a probe disclosed herein (e.g., a morpholino-based probe) may be a hybridization complex formed of a cellular nucleic acid in a sample and an exogenously added nucleic acid probe. The exogenously added nucleic acid probe may comprise an overhang that does not hybridize to the cellular nucleic acid but hybridizes to another probe (e.g., a detectably labelled probe such as a morpholino-based probe, or a circularizable probe or probe set). The exogenously added nucleic acid probe may be optionally ligated to a cellular nucleic acid molecule or another exogenous nucleic acid molecule. In other examples, a product comprising a target sequence for a probe disclosed herein (e.g., a morpholino-based probe) may an RCP of a circularizable probe or probe set which hybridizes to a cellular nucleic acid molecule (e.g., genomic DNA or mRNA) or product thereof (e.g., a transcript such as cDNA, a DNA-templated ligation product of two probes, or an RNA-templated ligation product of two probes). In other examples, a product comprising a target sequence for a probe disclosed herein (e.g., a morpholino-based probe) may a probe hybridizing to an RCP. The probe may comprise an overhang that does not hybridize to the RCP but hybridizes to another probe (e.g., a detectably labelled probe such as a morpholino-based probe). The probe may be optionally ligated to a cellular nucleic acid molecule or another probe, e.g., an anchor probe that hybridize to the RCP.

### C. Target Sequences

A target sequence for a probe disclosed herein (e.g., a morpholino-based detection probe and/or intermediate probe) may be comprised in any analyte disclose herein, including an endogenous analyte (e.g., a viral or cellular nucleic acid), a labelling agent (or reporter oligonucleotide attached thereto), or a product of or associated with an endogenous analyte and/or a labelling agent (or reporter oligonucleotide attached thereto). In some aspects, at least a portion of the target sequence and sequence comprised by the probe disclosed herein (e.g., a morpholino-based detection probe and/or intermediate probe) are complementary and capable of hybridization.

In some aspects, one or more of the target sequences includes one or more barcode(s), e.g., at least two, three, four, five, six, seven, eight, nine, ten, or more barcodes. Barcodes can spatially-resolve molecular components found in biological samples, for example, within a cell or a tissue sample. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI"). In some aspects, a barcode comprises about 4, 5, 6, 7, 8, 9, 10, 11, 12, 13, 14, 15, 16, 17, 18, 19, 20, 21, 22, 23, 24, 25, 26, 27, 28, 29, 30, or more than 30 nucleotides.

In some embodiments, a barcode includes two or more sub-barcodes that together function as a single barcode. For example, a polynucleotide barcode can include two or more polynucleotide sequences (e.g., sub-barcodes) that are separated by one or more non-barcode sequences. In some embodiments, the one or more barcode(s) can also provide a platform for targeting functionalities, such as oligonucleotides, oligonucleotide-antibody conjugates, oligonucleotide-streptavidin conjugates, modified oligonucleotides, affinity purification, detectable moieties, enzymes, enzymes for detection assays or other functionalities, and/or for detection and identification of the polynucleotide.

Sequence analysis of the nucleic acid molecules (including barcoded nucleic acid molecules or derivatives thereof) can be direct or indirect. Thus, the sequence analysis substrate (which can be viewed as the molecule which is subjected to the sequence analysis step or process) can directly be the barcoded nucleic acid molecule (e.g., a padlock probe) or it can be a molecule which is derived therefrom (e.g., a complement thereof, such as a RCA product).

Barcodes may be analyzed, detected, or sequenced using any suitable method disclosed herein or known in the art. In some embodiments, a N-mer barcode sequence comprises 4^{N} complexity given a sequencing read of N bases, and a much shorter sequencing read may be required for molecular identification compared to non-barcode sequencing methods such as direct sequencing. For example, 1024 molecular species may be identified using a 5-nucleotide barcode sequence (4⁵=1024), whereas 8 nucleotide barcodes can be used to identify up to 65,536 molecular species, a number greater than the total number of distinct genes in the human genome. In some embodiments, the barcode sequences contained in the probes or RCPs are detected, rather than endogenous sequences, which can be an efficient read-out in terms of information per cycle of sequencing. Because the barcode sequences are pre-determined, they can also be designed to feature error detection and correction mechanisms, see, e.g., US 2019/0055594 and US 2021/0164039, which are hereby incorporated by reference in their entirety.

Barcodes (e.g., primary and/or secondary barcode sequences) can be analyzed (e.g., detected or sequenced) using any suitable methods or techniques, including those described herein, such as RNA sequential probing of targets (RNA SPOTs), sequential fluorescent *in situ* hybridization (seqFISH), single-molecule fluorescent *in situ* hybridization (smFISH), multiplexed error-robust fluorescence *in situ* hybridization (MERFISH), *in situ* sequencing, hybridization-based *in situ* sequencing (HybISS), targeted *in situ* sequencing, fluorescent *in situ* sequencing (FISSEQ), sequencing by synthesis (SBS), sequencing by ligation (SBL), sequencing by hybridization (SBH), or spatially-resolved transcript amplicon readout mapping (STARmap).

In some embodiments, sequence analysis of the target and/or barcoded probes can be performed by sequential hybridization (e.g., sequencing by hybridization and/or sequential *in situ* fluorescence hybridization). Sequential fluorescence hybridization can involve sequential hybridization of detection probes comprising an oligonucleotide and a detectable label. In some embodiments, a method disclosed herein comprises sequential hybridization of nucleic acid analog probes disclosed herein, including detectably labeled probes (e.g., fluorophore conjugated oligonucleotides) and/or probes that are not detectably labeled *per se* but are capable of binding (e.g., via nucleic acid hybridization) and being detected by detectably labeled probes. Exemplary methods comprising sequential fluorescence hybridization of detectable probes are described in US 2019/0161796, US 2020/0224244, US 2022/0010358, US 2021/0340618, and WO 2021/138676, all of which are incorporated herein by reference.

Other examples of methods for sequencing genetic material include, but are not limited to, DNA hybridization methods (e.g., Southern blotting), restriction enzyme digestion methods, Sanger sequencing methods, next-generation sequencing methods (e.g., single-molecule real-time sequencing, nanopore sequencing, and Polony sequencing), ligation methods, and microarray methods. Additional examples of sequencing methods that can be used include targeted sequencing, single molecule real-time sequencing, exon sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing, MS-PET sequencing, and any combinations thereof.

### III. In situ Analysis

### A. Probes and Probe Hybridization

Annealing of oligonucleotide probes to target analytes (e.g., RNA or DNA) is essential when performing hybridization assays (e.g., *in situ* hybridization assays). In some embodiments, a method described herein comprises using a detection probe, wherein the probe comprises a sequence of nucleobases on a synthetic backbone, and the probe directly or indirectly hybridizes to a target sequence in the DNA concatemer (e.g., RCA). In some embodiments, the probe (e.g., detection probe and/or intermediate probe) comprises a single-stranded probe sequence. In some embodiments, the probe comprises a single-stranded region and optionally a double-stranded region. In some embodiments, the probe is an oligonucleotide. In some embodiments, at least some, each, or all of a plurality of detection probes are morpholino-based detection probes.

The target analyte may be any target molecule, including nucleic acid molecules, or analytes other than nucleic acid molecules, such as a proteins, peptides, carbohydrates etc. Various methods based upon detecting such a reporter target nucleic acid sequence, which is indicative of an underlying target analyte, are well described in the art, including, for example, immunoRCA and immunoPCR as noted above, and assays using padlock probes or proximity probes. The use of proximity probes comprising analyte-binding domains and nucleic acid domains which interact upon binding of the probes to a target analyte is widely known and described in the literature. In the context of proximity probes, a target nucleic acid sequence may be generated by extension or ligation of nucleic acid domains of proximity probes, or of additional nucleic acid molecules (e.g. oligonucleotides) which hybridize to the nucleic acid domain of a proximity probe. In the context of a padlock probe, a target nucleic acid sequence may be generated as the RCP of that probe, or indeed simply a result of ligation of the probe. In the context of immunoPCR or immunoRCA, a PCR or RCA product may be generated to provide a target nucleic acid sequence. Still further, a target nucleic acid sequence may be added to the sample as the nucleic acid domain of an analyte-binding probe or a labelling agent.

For example, a protein or other analyte in a sample may be detected by a labelling agent (e.g., an antibody or other analyte-specific binding partner), which is provided with an oligonucleotide (e.g., reporter oligonucleotide), and that oligonucleotide may be considered to be or comprise a target nucleic acid sequence. In this case, the hybridization probes may be designed to hybridize to that oligonucleotide, such that the oligonucleotide can be detected, and therefore can indicate the presence of the antibody, and hence the analyte. Similarly, an oligonucleotide sequence may be generated as part of an analyte detection assay, e.g. an extension or ligation product may be generated as the result of a proximity assay, and this oligonucleotide may be considered to be a target nucleic acid sequence.

In some embodiments, a probe (e.g., detection probe and/or intermediate probe) herein directly hybridizes to the target sequence. In alternative embodiments, the probe indirectly hybridizes to the target sequence In some embodiments, the hybridization occurs *in situ.* For example, the probe may hybridize to an intermediate probe (e.g., secondary probe, tertiary probe, etc.) which directly or indirectly hybridizes to the target sequence. The detection probe may directly hybridize to a first sequence of an intermediate probe, and the intermediate probe comprises a second sequence that directly hybridizes to the target sequence In some embodiments, the probe can comprise one or more sequences of nucleobases on a synthetic backbone and/or one or more sequences of nucleobases on a sugar-phosphate backbone, where the sequences can be arranged in any suitable order in the probe. In some embodiments, the sequence of nucleobases on the synthetic backbone is a first probe sequence, and the probe can further comprise a second probe sequence comprising nucleobases on a sugar-phosphate backbone. The first probe sequence may be 5' or 3' to the second probe sequence. In some embodiments, the probes comprises one or more first probe sequences and/or one or more second probe sequences. In some embodiments, the second probe sequence is a DNA sequence, an RNA sequence, or a combination thereof.

Direct or indirect *in situ* hybridization of the probe (e.g., detection probe and/or intermediate probe) to the DNA concatemer may occur in the presence of a hybridization buffer. The hybridization buffer may provide specificity of binding (e.g., between the DNA concatemer and the probe) while preserving the detection signal strength *in situ.* In some embodiments, the hybridization buffer comprises a reagent that increases the stability of the DNA concatamer. In some embodiments, the hybridization buffer comprises a reagent that increases the stability of the probe. In some embodiments, the hybridization buffer comprises a reagent that increases the stability of the intermolecular hybridization between the DNA concatemer and the probe. In some embodiments, the hybridization buffer comprises a reagent that selectively increases the stability of A:T base pairs. For example, the stability of the A:T base pairs may be increased to approximately the stability of G:C base pairs. In some embodiments, the hybridization buffer comprises a reagent that selectively increases the stability of A:T base pairs between the DNA concatamer and the probe. In some embodiments, the hybridization buffer reduces potential base pair mismatches (such as between the DNA concatamer and the probe) In some embodiments, the hybridization buffer improves the stringency in hybridization reaction. In some embodiments, the hybridization buffer reduces background signal (e.g., non-specific binding). In some embodiments, the hybridization buffer reduces non-specific binding of the probe to the DNA concatamer.

Exemplary reagents for use in the hybridization buffers described herein may include, but are not limited to, water, various non-ionic detergents, saline-sodium citrate (SSC), sodium phosphate, phosphate buffered saline (PBS), sodium dodecyl sulfate (SDS), bovine serum albumin (BSA), ethylenediaminetetracetic acid (EDTA), a sarkosyl compound (e.g., sodium lauroyl sarcosinate; sarkosyl, ammonium salt; or sarkosyl, potassium salt), tris(hydroxymethyl)aminomethane (tris), tris-HCl (tris hydrochloride), 3-morpholinopropane-1-sulfonic acid (MOPS), TAE buffer (tris EDTA), TBS buffer (tris buffered saline), bis-tris methane, 4-(2-hydroxyethyl)-1-piperazineethanesulfonic acid (HEPES buffer), dimethyl sulfoxide (DMSO), quaternary ammonium salts (e.g., tetramethylammonium chloride (TMAC)), trimethybenzylammonium chloride (TMBAC), tetraethylphosphonium chloride (TEPC), triethylbenzylammonium chloride (TEBAC), tetra-n-propylammonium chloride (TPAC), tri-n-butylbenzylammonium chloride (TBBAC), tetra-n-butylphosphonium chloride (TBPC), (3-((3-cholamidopropyl) dimethylammonio)-1-propanesulfonate) (3-((3-cholamidopropyl) dimethylammonio)-1-propanesulfonate) (CHAPS detergent), and choline dihydrogen phosphate (choline DHP). Exemplary reagents for use in the hybridization buffers described herein may include a zwitterionic detergent. Exemplary reagents for use in the hybridization buffers described herein may include a reagent suited for protecting the native state of analytes such as proteins in a biological sample.

In some embodiments, the hybridization buffer comprises ammonium ions. In some instances, the hybridization buffer comprises quaternary ammonium ions. In some embodiments, the hybridization buffer comprises TMAC. In some embodiments, the concentration of TMAC in the hybridization buffer is between 0.5X TMAC and 3X TMAC, such as between any of 3X TMAC and 2X TMAC, 2.5X TMAC and 1.5X TMAC, or 1X TMAC and 0.5X TMAC. In some embodiments, the hybridization buffer comprises between about 0.1M and about 3M TMAC, such as between any of about 0.1M and about 1M TMAC, about 0.5M and about 1.5M TMAC, and about 1M and about 3M TMAC. In some embodiments, the hybridization buffer comprises greater than about 0.1M TMAC, such as greater than any of about 0.2M TMAC, 0.3M TMAC, 0.4M TMAC, 0.5M TMAC, 0.6M TMAC, 0.7M TMAC, 0.8M TMAC, 0.9M TMAC, 1M TMAC, 1.5M TMAC, 2M TMAC, 2.5M TMAC, 3M TMAC, or more. In some embodiments, the hybridization buffer comprises less than about 3M TMAC, such as less than any of about 2.5M TMAC, 2M TMAC, 1.5M TMAC, 1M TMAC, 0.9M TMAC, 0.8M TMAC, 0.7M TMAC, 0.6M TMAC, 0.5M TMAC, 0.4M TMAC 0.3M TMAC, 0.2M TMAC, 0.1M TMAC, or less. In some embodiments, the hybridization buffer comprises 1M TMAC.

In some embodiments, the probe (e.g., detection probe and/or intermediate probe) is between about 5 and about 10, between about 10 and about 20, between about 20 and about 30, or between about 30 and about 40 nucleobases in length. In some embodiments, the probe is about 5, 10, 15, 20, 25, 30, 40, 45, or 50 nucleobases in length. In some embodiments, the probe is about 20 nucleobases in length. In some embodiments, the probe comprises nucleobases A, T, C, G, or U, or variants thereof, in any suitable order and combination. The probe may comprise suitably arranged A, T, C, G, or U, bases for Watson-Crick base pairing directly or indirectly to the DNA concatemer. In some embodiments, the probe comprises phosphorodiamidate linkages in the synthetic backbone. For example, the probe may contain uncharged phosphorodiamidate inter-subunit linkages instead of the anionic phosphodiester linkage found in natural nucleic acids. In some embodiments, the synthetic backbone of the probe comprises methylenemorpholine rings, which replace the deoxyribose (or ribose) rings characteristic of DNA and RNA oligonucleotide-based detection probes. In some embodiments, the probe comprises morpholine rings in the synthetic backbone. Preferably, the probe is a morpholino-based detection probe **(****FIG. 1A****).** In some embodiments, at least some, each, or all of a plurality of detection probes are morpholino-based detection probes.

In some embodiments, the probe (e.g., detection probe and/or intermediate probe) is less electrostatically negative than a reference probe having the same sequence of nucleobases on a sugar-phosphate backbone. The probe may be uncharged, or alternatively the probe may be positively charged. For example, the probe may comprise a synthetic backbone that is insensitive to anionic charge or other electrostatic properties of the DNA concatemer.

In some embodiments, the probe (e.g., detection probe and/or intermediate probe) further comprises a detectable moiety that is optionally modifiable. In some embodiments, the probe further comprises one or more detectable moieties. In some embodiments, the detectable moiety is for direct chemical, optical, or enzymatic detection In some embodiments, the detectable moiety is a chemical moiety for direct chemical, optical, or enzymatic detection or modification. In some embodiments, the detectable moiety is a chemical moiety for downstream chemical, optical, or enzymatic detection. In some embodiments, the detectable moiety is a chemical moiety for downstream chemical, optical, or enzymatic modification. For example, the detection probe may be detectably labeled (e.g., fluorescently labeled, *see* **FIG. 1B**). In some embodiments, fluorescently labeled detection probe is uncharged or less electrostatically negative than a reference detection probe having the same fluorescent label and sequence of nucleobases on a sugar-phosphate backbone. In some embodiments, the detectable moiety is conjugated to the 3' termini of the probe. In some embodiments, the detectable moiety is conjugated to the 5' termini of the probe.

In some aspects, provided herein is a method comprising contacting a target nucleic acid sequence with an intermediate probe (e.g., secondary probe, tertiary probe, etc.) which directly or indirectly hybridizes to the target sequence. The intermediate probe can be a nucleic acid analog probe (e.g., a morpholino-based probe) less electrostatically negative than a reference intermediate probe having the same sequence of nucleobases on a sugar-phosphate backbone. In some embodiments, the target nucleic acid sequence is a cellular nucleic acid sequence, such as a genomic DNA sequence or a cellular RNA sequence. In some embodiments, the target nucleic acid sequence is produced from an endogenous nucleic acid sequence (such as cDNA from mRNA) *in situ* in a sample comprising cells. In some embodiments, a primary probe binds directly to a cellular target sequence and can be used to generate a product thereof, such as an amplification (RCA) product. An intermediate probe sequence may be complementary to the target sequence (e.g., a single-stranded target sequence), allowing hybridization of the intermediate probe to the target sequence.

In some embodiments, a secondary probe is a nucleic acid analog probe (e.g., a morpholino-based probe), and the secondary probe is used to bind a primary probe or a portion thereof, e.g., a single-stranded overhang that does not hybridize to a target nucleic acid. In some embodiments, a tertiary probe is a nucleic acid analog probe (e.g., a morpholino-based probe), and the tertiary probe is used to bind a secondary probe or a portion thereof, e.g., a single-stranded overhang that does not hybridize to a primary probe. In some embodiments, additional nucleic acid analog probes (e.g., a morpholino-based probe as a quaternary probe) may be provided.

In some embodiments, intermediate probes (e.g., a secondary probe, tertiary probe, and/or higher order intermediate probes), which are nucleic acid analog probes (e.g., morpholino-based probes), are directly or indirectly labelled. In some embodiments, any one or more or all of the intermediate probes can be covalently conjugated to a detectable moiety. In some embodiments, any one or more or all of the intermediate probes is covalently conjugated to a chemical moiety. In some embodiments, any one or more or all of the intermediate probes can be a nucleic acid analog probe (e.g., a morpholino-based probe) and may be used as a detection oligonucleotide (e.g., detectably labeled probe). In some embodiments, a primary probe is detectably labeled, for example, while its target nucleic acid is not detectably labeled. In some embodiments, a morpholino-based secondary probe is detectably labeled, for example, while the primary probe to which the secondary probe binds is not detectably labeled. In some embodiments, a morpholino-based tertiary probe is detectably labeled, for example, while the secondary probe and/or primary probe is not detectably labeled.

In any of the preceding embodiments, a morpholino-based intermediate probe (e.g., a secondary probe, tertiary probe, and/or higher order intermediate probes), and/or detection probes may be a barcoded probe or barcoded probe set.

In some aspects, one or more of the nucleic acid analog probes (e.g., morpholino-based detection and/or intermediate probes) comprise one or more barcode(s), e.g., at least two, three, four, five, six, seven, eight, nine, ten, or more barcodes. In some aspects, one or more of the nucleic acid analog probes (e.g., morpholino-based detection and/or intermediate probes) comprise a sequence complementary to one or more barcode(s). Barcodes can spatially-resolve molecular components found in biological samples, for example, within a cell or a tissue sample. In some embodiments, a barcode includes two or more sub-barcodes that together function as a single barcode. For example, a polynucleotide barcode can include two or more polynucleotide sequences (e.g., sub-barcodes) that are separated by one or more non-barcode sequences. In some embodiments, the one or more barcode(s) can also provide a platform for targeting functionalities, such as oligonucleotides, oligonucleotide-antibody conjugates, oligonucleotide-streptavidin conjugates, modified oligonucleotides, affinity purification, detectable moieties, enzymes, enzymes for detection assays or other functionalities, and/or for detection and identification of the polynucleotide. In some aspects, the nucleic acid analog probes (e.g., morpholino-based detection and/or intermediate probes) can be used to detect barcode(s) (e.g., by hybridizing to a barcode sequence).

In some of any of the preceding embodiments, the methods further comprise forming an amplification product using a circular polynucleotide as a template. In some embodiments, the amplification product is formed using isothermal amplification or non-isothermal amplification. In some of any of the preceding embodiments, the amplification product is formed using rolling circle amplification (RCA). In some embodiments, the RCA comprises a linear RCA, a branched RCA, a dendritic RCA, or any combination thereof. In some embodiments, one or more of the nucleic acid analog probes (e.g., morpholino-based probes) are used to bind a single-stranded RCA product which comprises a sugar-phosphate backbone.

In some embodiments, the DNA concatemer resulting from amplification has a diameter of between about 0.1 µm and about 0.5 µm, such as between about 0.2 µm and about 0.3 µm or between about 0.3 µm and about 0.4 µm, between about 0.5 µm and about 1 µm, between about 1 µm and about 1.5 µm, or between about 0.1 µm and about 3 µm . In some embodiments, the DNA concatemer has a diameter of about 0.1 µm, 0.2 µm, 0.3 µm, 0.4 µm, 0.5 µm, 0.6 µm, 0.7 µm, 0.8 µm, 0.9 µm, 1 µm, 1.1 µm, 1.2 µm, 1.3 µm, 1.4 µm, 1.5 µm, 1.6 µm, 1.7 µm, 1.8 µm, 1.9 µm, 2 µm, 2.5 µm, or 3 µm, or a diameter in a range between any of the aforementioned values. Exemplary DNA concatemers such as RCA products are disclosed in Deng et al., Chem, 4(6): 1373-1386 (2018) and Hu and Kuang, Chem, 4(6): 1194-1196 (2018), incorporated herein by reference in their entirety.

In some aspects, provided herein is a method of a method of analyzing a biological sample, comprising: binding a plurality of detection probes directly or indirectly to a complex comprising a plurality of DNA concatemers in the biological sample, wherein a detection probe of the plurality of detection probes comprises a sequence of nucleobases on a synthetic backbone, and the detection probe directly or indirectly binds to a target sequence in any one or more of the DNA concatemers in the complex, and detecting signals from said plurality of bound detection probes, where the signal generated is greater than a plurality of reference detection probes having the same sequence of nucleobases on a sugar-phosphate backbone. In some embodiments, the plurality of DNA concatemers in the same complex are RCA products of the same analyte (e.g., a DNA or RNA molecule of the same gene of interest) or products of probes targeting the same analyte. In some examples, the RCA products can be generated using probes or probe sets (e.g., circular or padlock probes or SNAIL probe sets) that hybridize to the same nucleic acid molecule but at different sequences within the same molecule. In some embodiments, the DNA concatemer complex comprises two, three, four, five, or more DNA concatemers. In some embodiments, the DNA concatemer complex has a diameter between about 0.5 µm and about 1 µm, between about 1 µm and about 1.5 µm, or between about 0.1 µm and about 3 µm. In some embodiments, the DNA concatemer complex has a diameter of about 0.5 µm, 0.6 µm, 0.7 µm, 0.8 µm, 0.9 µm, 1 µm, 1.1 µm, 1.2 µm, 1.3 µm, 1.4 µm, 1.5 µm, 1.6 µm, 1.7 µm, 1.8 µm, 1.9 µm, 2 µm, 2.5 µm, or 3 µm, or a diameter in a range between any of the aforementioned values.

In some embodiments, the DNA concatemer is between about 1 and about 10, between about 10 and about 50, between about 45 and about 75, or between about 75 and 100 kilobases in length. In some embodiments, the DNA concatemer is about 1, 5, 10, 15, 20, 25, 30, 35, 40, 45, 50, 60, 65, 70, 75, 80, 85, 90, 95, or 100 kilobases in length, or in a range between any of the aforementioned values. Exemplary DNA concatemers such as RCA products are disclosed Blanco et al., JBC 264(15): 8935-8940 (1989) which is incorporated herein by reference in its entirety. in In some embodiments, the DNA concatemer comprises a target sequence that is repeated between about 100 and about 1,000, between about 1,000 and about 5,000, or between about 5,000 and about 10,000 times.

The RCA reaction may be useful in multiplex situations or where sequential labelling is taking place (e.g., where multiple products are sequentially generated for each analyte), and/or in the analysis of the results, e.g., where the targets are detected by imaging. In some embodiments, the method further comprises a series of hybridization, washing, and detection steps, to detect an analyte, optionally wherein the analyte is an RCA product, wherein the probes are nucleic acid analog probes (e.g., morpholino-based detection or intermediate probes). In some embodiments, a primary probe is a nucleic acid probe and a plurality of primary probes each comprising a detection moiety are used to detect the localization of target sequences having sugar-phosphate backbones within the sample. In some embodiments, a detection step using primary probes may comprise disrupting (e.g., by washing) the binding of the primary probes to remove them from the sample. In some embodiments, a plurality of intermediate probes (e.g., secondary probes), which are nucleic acid analog probes (e.g., morpholino-based probes), hybridize to one or more primary probes within the sample. In some embodiments, a plurality of intermediate probes each comprising a detection moiety are used to detect the localization of primary probes (which may have sugar-phosphate backbones or may be nucleic acid analog probes) within the sample. In some embodiments, a detection step using morpholino-based intermediate probes may comprise disrupting (e.g., by washing) the binding of the plurality of intermediate probes to remove them from the sample. In some embodiments, a plurality of tertiary probes, which are nucleic acid analog probes (e.g., morpholino-based probes), hybridize to one or more of the secondary probes within the sample. In some embodiments, a plurality of tertiary probes each comprising a detection moiety are used to detect the localization of secondary probes (which may have sugar-phosphate backbones or may be nucleic acid analog probes) within the sample. In some embodiments, the method may further comprise disrupting (e.g., by washing) the binding of the tertiary probes to remove them from the sample. In some embodiments, the detection moiety in each of the primary probes and intermediate probes (e.g., secondary, tertiary, and/or higher order intermediate probes) comprise a fluorophore. In some embodiments, each detection moiety in each of the primary and intermediate probes comprises a different fluorophore. In some embodiments, a wash step to disrupt and/or remove the binding of the intermediate probes such as secondary, tertiary, or higher order intermediate probes and/or detection probes from the sample is performed after a detection step.

In some embodiments, the fluorescently labeled detection probe hybridizes to an intermediate probe (e.g., a secondary probe or bridging probe) comprising a sequence that directly hybridizes to the target sequence of the RCA product. In some embodiments, at least about 5%, at least about 10%, at least about 25%, at least about 50%, at least about 75%, or at least about 90% of the copies of the target sequence in the RCA product are hybridized directly or indirectly to one or more molecules of the fluorescently labeled detection probe. In some embodiments, about 5%, 10%, 20%, 30%, 40%, 50%, 60%, 70%, 80%, or 90% of the copies of the target sequence in the RCA product are hybridized directly or indirectly to one or more molecules of the fluorescently labeled detection probe.

In some embodiments, the method may further comprise removing one or more molecules of the probe (e.g., detection probe and/or intermediate probe) that have not hybridized to the target sequence. This removing step may occur, for example, prior to the detection step. In some examples, the probe is a first probe and the method further comprises removing molecules of the first probe hybridized to the target sequence, prior to contacting the biological sample with a second probe comprising a sequence of nucleobases on a synthetic backbone. In some embodiments, the target sequence is a first target sequence, and the second probe directly or indirectly hybridizes to a second target sequence in the DNA concatemer. The first and second target sequences may be the same. Alternatively, the first and second target sequences may be different.

### B. Probe Detection and Analysis

In some aspects, the methods disclosed herein involve the use of one or more probes or probe sets that directly or indirectly hybridize to a target nucleic acid, such as an RNA molecule. In some aspects, a sample is contacted with a plurality of barcoded probe sets, each for analyzing an analyte (e.g., biomarker) panel. The sample can be contacted, in separate rounds, with different detectably labeled oligonucleotides for analyzing one or more sets in the barcoded probe sets (and thereby analyzing the one or more analyte panels corresponding to the one or more sets). In some embodiments, the detectably labeled probes (e.g., detection probe and/or intermediate probe) can be changed between rounds while the primary or intermediate probes remain bound to analytes in the sample. The primary or intermediate probes may be barcoded probes.

In some embodiments, a detectably labeled probe (e.g., detection probe) directly hybridizes to its target, e.g., a transcript or DNA locus. In some embodiments, a detectably labeled oligonucleotide specifically interacts with (recognizes) its target through binding or hybridization to one or more intermediate, e.g., an oligonucleotide, that is bound, hybridized, or otherwise specifically linked to the target. In some embodiments, an intermediate probe comprises a barcode.

In some embodiments, an intermediate probe (e.g., intermediate oligonucleotide) is hybridized against its target with an overhang such that a second oligonucleotide with complementary sequence (detection probe) can bind to it. In some embodiments, an intermediate probe targets a nucleic acid and is optionally labeled with a detectable moiety, and comprises an overhang sequence after hybridization with the target. In some embodiments, an intermediate probe comprises a sequence that hybridizes to a target, an overhang sequence, and optionally a detectable moiety. In some embodiments, an intermediate probe (e.g., barcoded probes that directly bind analytes in a sample) comprises a sequence that hybridizes to a target and an overhang sequence In some embodiments, an intermediate probe does not have a detectable moiety. In some embodiments, a second oligonucleotide is a detectably labeled oligonucleotide (e.g., a detection probe). In some embodiments, a second detectably labeled oligonucleotide (e.g., a detection probe) is labeled with a dye. In some embodiments, a detectably labeled oligonucleotide (e.g., a detection probe) is labeled with an HCR polymer. In some embodiments, intermediate probes bound to targets are preserved through multiple contacting, removing and/or imaging steps; sequential barcodes are provided through combinations of detectable labels that are linked to intermediate oligonucleotides through bridge probes in the contacting and imaging steps. For example, when intermediate probes are bound to targets, barcodes are provided by detectably labeled oligonucleotides that hybridize with intermediate probes through their overhang sequences. After an imaging step, intermediate probes are optionally removed as described herein. In some embodiments, one intermediate probe is employed for a target. In some embodiments, two or more intermediate probes are employed for a target. In some embodiments, three or more intermediate probes are employed for a target. In some embodiments, four or more intermediate probes are employed for a target. In some embodiments, five or more intermediate probes are employed for a target.

In some embodiments, each intermediate probe (e.g., intermediate oligonucleotide) hybridizes with a different sequence of a target. In some embodiments, each intermediate probe (e.g., intermediate oligonucleotide) hybridizes with the same sequence of a target. In some embodiments, two or more intermediate oligonucleotides of a target comprises the same overhang sequence In some embodiments, two or more intermediate oligonucleotides of a target comprises different overhang sequences. In some embodiments, a plurality of intermediate probes hybridize with the same sequence of a target, wherein two or more of the intermediate probes comprise a different overhang sequence. In some embodiments, each detectable probe of a plurality of detectable probes for the same target comprises a different sequence complementary to each overhang sequence shared by a plurality of intermediate probes comprising the same sequence for hybridizing to the same target. In some embodiments, an intermediate probe comprises a sequence complementary to a target, and a sequence complementary to a detectably labeled oligonucleotide (e.g., detection probe).

In some embodiments, each detectably labeled oligonucleotide (e.g., detection probe or intermediate probe) in a set has a different target or is associated with a different target, e.g., a transcript or DNA locus. In some embodiments, two or more detectably labeled oligonucleotides (e.g., detection probe or intermediate probe) in a set have the same target or is associated with the same target. In some embodiments, two or more detectably labeled oligonucleotides (e.g., detection probe or intermediate probe) target the same transcript. In some embodiments, two or more detectably labeled oligonucleotides (e.g., detection probe or intermediate probe) target the same DNA locus. In some embodiments, about 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90 or 100 detectably labeled oligonucleotides the same target. In some embodiments, two or more detectably labeled oligonucleotides (e.g., detection probes or intermediate probes) target the same target. In some embodiments, five or more detectably labeled oligonucleotides target the same target.

In some embodiments, among other things, using multiple detectably labeled oligonucleotides (e.g., detection probe or intermediate probe) for the same target increases signal intensity. In some embodiments, each detectably labeled oligonucleotide (e.g., detection probe or intermediate probe) in a set targeting the same target interacts with a different portion of a target.

In some embodiments, all detectably labeled oligonucleotides (e.g., detection probes) for a target in a set have the same detectable moieties. In some embodiments, all detectably labeled oligonucleotides are labeled in the same way. In some embodiments, all the detectably labeled oligonucleotides for a target have the same fluorophore. In some embodiments, two or more detection probes for a target have different fluorophores.

In some embodiments, detectably labeled oligonucleotides for a target are positioned within a targeted region of a target. A targeted region can have various lengths. In some embodiments, a targeted region is about 20 nucleobases in length. In some embodiments, a targeted region is about 30 nucleobases in length. In some embodiments, a targeted region is about 40 nucleobases in length. In some embodiments, a targeted region is about 50 nucleobases in length. In some embodiments, a targeted region is about 60 nucleobases in length. In some embodiments, a targeted region is about 80 bp in length. In some embodiments, a targeted region is about 100 nucleobases in length. In some embodiments, a targeted region is about 150 nucleobases in length. In some embodiments, a targeted region is about 200 nucleobases in length. In some embodiments, a targeted region is about 250 nucleobases in length. In some embodiments, a targeted region is about 300 nucleobases in length. In some embodiments, a targeted region is about 350 nucleobases in length. In some embodiments, a targeted region is about 400 nucleobases in length. In some embodiments, a targeted region is about 450 nucleobases in length. In some embodiments, a targeted region is about 500 nucleobases in length. In some embodiments, a targeted region is about 600 nucleobases in length. In some embodiments, a targeted region is about 700 nucleobases in length. In some embodiments, a targeted region is about 800 nucleobases in length. In some embodiments, a targeted region is about 900 nucleobases in length. In some embodiments, a targeted region is about 1,000 nucleobases in length. In some embodiments, detectably labeled oligonucleotides for a target are positioned in proximity to each other on the target.

In some embodiments, targets of one set of detectably labeled oligonucleotides are also targets of another set. In some embodiments, targets of one set of detectably labeled oligonucleotides overlap with those of another set. In some embodiments, the overlap is more than 10%, 20%, 30%, 40%, 50%, 60%, 70%, 80%, 90%, 95%, or 100%. In some embodiments, targets of one set of detectably labeled oligonucleotides are the same as targets of another set. In some embodiments, each set of detectably labeled oligonucleotides targets the same targets.

As used herein, a detectably labeled oligonucleotide is labeled with a detectable moiety. In some embodiments, a detectably labeled oligonucleotide comprises one detectable moiety. In some embodiments, a detectably labeled oligonucleotide comprises two or more detectable moieties. In some embodiments, a detectably labeled oligonucleotide has one detectable moiety. In some embodiments, a detectably labeled oligonucleotide has two or more detectable moiety.

Probes and methods for binding and identifying a target nucleic acid have been described in, e.g., US2003/0013091, US2007/0166708, US2010/0015607, US2010/0261026, US2010/0262374, US2010/0112710, US2010/0047924, and US2014/0371088, each of which is incorporated herein by reference in its entirety.

In some embodiments, a detectable moiety is or comprises a nanomaterial. In some embodiments, a detectable moiety is or compresses a nanoparticle. In some embodiments, a detectable moiety is or comprises a quantum dot. In some embodiments, a detectable moiety is a quantum dot. In some embodiments, a detectable moiety comprises a quantum dot. In some embodiments, a detectable moiety is or comprises a gold nanoparticle. In some embodiments, a detectable moiety is a gold nanoparticle. In some embodiments, a detectable moiety comprises a gold nanoparticle.

One of skill in the art understands that, in some embodiments, selection of label for a particular probe (e.g., detection probe) in a particular cycle may be determined based on a variety of factors, including, for example, size, types of signals generated, manners attached to or incorporated into a probe, properties of the cellular constituents including their locations within the cell, properties of the cells, types of interactions being analyzed, and etc.

For example, in some embodiments, probes (e.g., detection probe) are labeled with either Cy3 or Cy5 that has been synthesized to carry an N-hydroxysuccinimidyl ester (NHS-ester) reactive group. Since NHS-esters react readily with aliphatic amine groups, nucleotides can be modified with aminoalkyl groups. This can be done through incorporating aminoalkyl-modified nucleotides during synthesis reactions. In some embodiments, a label is used in every 60 bases to avoid quenching effects.

In some embodiments, sequence analysis of the target and/or barcoded probes can be performed by sequential fluorescence hybridization (e.g., sequencing by hybridization). Sequential fluorescence hybridization can involve sequential hybridization of detection probes comprising an oligonucleotide and a detectable label.

In some embodiments, sequencing can be performed by sequencing-by-synthesis (SBS). In some embodiments, a sequencing primer is complementary to sequences at or near the one or more barcode(s). In such embodiments, sequencing-by-synthesis can comprise reverse transcription and/or amplification in order to generate a template sequence from which a primer sequence can bind. Exemplary SBS methods comprise those described for example, but not limited to, US 2007/0166705, US 2006/0188901, US 7,057,026, US 2006/0240439, US 2006/0281109, US 10,953,379, US 2005/0100900, WO 06/064199, US 10,710,046, US 2012/0270305, US 2013/0260372, and US 2013/0079232.

In some embodiments, sequencing can be performed using single molecule sequencing by ligation. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. Aspects and features involved in sequencing by ligation are described, for example, in Shendure et al. Science (2005), 309: 1728-1732, and in US 5,599,675; US 5,750,341; US 6,969,488; US 6,172,218; US and 6,306,597.

In some embodiments, the barcodes are targeted by detectably labeled detection oligonucleotides (e.g., detection probes), such as fluorescently labeled oligonucleotides (e.g., fluorescently labeled detection probes). In some embodiments, one or more decoding schemes are used to decode the signals, such as fluorescence, for sequence determination. In any of the embodiments herein, barcodes (e.g., primary and/or secondary barcode sequences) can be analyzed (e.g., detected or sequenced) using any suitable methods or techniques, comprising those described herein, such as RNA sequential probing of targets (RNA SPOTs), sequential fluorescent *in situ* hybridization (seqFISH), single-molecule fluorescent *in situ* hybridization (smFISH), multiplexed error-robust fluorescence *in situ* hybridization (MERFISH), hybridization-based *in situ* sequencing (HybISS), *in situ* sequencing, targeted *in situ* sequencing, fluorescent *in situ* sequencing (FISSEQ), or spatially-resolved transcript amplicon readout mapping (STARmap). In some embodiments, the methods provided herein comprise analyzing the barcodes by sequential hybridization and detection with a plurality of labelled probes (e.g., detection oligonucleotides). Exemplary decoding schemes are described in Eng et al., "Transcriptome-scale Super-Resolved Imaging in Tissues by RNA SeqFISH+," Nature 568(7751):235-239 (2019); Chen et al., "Spatially resolved, highly multiplexed RNA profiling in single cells," Science; 348(6233):aaa6090 (2015); Gyllborg et al., Nucleic Acids Res (2020) 48(19):e112; US 10,457,980 B2; US 2016/0369329 A1; WO 2018/026873 A1; and US 2017/0220733 A1, all of which are incorporated by reference in their entirety. In some embodiments, these assays enable signal amplification, combinatorial decoding, and error correction schemes at the same time.

In some embodiments, nucleic acid hybridization can be used for sequencing. These methods utilize labeled nucleic acid decoder probes that are complementary to at least a portion of a barcode sequence. Multiplex decoding can be performed with pools of many different probes with distinguishable labels. Non-limiting examples of nucleic acid hybridization sequencing are described for example in US 8,460,865, and in Gunderson et al., Genome Research 14:870-877 (2004), the entire contents of each of which are incorporated herein by reference. In any of the preceding embodiments, the methods provided herein can include analyzing the barcodes by sequential hybridization and detection with a plurality of labelled probes (e.g., detection probes).

In some embodiments, real-time monitoring of DNA polymerase activity can be used during sequencing. For example, nucleotide incorporations can be detected through fluorescence resonance energy transfer (FRET), as described for example in Levene et al., Science (2003), 299, 682-686, Lundquist et al., Opt. Lett. (2008), 33, 1026-1028, and Korlach et al., Proc. Natl. Acad. Sci. USA (2008), 105, 1176-1181.

In some aspects, the analysis and/or sequence determination can be carried out at room temperature for best preservation of tissue morphology with low background noise and error reduction. In some embodiments, the analysis and/or sequence determination comprises eliminating error accumulation as sequencing proceeds.

In some embodiments, the analysis and/or sequence determination involves washing to remove unbound polynucleotides, thereafter revealing a fluorescent product for imaging.

In some aspects, the detection (comprising imaging) is carried out using any of a number of different types of microscopy, e.g., confocal microscopy, two-photon microscopy, light-field microscopy, intact tissue expansion microscopy, and/or CLARITY^{™}-optimized light sheet microscopy (COLM).

In some embodiments, fluorescence microscopy is used for detection and imaging of the detection probe. In some aspects, a fluorescence microscope is an optical microscope that uses fluorescence and phosphorescence instead of, or in addition to, reflection and absorption to study properties of organic or inorganic substances. In fluorescence microscopy, a sample is illuminated with light of a wavelength which excites fluorescence in the sample. The fluoresced light, which is usually at a longer wavelength than the illumination, is then imaged through a microscope objective. Two filters may be used in this technique; an illumination (or excitation) filter which ensures the illumination is near monochromatic and at the correct wavelength, and a second emission (or barrier) filter which ensures none of the excitation light source reaches the detector. Alternatively, these functions may both be accomplished by a single dichroic filter. The "fluorescence microscope" comprises any microscope that uses fluorescence to generate an image, whether it is a more simple set up like an epifluorescence microscope, or a more complicated design such as a confocal microscope, which uses optical sectioning to get better resolution of the fluorescent image.

In some embodiments, confocal microscopy is used for detection and imaging of the detection probe. Confocal microscopy uses point illumination and a pinhole in an optically conjugate plane in front of the detector to eliminate out-of-focus signal. As only light produced by fluorescence very close to the focal plane can be detected, the image's optical resolution, particularly in the sample depth direction, is much better than that of wide-field microscopes. However, as much of the light from sample fluorescence is blocked at the pinhole, this increased resolution is at the cost of decreased signal intensity - so long exposures are often required As only one point in the sample is illuminated at a time, 2D or 3D imaging requires scanning over a regular raster (i.e., a rectangular pattern of parallel scanning lines) in the specimen. The achievable thickness of the focal plane is defined mostly by the wavelength of the used light divided by the numerical aperture of the objective lens, but also by the optical properties of the specimen. The thin optical sectioning possible makes these types of microscopes particularly good at 3D imaging and surface profiling of samples. CLARITY^{™}-optimized light sheet microscopy (COLM) provides an alternative microscopy for fast 3D imaging of large clarified samples. CaLM interrogates large immunostained tissues, permits increased speed of acquisition and results in a higher quality of generated data.

Other types of microscopy that can be employed comprise bright field microscopy, oblique illumination microscopy, dark field microscopy, phase contrast, differential interference contrast (DIC) microscopy, interference reflection microscopy (also known as reflected interference contrast, or RIC), single plane illumination microscopy (SPIM), super-resolution microscopy, laser microscopy, electron microscopy (EM), Transmission electron microscopy (TEM), Scanning electron microscopy (SEM), reflection electron microscopy (REM), Scanning transmission electron microscopy (STEM) and low-voltage electron microscopy (LVEM), scanning probe microscopy (SPM), atomic force microscopy (ATM), ballistic electron emission microscopy (BEEM), chemical force microscopy (CFM), conductive atomic force microscopy (C- AFM), electrochemical scanning tunneling microscope (ECSTM), electrostatic force microscopy (EFM), fluidic force microscope (FluidFM), force modulation microscopy (FMM), feature-oriented scanning probe microscopy (FOSPM), kelvin probe force microscopy (KPFM), magnetic force microscopy (MFM), magnetic resonance force microscopy (MRFM), near-field scanning optical microscopy (NSOM) (or SNOM, scanning near-field optical microscopy, SNOM, Piezoresponse Force Microscopy (PFM), PSTM, photon scanning tunneling microscopy (PSTM), PTMS, photothermal microspectroscopy/ microscopy (PTMS), SCM, scanning capacitance microscopy (SCM), SECM, scanning electrochemical microscopy (SECM), SGM, scanning gate microscopy (SGM), SHPM, scanning Hall probe microscopy (SHPM), SICM, scanning ion-conductance microscopy (SICM), SPSM spin polarized scanning tunneling microscopy (SPSM), SSRM, scanning spreading resistance microscopy (SSRM), SThM, scanning thermal microscopy (SThM), STM, scanning tunneling microscopy (STM), STP, scanning tunneling potentiometry (STP), SVM, scanning voltage microscopy (SVM), and synchrotron x-ray scanning tunneling microscopy (SXSTM), and intact tissue expansion microscopy (exM).

The probes described herein may be used for analyzing a sequence of the DNA concatemer. In some embodiments, the sequence to be analyzed is a nucleic acid barcode sequence. For example, the nucleic acid barcode sequence may correspond to an analyte or a portion (e.g., a nucleic acid sequence) thereof or a labelling agent for the analyte or portion thereof in the biological sample.

In some embodiments, the detection further comprises imaging the complex (e.g., DNA concatemer) comprising the probe, e.g., by optical imaging. For example, the one or more probes may be barcoded probes comprising one or more nucleic acid barcode sequences, which can be directly or indirectly bound by detectably-labeled detection probes. A detectable signal or a series of signals such as fluorescence comprising a spatial pattern and/or a temporal pattern may be analyzed to reveal the presence/absence, distribution, location, amount, level, expression, or activity of the one or more analytes in the sample. In some embodiments, the one or more analytes (e.g., target sequences within a DNA concatemer) are analyzed (e.g., by imaging) *in situ* in a tissue sample without migrating out of a cell of the tissue sample. In some embodiments, the one or more analytes are analyzed (e.g., by imaging) *in situ* in a tissue sample without migrating out of the tissue sample, e.g., onto a substrate. In some embodiments, the probe (e.g., detection probe) comprises the analyte-binding moiety (e.g., antibody) and the nucleic acid barcode sequence is not cleaved during the *in situ* analysis.

In some cases, the analysis is performed on one or more images captured, and may comprise processing the image(s) and/or quantifying signals observed. For example, the analysis may comprise processing information of one or more cell types, one or more types of biomarkers, a number or level of a biomarker, and/or a number or level of cells detected in a particular region of the sample. In some embodiments, the analysis comprises detecting a sequence e.g., a barcode present in the sample. In some embodiments, the analysis includes quantification of puncta (e.g., if amplification products are detected). In some cases, the analysis includes determining whether particular cells and/or signals are present that correlate with one or more biomarkers from a particular panel. In some embodiments, the obtained information may be compared to a positive and negative control, or to a threshold of a feature to determine if the sample exhibits a certain feature or phenotype. In some cases, the information may comprise signals from a cell, a region, and/or comprise readouts from multiple detectable labels. In some case, the analysis further includes displaying the information from the analysis or detection step. In some embodiments, software may be used to automate the processing, analysis, and/or display of data.

### C. Exemplary Advantages of Nucleic Acid Analog Probes

The method of using fluorescently labeled morpholinos as detection probes during the chemical/optical decoding cycles during *in situ* analysis, as described herein, allows for increased sensitivity *of in situ* sequencing. In some embodiments, morpholinos maximize the ability of detection probes to hybridize to all available docking sites in the amplification product (e.g., DNA concatemer), as shown in FIG. 2A compared to **FIG. 2B****.**

Incorporating morpholinos (e.g., phosphorodiamidate linkages) into detection probes during in situ sequencing significantly increased the brightness of each amplicon (e.g., DNA concatemer) resulting in increased sensitivity. This chemical approach affords the ability for less amplification time and a smaller amplicon size, while retaining an equivalent or even higher degree of signal brightness. In some embodiments, the advantages afforded by a method described herein are due to the ability of more detection probes hybridizing to previously inaccessible sites within the DNA concatemer **(****FIG. 2B****),** compared to detection probes not comprising morpholinos **(****FIG. 2A****).** Furthermore, by brighter and smaller amplicons may decrease optical crowding, allowing for more genes to be analyzed in their positional context during a single experiment.

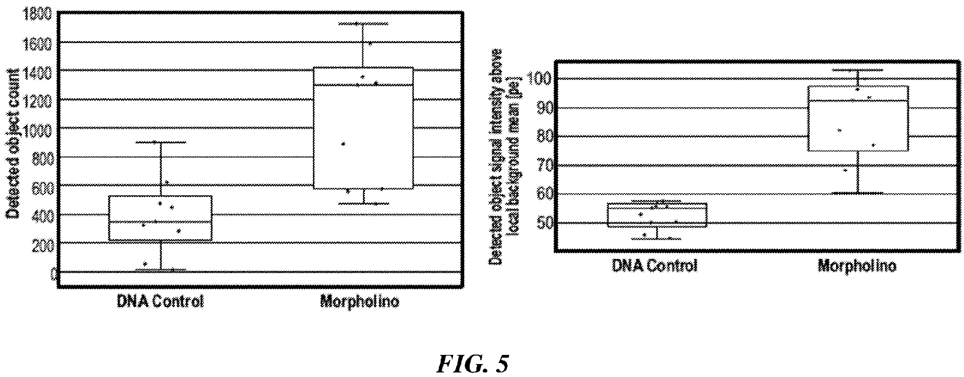

In some aspects, an advantage of the presently disclosed method is that there is less amplification time and smaller RCA products (e.g., DNA concatemers in the submicron range), while retaining an equivalent or even higher degree of signal brightness. Therefore, this method creates brighter and smaller amplicons, to decrease optical crowding and allow more genes to be analyzed in their positional context during a single experiment. The method described may also permit single probe/RNA implementation; rather than using the multiple probes/RNA (e.g., 4 probes/RNA) design. Using uncharged oligonucleotide analogs (e.g., morpholinos) would afford a variety of advantages over the current system, including decreasing DNA concatemer size while increasing brightness of the amplicon may be achieved **(****FIG. 5****).**

In some embodiments, a morpholino-based detection probe allows for only one circularizable probe (e.g., padlock probe) per RNA, due to increased detection sensitivity. Typical *in situ* sequencing analyses requires anywhere from 4-12 padlock probes/RNA in order to generate a bright enough signal, employing traditional detection oligonucleotides that do not take into account the electrostatic effects of the DNA concatemer. A single padlock probe/RNA affords several additional advantages, including: 1) less padlock probes required; 2) increased resolution of splice variants in their unique locations; 3) potentially less amplification time; 4) smaller DNA concatemer size, reducing molecular/optical crowding; and, 5) linear dilution capabilities to permit control over molecular crowding.

In some embodiments, using one padlock probe/RNA affords the ability to have increased resolution of instances of alternative splicing, as illustrated in **FIG. 4****.** For example, one padlock probe may only result in amplification of a specified region of DNA if two exons are immediately adjacent to one another (e.g., exons straddling a splice site junction for isoform 'X', as shown in **FIG. 4****).** Alternatively, if said exons are not immediately adjacent to one another (e.g., the purple exon has been spliced out of **FIG. 4****),** the padlock probe will not hybridize, and no amplification will occur. In some embodiments, a morpholino-based detection oligo allows increased resolution of splice variants, compared to traditional detection oligonucleotides, since increased detection sensitivity means that RCA products can be smaller, and/or that fewer padlock probes (e.g., only one padlock probe per nucleic acid target) are needed to hybridize to a target sequence.

### IV. Compositions and Kits

In some aspects, provided herein is a composition, comprising: a DNA concatemer comprising multiple copies of a target sequence, molecules of an intermediate probe that hybridizes to the target sequence, and molecules of a fluorescently labeled probes (e.g., intermediate and/or detection probes) comprising a sequence of nucleobases on a synthetic backbone that hybridizes to the intermediate probe, wherein the DNA concatemer, molecules of the intermediate probe and molecules of the fluorescently labeled detection probe (e.g., detectable probes) form a hybridization complex. In the composition of any of the preceding embodiments, the DNA concatemer may be a rolling circle amplification (RCA) product. In the composition of any of the preceding embodiments, the RCA product may be generated *in situ* in a biological sample, using a circular or circularizable (e.g., padlock) probe or probe set that may directly or indirectly bind to an analyte in the biological sample as template.

Also provided herein are kits, for example comprising a probe (e.g., intermediate and/or detection probe) with a synthetic backbone (optionally comprising a fluorescent moiety) and reagents for performing the methods provided herein, for example reagents required for one or more steps comprising hybridization, ligation, amplification, detection, sequencing, and/or sample preparation as described herein.

In some aspects, provided herein is a kit comprising: a fluorescently labeled probe (e.g. detection probe) comprising a sequence of nucleobases on a synthetic backbone that hybridizes to an intermediate probe, and the intermediate probe which comprises a sequence of nucleobases on a synthetic backbone that hybridizes to a target sequence. In some embodiments, the fluorescently labeled detection probe and/or the intermediate probe may be morpholino. In some embodiments, the fluorescently labeled detection probe and/or the intermediate probe may comprise phosphorodiamidate inter-subunit linkages. In some embodiments, the kit may further comprise a circular or circularizable (e.g., padlock) probe or probe set (primary, secondary, or other intermediate probes) comprising the target sequence or a complement thereof, wherein the probe or probe set may be capable of directly or indirectly binding to an analyte in a biological sample and may generate a rolling circle amplification product using the probe or probe set as template. In some embodiments, the kit further comprises a target nucleic acid comprised in a biological sample (e.g., a biological sample comprising a DNA concatemer).

The various components of the kit may be present in separate containers or certain compatible components may be pre-combined into a single container. In some embodiments, the kits further contain instructions for using the components of the kit to practice the provided methods.

In some embodiments, the kits can contain reagents and/or consumables required for performing one or more steps of the provided methods. In some embodiments, the kits contain reagents for fixing, embedding, and/or permeabilizing the biological sample. In some embodiments, the kits contain reagents, such as enzymes and buffers for ligation and/or amplification, such as ligases and/or polymerases. In some aspects, the kit can also comprise any of the reagents described herein, e.g., hybridization buffer, wash buffer and ligation buffer. In some embodiments, the kits contain reagents for detection and/or sequencing, such as barcode detection probes or detectable labels. In some embodiments, the kits optionally contain other components, for example nucleic acid primers, enzymes and reagents, buffers, nucleotides, modified nucleotides, reagents for additional assays.

### V. Terminology

Specific terminology is used throughout this disclosure to explain various aspects of the apparatus, systems, methods, and compositions that are described.

Having described some illustrative embodiments of the present disclosure, it should be apparent to those skilled in the art that the foregoing is merely illustrative and not limiting, having been presented by way of example only. Numerous modifications and other illustrative embodiments are within the scope of one of ordinary skill in the art and are contemplated as falling within the scope of the present disclosure. In particular, although many of the examples presented herein involve specific combinations of method acts or system elements, it should be understood that those acts and those elements may be combined in other ways to accomplish the same objectives.

As used herein, the singular forms "a," "an," and "the" include plural referents unless the context clearly dictates otherwise. For example, "a" or "an" means "at least one" or "one or more."

The term "about" as used herein refers to the usual error range for the respective value readily known to the skilled person in this technical field. Reference to "about" a value or parameter herein includes (and describes) embodiments that are directed to that value or parameter per se.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

### (i) Barcode

A "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes.

Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI").

Barcodes can spatially-resolve molecular components found in biological samples, for example, at single-cell resolution (e.g., a barcode can be or can include a "spatial barcode"). In some embodiments, a barcode includes both a UMI and a spatial barcode. In some embodiments, a barcode includes two or more sub-barcodes that together function as a single barcode. For example, a polynucleotide barcode can include two or more polynucleotide sequences (e.g., sub-barcodes) that are separated by one or more non-barcode sequences.

### (ii) Nucleic Acid and Nucleotide

The terms "nucleic acid" and "nucleotide" are intended to be consistent with their use in the art and to include naturally-occurring species or functional analogs thereof. Particularly useful functional analogs of nucleic acids are capable of hybridizing to a nucleic acid in a sequence-specific fashion (e.g., capable of hybridizing to two nucleic acids such that ligation can occur between the two hybridized nucleic acids) or are capable of being used as a template for replication of a particular nucleotide sequence. Naturally-occurring nucleic acids generally have a backbone containing phosphodiester bonds. An analog structure can have an alternate backbone linkage including any of a variety of those known in the art. Naturally-occurring nucleic acids generally have a deoxyribose sugar (e.g., found in deoxyribonucleic acid (DNA)) or a ribose sugar (e.g. found in ribonucleic acid (RNA)).

A nucleic acid can contain nucleotides having any of a variety of analogs of these sugar moieties that are known in the art. A nucleic acid can include native or non-native nucleotides. In this regard, a native deoxyribonucleic acid can have one or more bases selected from the group consisting of adenine (A), thymine (T), cytosine (C), or guanine (G), and a ribonucleic acid can have one or more bases selected from the group consisting of uracil (U), adenine (A), cytosine (C), or guanine (G). Useful non-native bases that can be included in a nucleic acid or nucleotide are known in the art.

### (iii) Probe and Target

A "probe" or a "target," when used in reference to a nucleic acid or sequence of a nucleic acids, is intended as a semantic identifier for the nucleic acid or sequence in the context of a method or composition, and does not limit the structure or function of the nucleic acid or sequence beyond what is expressly indicated.

### (iv) Oligonucleotide and Polynucleotide

The terms "oligonucleotide" and "polynucleotide" are used interchangeably to refer to a single-stranded multimer of nucleotides from about 2 to about 500 nucleotides in length. Oligonucleotides can be synthetic, made enzymatically (e.g., via polymerization), or using a "split-pool" method. Oligonucleotides can include ribonucleotide monomers (i.e., can be oligoribonucleotides) and/or deoxyribonucleotide monomers (i.e., oligodeoxyribonucleotides). In some examples, oligonucleotides can include a combination of both deoxyribonucleotide monomers and ribonucleotide monomers in the oligonucleotide (e.g., random or ordered combination of deoxyribonucleotide monomers and ribonucleotide monomers). An oligonucleotide can be 4 to 10, 10 to 20, 21 to 30, 31 to 40, 41 to 50, 51 to 60, 61 to 70, 71 to 80, 80 to 100, 100 to 150, 150 to 200, 200 to 250, 250 to 300, 300 to 350, 350 to 400, or 400-500 nucleotides in length, for example. Oligonucleotides can include one or more functional moieties that are attached (e.g., covalently or non-covalently) to the multimer structure. For example, an oligonucleotide can include one or more detectable labels (e.g., a radioisotope or fluorophore).

### (v) Hybridizing, Hybridize, Annealing, and Anneal

The terms "hybridizing," "hybridize," "annealing," and "anneal" are used interchangeably in this disclosure, and refer to the pairing of substantially complementary or complementary nucleic acid sequences within two different molecules. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

### (vi) Primer

A "primer" is a single-stranded nucleic acid sequence having a 3' end that can be used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases. A primer, may in some cases, refer to a primer binding sequence.

### (vii) Primer Extension

A "primer extension" refers to any method where two nucleic acid sequences (e.g., a constant region from each of two distinct capture probes) become linked (e.g., hybridized) by an overlap of their respective terminal complementary nucleic acid sequences (i.e., for example, 3' termini). Such linking can be followed by nucleic acid extension (e.g., an enzymatic extension) of one, or both termini using the other nucleic acid sequence as a template for extension. Enzymatic extension can be performed by an enzyme including, but not limited to, a polymerase and/or a reverse transcriptase.

### (viii) Nucleic Acid Extension

A "nucleic acid extension" generally involves incorporation of one or more nucleic acids (e.g., A, G, C, T, U, nucleotide analogs, or derivatives thereof) into a molecule (such as, but not limited to, a nucleic acid sequence) in a template-dependent manner, such that consecutive nucleic acids are incorporated by an enzyme (such as a polymerase or reverse transcriptase), thereby generating a newly synthesized nucleic acid molecule. For example, a primer that hybridizes to a complementary nucleic acid sequence can be used to synthesize a new nucleic acid molecule by using the complementary nucleic acid sequence as a template for nucleic acid synthesis. Similarly, a 3' polyadenylated tail of an mRNA transcript that hybridizes to a poly (dT) sequence (e.g., capture domain) can be used as a template for single-strand synthesis of a corresponding cDNA molecule.

### (ix) PCR Amplification

A "PCR amplification" refers to the use of a polymerase chain reaction (PCR) to generate copies of genetic material, including DNA and RNA sequences. Suitable reagents and conditions for implementing PCR are described, for example, in U.S. Patents 4,683,202, 4,683,195, 4,800,159, 4,965,188, and 5,512,462, the entire contents of each of which are incorporated herein by reference. In a typical PCR amplification, the reaction mixture includes the genetic material to be amplified, an enzyme, one or more primers that are employed in a primer extension reaction, and reagents for the reaction. The oligonucleotide primers are of sufficient length to provide for hybridization to complementary genetic material under annealing conditions. The length of the primers generally depends on the length of the amplification domains, but will typically be at least 4 bases, at least 5 bases, at least 6 bases, at least 8 bases, at least 9 bases, at least 10 base pairs (bp), at least 11 bp, at least 12 bp, at least 13 bp, at least 14 bp, at least 15 bp, at least 16 bp, at least 17 bp, at least 18 bp, at least 19 bp, at least 20 bp, at least 25 bp, at least 30 bp, at least 35 bp, and can be as long as 40 bp or longer, where the length of the primers will generally range from 18 to 50 bp. The genetic material can be contacted with a single primer or a set of two primers (forward and reverse primers), depending upon whether primer extension, linear or exponential amplification of the genetic material is desired.

In some embodiments, the PCR amplification process uses a DNA polymerase enzyme. The DNA polymerase activity can be provided by one or more distinct DNA polymerase enzymes. In certain embodiments, the DNA polymerase enzyme is from a bacterium, e.g., the DNA polymerase enzyme is a bacterial DNA polymerase enzyme. For instance, the DNA polymerase can be from a bacterium of the genus *Escherichia, Bacillus, Thermophilus,* or *Pyrococcus.*

Suitable examples of DNA polymerases that can be used include, but are not limited to: *E.coli* DNA polymerase I, Bsu DNA polymerase, Bst DNA polymerase, Taq DNA polymerase, VENT^{™} DNA polymerase, DEEPVENT^{™} DNA polymerase, LongAmp^{®} Taq DNA polymerase, LongAmp^{®} Hot Start Taq DNA polymerase, Crimson LongAmp^{®} Taq DNA polymerase, Crimson Taq DNA polymerase, OneTaq^{®} DNA polymerase, OneTaq^{®} Quick-Load^{®} DNA polymerase, Hemo KlenTaq^{®} DNA polymerase, REDTaq^{®} DNA polymerase, Phusion^{®} DNA polymerase, Phusion^{®} High-Fidelity DNA polymerase, Platinum Pfx DNA polymerase, AccuPrime Pfx DNA polymerase, Phi29 DNA polymerase, Klenow fragment, Pwo DNA polymerase, Pfu DNA polymerase, T4 DNA polymerase and T7 DNA polymerase enzymes.

The term "DNA polymerase" includes not only naturally-occurring enzymes but also all modified derivatives thereof, including also derivatives of naturally-occurring DNA polymerase enzymes. For instance, in some embodiments, the DNA polymerase can have been modified to remove 5'-3' exonuclease activity. Sequence-modified derivatives or mutants of DNA polymerase enzymes that can be used include, but are not limited to, mutants that retain at least some of the functional, e.g. DNA polymerase activity of the wild-type sequence. Mutations can affect the activity profile of the enzymes, e.g. enhance or reduce the rate of polymerization, under different reaction conditions, e.g. temperature, template concentration, primer concentration, etc. Mutations or sequence-modifications can also affect the exonuclease activity and/or thermostability of the enzyme.

In some embodiments, PCR amplification can include reactions such as, but not limited to, a strand-displacement amplification reaction, a rolling circle amplification reaction, a ligase chain reaction, a transcription-mediated amplification reaction, an isothermal amplification reaction, and/or a loop-mediated amplification reaction.

In some embodiments, PCR amplification uses a single primer that is complementary to the 3' tag of target DNA fragments. In some embodiments, PCR amplification uses a first and a second primer, where at least a 3' end portion of the first primer is complementary to at least a portion of the 3' tag of the target nucleic acid fragments, and where at least a 3' end portion of the second primer exhibits the sequence of at least a portion of the 5' tag of the target nucleic acid fragments. In some embodiments, a 5' end portion of the first primer is non-complementary to the 3' tag of the target nucleic acid fragments, and a 5' end portion of the second primer does not exhibit the sequence of at least a portion of the 5' tag of the target nucleic acid fragments. In some embodiments, the first primer includes a first universal sequence and/or the second primer includes a second universal sequence.

In some embodiments (e.g., when the PCR amplification amplifies captured DNA), the PCR amplification products can be ligated to additional sequences using a DNA ligase enzyme. The DNA ligase activity can be provided by one or more distinct DNA ligase enzymes. In some embodiments, the DNA ligase enzyme is from a bacterium, e.g., the DNA ligase enzyme is a bacterial DNA ligase enzyme. In some embodiments, the DNA ligase enzyme is from a virus (e.g., a bacteriophage). For instance, the DNA ligase can be T4 DNA ligase. Other enzymes appropriate for the ligation step include, but are not limited to, Tth DNA ligase, Taq DNA ligase, *Thermococcus sp.* (strain 9oN) DNA ligase (9oNTM DNA ligase, available from New England Biolabs, Ipswich, MA), and Ampligase^{™} (available from Epicentre Biotechnologies, Madison, WI). Derivatives, e.g. sequence-modified derivatives, and/or mutants thereof, can also be used.

In some embodiments, genetic material is amplified by reverse transcription polymerase chain reaction (RT-PCR). The desired reverse transcriptase activity can be provided by one or more distinct reverse transcriptase enzymes, suitable examples of which include, but are not limited to: M-MLV, MuLV, AMV, HIV, ArrayScript^{™}, MultiScribe^{™}, ThermoScript^{™}, and SuperScript^{®} I, II, III, and IV enzymes. "Reverse transcriptase" includes not only naturally occurring enzymes, but all such modified derivatives thereof, including also derivatives of naturally-occurring reverse transcriptase enzymes.

In addition, reverse transcription can be performed using sequence-modified derivatives or mutants of M-MLV, MuLV, AMV, and HIV reverse transcriptase enzymes, including mutants that retain at least some of the functional, e.g. reverse transcriptase, activity of the wild-type sequence. The reverse transcriptase enzyme can be provided as part of a composition that includes other components, e.g. stabilizing components that enhance or improve the activity of the reverse transcriptase enzyme, such as RNase inhibitor(s), inhibitors of DNA-dependent DNA synthesis, e.g. actinomycin D. Many sequence-modified derivative or mutants of reverse transcriptase enzymes, e.g. M-MLV, and compositions including unmodified and modified enzymes are commercially available, e.g. ArrayScript^{™}, Multi Scribe^{™}, ThermoScript^{™}, and SuperScript^{®} I, II, III, and IV enzymes.

Certain reverse transcriptase enzymes (e.g. Avian Myeloblastosis Virus (AMV) Reverse Transcriptase and Moloney Murine Leukemia Virus (M-MuLV, MMLV) Reverse Transcriptase) can synthesize a complementary DNA strand using both RNA (cDNA synthesis) and single-stranded DNA (ssDNA) as a template. Thus, in some embodiments, the reverse transcription reaction can use an enzyme (reverse transcriptase) that is capable of using both RNA and ssDNA as the template for an extension reaction, e.g. an AMV or MMLV reverse transcriptase.

In some embodiments, the quantification of RNA and/or DNA is carried out by real-time PCR (also known as quantitative PCR or qPCR), using techniques well known in the art, such as but not limited to "TAQMAN^{™}" or "SYBR^{®}", or on capillaries ("LightCycler^{®} Capillaries"). In some embodiments, the quantification of genetic material is determined by optical absorbance and with real-time PCR. In some embodiments, the quantification of genetic material is determined by digital PCR. In some embodiments, the genes analyzed can be compared to a reference nucleic acid extract (DNA and RNA) corresponding to the expression (mRNA) and quantity (DNA) in order to compare expression levels of the target nucleic acids.

### (x) Antibody

An "antibody" is a polypeptide molecule that recognizes and binds to a complementary target antigen. Antibodies typically have a molecular structure shape that resembles a Y shape. Naturally-occurring antibodies, referred to as immunoglobulins, belong to one of the immunoglobulin classes IgG, IgM, IgA, IgD, and IgE. Antibodies can also be produced synthetically. For example, recombinant antibodies, which are monoclonal antibodies, can be synthesized using synthetic genes by recovering the antibody genes from source cells, amplifying into an appropriate vector, and introducing the vector into a host to cause the host to express the recombinant antibody. In general, recombinant antibodies can be cloned from any species of antibody-producing animal using suitable oligonucleotide primers and/or hybridization probes. Recombinant techniques can be used to generate antibodies and antibody fragments, including non-endogenous species.

Synthetic antibodies can be derived from non-immunoglobulin sources. For example, antibodies can be generated from nucleic acids (e.g., aptamers), and from non-immunoglobulin protein scaffolds (such as peptide aptamers) into which hypervariable loops are inserted to form antigen binding sites. Synthetic antibodies based on nucleic acids or peptide structures can be smaller than immunoglobulin-derived antibodies, leading to greater tissue penetration.

Antibodies can also include affimer proteins, which are affinity reagents that typically have a molecular weight of about 12-14 kDa. Affimer proteins generally bind to a target (e.g., a target protein) with both high affinity and specificity. Examples of such targets include, but are not limited to, ubiquitin chains, immunoglobulins, and C-reactive protein. In some embodiments, affimer proteins are derived from cysteine protease inhibitors, and include peptide loops and a variable N-terminal sequence that provides the binding site.

Antibodies can also refer to an "epitope binding fragment" or "antibody fragment," which as used herein, generally refers to a portion of a complete antibody capable of binding the same epitope as the complete antibody, albeit not necessarily to the same extent. Although multiple types of epitope binding fragments are possible, an epitope binding fragment typically comprises at least one pair of heavy and light chain variable regions (VH and VL, respectively) held together (e.g., by disulfide bonds) to preserve the antigen binding site, and does not contain all or a portion of the Fc region. Epitope binding fragments of an antibody can be obtained from a given antibody by any suitable technique (e.g., recombinant DNA technology or enzymatic or chemical cleavage of a complete antibody), and typically can be screened for specificity in the same manner in which complete antibodies are screened. In some embodiments, an epitope binding fragment comprises an F(ab')₂ fragment, Fab' fragment, Fab fragment, Fd fragment, or Fv fragment. In some embodiments, the term "antibody" includes antibody-derived polypeptides, such as single chain variable fragments (scFv), diabodies or other multimeric scFvs, heavy chain antibodies, single domain antibodies, or other polypeptides comprising a sufficient portion of an antibody (*e*.*g*., one or more complementarity determining regions (CDRs)) to confer specific antigen binding ability to the polypeptide.

### (xi) Affinity Group

An "affinity group" is a molecule or molecular moiety which has a high affinity or preference for associating or binding with another specific or particular molecule or moiety. The association or binding with another specific or particular molecule or moiety can be via a non-covalent interaction, such as hydrogen bonding, ionic forces, and van der Waals interactions. An affinity group can, for example, be biotin, which has a high affinity or preference to associate or bind to the protein avidin or streptavidin. An affinity group, for example, can also refer to avidin or streptavidin which has an affinity to biotin. Other examples of an affinity group and specific or particular molecule or moiety to which it binds or associates with include, but are not limited to, antibodies or antibody fragments and their respective antigens, such as digoxigenin and anti-digoxigenin antibodies, lectin, and carbohydrates (e.g., a sugar, a monosaccharide, a disaccharide, or a polysaccharide), and receptors and receptor ligands.

Any pair of affinity group and its specific or particular molecule or moiety to which it binds or associates with can have their roles reversed, for example, such that between a first molecule and a second molecule, in a first instance the first molecule is characterized as an affinity group for the second molecule, and in a second instance the second molecule is characterized as an affinity group for the first molecule.

### (xii) Label, Detectable Label, and Optical Label

The terms "detectable label," "optical label," and "label" are used interchangeably herein to refer to a directly or indirectly detectable moiety that is associated with (e.g., conjugated to) a molecule to be detected, e.g., a probe for *in situ* assay, a capture probe or analyte. The detectable label can be directly detectable by itself (e.g., radioisotope labels or fluorescent labels) or, in the case of an enzymatic label, can be indirectly detectable, e.g., by catalyzing chemical alterations of a substrate compound or composition, which substrate compound or composition is directly detectable. Detectable labels can be suitable for small scale detection and/or suitable for high-throughput screening. As such, suitable detectable labels include, but are not limited to, radioisotopes, fluorophores, chemiluminescent compounds, bioluminescent compounds, and dyes.

The detectable label can be qualitatively detected (e.g., optically or spectrally), or it can be quantified. Qualitative detection generally includes a detection method in which the existence or presence of the detectable label is confirmed, whereas quantifiable detection generally includes a detection method having a quantifiable (e.g., numerically reportable) value such as an intensity, duration, polarization, and/or other properties. In some embodiments, the detectable label is bound to a feature or to a capture probe associated with a feature. For example, detectably labeled features can include a fluorescent, a colorimetric, or a chemiluminescent label attached to a bead (see, for example, Rajeswari et al., J. Microbiol Methods 139:22-28, 2017, and Forcucci et al., J. Biomed Opt. 10: 105010, 2015, the entire contents of each of which are incorporated herein by reference).

In some embodiments, a plurality of detectable labels can be attached to a feature, capture probe, or composition to be detected. For example, detectable labels can be incorporated during nucleic acid polymerization or amplification (e.g., Cy5^{®}-labelled nucleotides, such as Cy5^{®}-dCTP). Any suitable detectable label can be used. In some embodiments, the detectable label is a fluorophore. For example, the fluorophore can be from a group that includes: 7-AAD (7-Aminoactinomycin D), Acridine Orange (+DNA), Acridine Orange (+RNA), Alexa Fluor^{®} 350, Alexa Fluor^{®} 430, Alexa Fluor^{®} 488, Alexa Fluor^{®} 532, Alexa Fluor^{®} 546, Alexa Fluor^{®} 555, Alexa Fluor^{®} 568, Alexa Fluor^{®} 594, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, Allophycocyanin (APC), AMCA / AMCA-X, 7-Aminoactinomycin D (7-AAD), 7- Amino-4-methylcoumarin, 6-Aminoquinoline, Aniline Blue, ANS, APC-Cy7, ATTO-TAG^{™} CBQCA, ATTO-TAG^{™} FQ, Auramine O-Feulgen, BCECF (high pH), BFP (Blue Fluorescent Protein), BFP / GFP FRET, BOBO^{™}-1 / BO-PRO^{™}-1, BOBO^{™}-3 / BO-PRO^{™}-3, BODIPY^{®} FL, BODIPY^{®} TMR, BODIPY^{®} TR-X, BODIPY^{®} 530/550, BODIPY^{®} 558/568, BODIPY^{®} 564/570, BODIPY^{®} 581/591, BODIPY^{®} 630/650-X, BODIPY^{®} 650-665-X, BTC, Calcein, Calcein Blue, Calcium Crimson^{™}, Calcium Green-1^{™}, Calcium Orange^{™}, Calcofluor^{®} White, 5-Carboxyfluoroscein (5-FAM), 5-Carboxynaphthofluoroscein, 6-Carboxyrhodamine 6G, 5-Carboxytetramethylrhodamine (5-TAMRA), Carboxy-X-rhodamine (5-ROX), Cascade Blue^{®}, Cascade Yellow^{™}, CCF2 (GeneBLAzer^{™}), CFP (Cyan Fluorescent Protein), CFP / YFP FRET, Chromomycin A3, Cl-NERF (low pH), CPM, 6-CR 6G, CTC Formazan, Cy2^{®}, Cy3^{®}, Cy3.5^{®}, Cy5^{®}, Cy5.5^{®}, Cy7^{®}, Cychrome (PE-Cy5), Dansylamine, Dansyl cadaverine, Dansylchloride, DAPI, Dapoxyl, DCFH, DHR, DiA (4-Di-16-ASP), DiD (DilC18(5)), DIDS, Dil (DilC18(3)), DiO (DiOC18(3)), DiR (DilC18(7)), Di-4 ANEPPS, Di-8 ANEPPS, DM-NERF (4.5-6.5 pH), DsRed (Red Fluorescent Protein), EBFP, ECFP, EGFP, ELF^{®} -97 alcohol, Eosin, Erythrosin, Ethidium bromide, Ethidium homodimer-1 (EthD-1), Europium (III) Chloride, 5-FAM (5-Carboxyfluorescein), Fast Blue, Fluorescein-dT phosphoramidite, FITC, Fluo-3, Fluo-4, FluorX^{®}, Fluoro-Gold^{™} (high pH), Fluoro-Gold^{™} (low pH), Fluoro-Jade, FM^{®} 1-43, Fura-2 (high calcium), Fura-2 / BCECF, Fura Red^{™} (high calcium), Fura Red^{™} / Fluo-3, GeneBLAzer^{™} (CCF2), GFP Red Shifted (rsGFP), GFP Wild Type, GFP / BFP FRET, GFP / DsRed FRET, Hoechst 33342 & 33258, 7-Hydroxy-4-methylcoumarin (pH 9), 1,5 IAEDANS, Indo-1 (high calcium), Indo-1 (low calcium), Indodicarbocyanine, Indotricarbocyanine, JC-1, 6-JOE, JOJO^{™}-1 / JO-PRO^{™}-1, LDS 751 (+DNA), LDS 751 (+RNA), LOLO^{™}-1 / LO-PRO^{™}-1, Lucifer Yellow, LysoSensor^{™} Blue (pH 5), LysoSensor^{™} Green (pH 5), LysoSensor^{™} Yellow/Blue (pH 4.2), LysoTracker^{®} Green, LysoTracker^{®} Red, LysoTracker^{®} Yellow, Mag-Fura-2, Mag-Indo-1, Magnesium Green^{™}, Marina Blue^{®}, 4-Methylumbelliferone, Mithramycin, MitoTracker^{®} Green, MitoTracker^{®} Orange, MitoTracker^{®} Red, NBD (amine), Nile Red, Oregon Green^{®} 488, Oregon Green^{®} 500, Oregon Green^{®} 514, Pacific Blue, PBF1, PE (R-phycoerythrin), PE-Cy5, PE-Cy7, PE-Texas Red, PerCP (Peridinin chlorphyll protein), PerCP-Cy5.5 (TruRed), PharRed (APC-Cy7), C-phycocyanin, R-phycocyanin, R-phycoerythrin (PE), PI (Propidium Iodide), PKH26, PKH67, POPO^{™}-1 / PO-PRO^{™}-1, POPO^{™}-3 / PO-PRO^{™}-3, Propidium Iodide (PI), PyMPO, Pyrene, Pyronin Y, Quantam Red (PE-Cy5), Quinacrine Mustard, R670 (PE-Cy5), Red 613 (PE-Texas Red) , Red Fluorescent Protein (DsRed), Resorufin, RH 414, Rhod-2, Rhodamine B, Rhodamine Green^{™}, Rhodamine Red^{™}, Rhodamine Phalloidin, Rhodamine 110, Rhodamine 123, 5-ROX (carboxy-X-rhodamine), S65A, S65C, S65L, S65T, SBFI, SITS, SNAFL^{®}-1 (high pH), SNAFL^{®}-2, SNARF^{®}-1 (high pH), SNARF^{®}-1 (low pH), Sodium Green^{™}, SpectrumAqua^{®}, SpectrumGreen^{®} #1, SpectrumGreen^{®} #2, SpectrumOrange^{®}, SpectrumRed^{®}, SYTO^{®} 11, SYTO^{®} 13, SYTO^{®} 17, SYTO^{®} 45, SYTOX^{®} Blue, SYTOX^{®} Green, SYTOX^{®} Orange, 5-TAMRA (5-Carboxytetramethylrhodamine), Tetramethylrhodamine (TRITC), Texas Red^{®} / Texas Red^{®}-X, Texas Red^{®}-X (NHS Ester), Thiadicarbocyanine, Thiazole Orange, TOTO^{®}-1 / TO-PRO^{®}-1, TOTO^{®}-3 / TO-PRO^{®}-3, TO-PRO^{®}-5, Tri-color (PE-Cy5), TRITC (Tetramethylrhodamine), TruRed (PerCP-Cy5.5), WW 781, X-Rhodamine (XRITC), Y66F, Y66H, Y66W, YFP (Yellow Fluorescent Protein), YOYO^{®}-1 / YO-PRO^{®}-1, YOYO^{®}-3 / YO-PRO^{®}-3, 6-FAM (Fluorescein), 6-FAM (NHS Ester), 6-FAM (Azide), HEX, TAMRA (NHS Ester), Yakima Yellow, MAX, TET, TEX615, ATTO 488, ATTO 532, ATTO 550, ATTO 565, ATTO Rho101, ATTO 590, ATTO 633, ATTO 647N, TYE 563, TYE 665, TYE 705, 5' IRDye^{®} 700, 5' IRDye^{®} 800, 5' IRDye^{®} 800CW (NHS Ester), WellRED D4 Dye, WellRED D3 Dye, WellRED D2 Dye, Lightcycler^{®} 640 (NHS Ester), and Dy 750 (NHS Ester).

As mentioned above, in some embodiments, a detectable label is or includes a luminescent or chemiluminescent moiety. Common luminescent/chemiluminescent moieties include, but are not limited to, peroxidases such as horseradish peroxidase (HRP), soybean peroxidase (SP), alkaline phosphatase, and luciferase. These protein moieties can catalyze chemiluminescent reactions given the appropriate substrates (e.g., an oxidizing reagent plus a chemiluminescent compound. A number of compound families are known to provide chemiluminescence under a variety of conditions. Non-limiting examples of chemiluminescent compound families include 2,3-dihydro-l,4-phthalazinedione luminol, 5-amino-6,7,8-trimethoxy- and the dimethylamino[ca]benz analog. These compounds can luminesce in the presence of alkaline hydrogen peroxide or calcium hypochlorite and base. Other examples of chemiluminescent compound families include, e.g., 2,4,5-triphenylimidazoles, para-dimethylamino and - methoxy substituents, oxalates such as oxalyl active esters, p-nitrophenyl, N-alkyl acridinum esters, luciferins, lucigenins, or acridinium esters. In some embodiments, a detectable label is or includes a metal-based or mass-based label. For example, small cluster metal ions, metals, or semiconductors may act as a mass code. In some examples, the metals can be selected from Groups 3-15 of the periodic table, e.g., Y, La, Ag, Au, Pt, Ni, Pd, Rh, Ir, Co, Cu, Bi, or a combination thereof.

### EXAMPLES

The following examples are included for illustrative purposes only and are not intended to limit the scope of the present disclosure.

### Example 1: Uncharged nucleic acid probes increase in situ detection signals

This example demonstrates a method of increasing the number of accessible sites for detection probe (e.g., detection oligonucleotide) hybridization during *in situ* analysis, therefore increasing *in situ* detection sensitivity. In particular, this example demonstrates the use of uncharged detection probes comprising a morpholino backbone **(****FIG. 1B****)** directly hybridizing to a target sequence, to overcome the electrostatic effects and/or other barriers related to the physical chemistry of amplicons generated during *in situ* sequencing.

In some embodiments, *in situ* analysis is conducted, producing various rolling circle amplicon (RCA) products, e.g., DNA concatemers, during the amplification step. The DNA concatemers are generated by hybridizing padlock probes (e.g., primary probes) to target nucleic acids in the sample, ligating the padlock probes, and using the circularized padlock probe as template for amplification. Such DNA concatemers may have a large negative charge, due to the negative phosphate charges along the DNA concatemer backbone. The negatively charged DNA concatemer backbone may induce "swelling" of the amplicon structure through internal coulombic forces, while still presenting an anionic charge field sufficient to inhibit detection probe hybridization towards the center of the DNA concatemer **(****FIG. 2A****).** In some examples, wherein a standard detection probe (e.g., a fluorescent DNA detection oligonucleotide) is used to detect DNA concatemers, the charge field (e.g., as shown in **FIG. 2A****)** may prevent a significant number of detection probes from penetrating the RCA due to electrostatic repulsion.

In some embodiments, the method provided herein comprises using morpholino detection probes that directly hybridize to target sequences within DNA concatemers (e.g., amplicons from RCA) produced during *in situ* analysis. Morpholinos are a class of nucleic acid analog that have methylenemorpholine rings in their backbones, which replace the deoxyribose (or ribose) rings characteristic of DNA and RNA oligonucleotides (FIG. 1A). Morpholinos contain uncharged phosphorodiamidate inter-subunit linkages instead of the anionic phosphodiester linkage found in natural nucleic acids. The morpholine rings carry A, C, G or T bases suitably arranged for Watson-Crick base pairing and are commercially available **(****FIG. 1B**).

When a fluorescent morpholino detection probe directly hybridizes to a target sequence within a DNA concatemers (e.g., amplicons from RCA), many more detection probes may dock on and within the DNA concatemer compared to reference probes not comprising morpholinos, as morpholinos are insensitive to electrostatic effects (FIG. 2B). Therefore, morpholinos may increase the brightness of DNA concatemers, and ultimately may increase the detection sensitivity of *in situ* sequencing. In some cases, the use of morpholino detection probes also decreases optical crowding that may be observed when using reference probes not comprising morpholinos.

In some examples, only a single padlock probe/RNA comprising an uncharged backbone (e.g., a morpholino backbone) may be required for efficient *in situ* detection. As shown in **FIG. 4****,** this experimental design can be used to determine the positional location of various individual isoforms of a gene of interest simultaneously (e.g., single probes could be designed to straddle unique exon/exon junctions).

### Example 2: Indirect hybridization using uncharged nucleic acid probes to increase in situ detection signals

This example demonstrates the use of uncharged detection probes comprising a morpholino backbone **(****FIG. 1B****)** indirectly hybridizing to a target sequence, to overcome the electrostatic effects of amplicons generated during *in situ* sequencing.

*In situ* analysis is conducted, producing various rolling circle amplicon (RCA) products, e.g., DNA concatemers, during the amplification step. The DNA concatemers are generated by hybridizing padlock probes (e.g., primary probes) to target nucleic acids in the sample, ligating the padlock probes, and using the circularized padlock probe as template for amplification. In some embodiments, an intermediate probe directly hybridizes to a target sequence within the RCA product **(****FIG. 3**). The intermediate probe has a region for hybridizing to a target sequence within the RCA product and an overhang region for hybridizing to a fluorescently labeled detection probe. The fluorescently labeled detection probe hybridizes to the sequence of the intermediate probe, e.g., indirectly hybridizes to the target sequence, forming a detection complex. In some embodiments, the fluorescently labeled detection probe comprises a morpholino backbone. In some embodiments, the fluorescently labeled detection probe and the intermediate probe comprise a morpholino backbone. Once the fluorescently labeled detection probe is hybridized to the intermediate probe, the target may be detected.

### Example 3: Morpholino probes increase in situ detection signals

This example demonstrates the use of uncharged detection probes comprising a morpholino backbone (an exemplary structure shown in **FIG. 1B****)** to increase the number and intensity of *in situ* detection signals compared to DNA detection probes. The DNA concatemers were generated by hybridizing circularizable probes (e.g., padlock probes) to target nucleic acids in cells in wells on a plate, ligating the circularizable probes, and using the circularized probe as template for amplification.

Padlock probes designed to target ActB were added to the sample of fixed and permeabilized cultured cells (3T3). The padlock probes were ligated to generate circularized probes and the sample was then incubated with a rolling circle amplification (RCA) mixture containing a Phi29 DNA polymerase and dNTPs for RCA of the circularized probes to generate DNA concatemers.

The DNA concatemers were detected *in situ* in cells on the plate. Morpholino or DNA control detection probes of the same length (20 base pairs) and targeting the same sequence of the DNA concatemers and comprising the same 3' 6-FAM (6-Carboxyfluorescein) fluorophore were allowed to hybridize the DNA concatemers in water or a hybridization buffer using a standard hybridization protocol. The hybridization buffer comprised 1x tetramethylammonium chloride (TMAC). Nine images were taken per well to detect the *in situ* signals of the DNA concatemers hybridized with either the morpholino or DNA control detection probes.

As shown in the left panel of **FIG. 5****,** more DNA concatemers (e.g., "spots") were detected using the morpholino detection probes compared to the DNA control detection probes in the TMAC hybridization buffer. The morpholino detection probes also generated increased detection signal intensity above background (e.g., appear brighter) compared to the DNA control detection probes in the TMAC hybridization buffer **(****FIG. 5****,** right panel). These data indicate that detection probes comprising a morpholino backbone can successfully detect DNA concatemers, and the *in situ* detection signals produced using such probes are more intense than the DNA counterparts.

The present disclosure is not intended to be limited in scope to the particular disclosed embodiments, which are provided, for example, to illustrate various aspects of the present disclosure. Various modifications to the compositions and methods described will become apparent from the description and teachings herein. Such variations may be practiced without departing from the true scope and spirit of the disclosure and are intended to fall within the scope of the present disclosure.

### NUMBERED EMBODIMENTS

1. A method of analyzing a biological sample, comprising:
   binding a plurality of detection probes directly or indirectly to a DNA concatemer in the biological sample, wherein a detection probe of the plurality of detection probes comprises a sequence of nucleobases on a synthetic backbone, and the detection probe directly or indirectly binds to a target sequence in the DNA concatemer, and
   detecting signals from said plurality of bound detection probes, where the signal generated is greater than a plurality of reference detection probes having the same sequence of nucleobases on a sugar-phosphate backbone.
2. The method of embodiment 1, wherein the detection probe is less electrostatically negative than the reference detection probe.
3. The method of embodiment 1 or 2, wherein the detection probe is directly or indirectly coupled to a detectable label, optionally a fluorescent label.
4. The method of any one of embodiments 1-3, wherein the detection probe directly hybridizes to the target sequence.
5. The method of any one of embodiments 1-3, wherein the detection probe hybridizes to an intermediate probe which directly or indirectly binds to the target sequence.
6. The method of embodiment 5, wherein the detection probe directly hybridizes to an intermediate probe that directly hybridizes to the target sequence.
7. The method of any one of embodiments 1-6, wherein the detection probe comprises a single-stranded region and optionally a double-stranded region.
8. The method of any one of embodiments 1-7, wherein the sequence of nucleobases on the synthetic backbone is a first probe sequence, and the detection probe further comprises a second probe sequence comprising nucleobases on a sugar-phosphate backbone.
9. The method of embodiment 8, wherein the first probe sequence is 5' or 3' to the second probe sequence.
10. The method of embodiment 8 or 9, wherein the detection probe comprises one or more first probe sequences and/or one or more second probe sequences.
11. The method of any one of embodiments 8-10, wherein the second probe sequence is a DNA sequence, an RNA sequence, or a combination thereof.
12. The method of any one of embodiments 1-11, wherein the detection probe comprises nucleobases A, T, C, G, or U, or variants thereof, in any suitable order and combination.
13. The method of any one of embodiments 1-12, wherein the detection probe is between about 5 and about 50 nucleobases in length.
14. The method of any one of embodiments 1-13, wherein the detection probe comprises phosphorodiamidate linkages in the synthetic backbone.
15. The method of any one of embodiments 1-14, wherein the detection probe comprises morpholine rings in the synthetic backbone.
16. The method of any one of embodiments 1-15, wherein the detection probe is a morpholino.
17. The method of any one of embodiments 1-16, wherein the detection probe is uncharged or positively charged.
18. The method of any one of embodiments 1-17, wherein the DNA concatemer comprises one or more barcode sequences, optionally wherein the target sequence comprises a barcode sequence corresponding to an analyte in the biological sample.
19. The method of any one of embodiments 1-18, wherein the DNA concatemer is an extension product, a replication product, a reverse transcription product, and/or an amplification product of an endogenous nucleic acid molecule in the biological sample.
20. The method of any one of embodiments 1-18, wherein the DNA concatemer is an extension product, a replication product, a reverse transcription product, and/or an amplification product of a labelling agent that directly or indirectly binds to an analyte in the biological sample.
21. The method of embodiment 20, wherein the labelling agent comprises a reporter oligonucleotide, optionally wherein the reporter oligonucleotide comprises one or more barcode sequences and the DNA concatemer comprises one or a plurality of copies of the one or more barcode sequences.
22. The method of any one of embodiments 1-21, wherein the DNA concatemer is a rolling circle amplification (RCA) product of a circular or circularizable probe or probe set that hybridizes to a DNA or RNA molecule in the biological sample.
23. The method of embodiment 22, wherein DNA concatemers generated from a plurality of different mRNA and/or cDNA molecules are analyzed, a barcode sequence in a particular circular or circularizable probe or probe set uniquely corresponds to a particular mRNA or cDNA molecule, and the particular circular or circularizable probe or probe set further comprises an anchor sequence that is common among circular or circularizable probes or probe sets for a subset of the plurality of different mRNA and/or cDNA molecules.
24. The method of any of embodiments 20-23, wherein the labelling agent comprises a binding moiety that directly or indirectly binds to a non-nucleic acid analyte in the biological sample, and the reporter oligonucleotide in the labelling agent identifies the binding moiety and/or the non-nucleic acid analyte.
25. The method of embodiment 24, wherein the binding moiety of the labelling agent comprises an antibody or antigen binding fragment thereof that directly or indirectly binds to a protein analyte, and the DNA concatemer is a rolling circle amplification (RCA) product of a circular or circularizable probe or probe set that hybridizes to a reporter oligonucleotide of the labelling agent.
26. The method of any one of embodiments 1-25, wherein the DNA concatemer is in the form of a nanoball having a diameter of between about 0.1 µm and about 3 µm, e.g., between about 0.1 µm and about 0.5 µm, between about 0.5 µm and about 1 µm, between about 0.8 µm and about 1.3 µm, or between about 1 µm and about 1.5 µm.
27. The method of any one of embodiments 1-26, wherein the DNA concatemer is between about 1 and about 15 kilobases, between about 15 and about 25 kilobases, between about 25 and about 35 kilobases, between about 35 and about 45 kilobases, between about 45 and about 55 kilobases, between about 55 and about 65 kilobases, between about 65 and about 75 kilobases, or more than 75 kilobases in length.
28. The method of any one of embodiments 1-27, wherein the DNA concatemer comprises between about 10 and about 100, between about 100 and about 1,000, between about 1,000 and about 5,000, between about 5,000 and about 10,000, or more than 10,000 copies of the target sequence.
29. The method of embodiment 28, wherein at least about 10%, at least about 25%, at least about 50%, at least about 75%, or at least about 90% of the copies of the target sequence in the DNA concatemer are bound directly or indirectly to one or more molecules of the plurality of detection probes.
30. The method of any one of embodiments 1-29, wherein the DNA concatemer is generated *in situ.*
31. The method of any one of embodiment 1-30, wherein the DNA concatemer is immobilized in the biological sample.
32. The method of any one of embodiment 1-31, wherein the DNA concatemer is crosslinked to one or more other molecules in the biological sample.
33. The method of any one of embodiment 1-32, wherein a complex comprising multiple molecules of the detection probe is detected *in situ* in the biological sample.
34. The method of embodiment 33, wherein the method comprises imaging the biological sample to detect the complex, optionally using fluorescent microscopy.
35. The method of any one of embodiment 1-34, wherein a sequence of the DNA concatemer molecule is analyzed *in situ* in the biological sample.
36. The method of any one of embodiment 1-35, wherein the detection probe is for analyzing a sequence of the DNA concatemer by sequential hybridization, sequencing by hybridization, sequencing by ligation, sequencing by synthesis, sequencing by binding, or a combination thereof.
37. The method of embodiment 35 or 36, wherein the sequence to be analyzed comprises a barcode sequence corresponding to an analyte or a portion thereof in the biological sample.
38. The method of any one of embodiments 1-37, further comprising removing one or more molecules of the detection probe that have not bound to the target sequence.
39. The method of any one of embodiments 1-33, wherein the detection probe of the plurality of detection probes is a first detection probe and the method further comprises removing molecules of the first detection probe bound to the target sequence, prior to contacting the biological sample with a plurality of second detection probes comprising a sequence of nucleobases on a synthetic backbone.
40. The method of embodiment 39, wherein the target sequence is a first target sequence, and a second detection probe of the plurality of second detection probes directly or indirectly binds to a second target sequence in the DNA concatemer, wherein the first and second target sequences are the same or different.
41. The method of any one of embodiments 1-33, wherein the detection probe is a first detection probe and hybridizes to a first intermediate probe which hybridizes to the target sequence, optionally wherein the first intermediate probe comprises a sequence of nucleobases on a synthetic backbone.
42. The method of embodiment 41, wherein the method further comprises removing molecules of the first detection probe and/or the first intermediate probe bound to the target sequence.
43. The method of embodiment 41 or 42, wherein the method further comprises contacting the biological sample with a plurality of second detection probes comprising a sequence of nucleobases on a synthetic backbone and a second intermediate probe, wherein the plurality of second detection probes hybridize to the second intermediate probe which hybridizes to the target sequence, optionally wherein the first intermediate probe comprises a sequence of nucleobases on a synthetic backbone.
44. The method of any one of embodiments 1-43, wherein the biological sample is a processed or cleared biological sample.
45. The method of any one of embodiments 1-44, wherein the biological sample is a tissue sample.
46. The method of embodiment 45, wherein the tissue sample is a tissue slice between about 1 µm and about 50 µm in thickness, optionally wherein the tissue slice is between about 5 µm and about 35 µm in thickness.
47. The method of embodiment 45 or 46, wherein the tissue sample is embedded in a hydrogel.
48. The method of embodiment 47, wherein the analyte or the DNA concatemer is crosslinked to the hydrogel.
49. The method of embodiment 48, wherein the analyte or the DNA concatemer comprises one or more functional groups for attachment to the hydrogel.
50. The method of embodiment 48 or 49, wherein the analytes comprise biomolecules and/or products thereof from a tissue sample.
51. A method of analyzing a biological sample, comprising:
   (a) contacting a biological sample with a plurality of fluorescently labeled detection probes, wherein:
      the biological sample comprises a rolling circle amplification (RCA) product immobilized therein, wherein the RCA product comprises multiple copies of a target sequence,
      a fluorescently labeled detection probe of the plurality of fluorescently labeled detection probes comprises a sequence of nucleobases on a synthetic backbone which hybridizes to the target sequence or an intermediate probe that hybridizes to the target sequence,
   (b) optionally removing molecules of the fluorescently labeled detection probe and/or the intermediate probe that have not hybridized to the RCA product; and
   (c) detecting molecules of the fluorescently labeled detection probe bound to the RCA product in the biological sample,
   wherein the signals generated by the fluorescently labeled detection probes hybridized to the RCA product is greater than a plurality of reference detection probes having the same fluorescent label and sequence of nucleobases on a sugar-phosphate backbone.
52. The method of embodiment 51, further comprising prior to step (a):
   contacting the biological sample with a circular or circularizable probe or probe set that directly or indirectly binds to an analyte in the biological sample, and
   generating the RCA product using the circular or circularizable probe or probe set as template.
53. The method of embodiment 51 or 52, wherein the fluorescently labeled detection probe is uncharged or less electrostatically negative than a reference detection probe having the same fluorescent label and sequence of nucleobases on a sugar-phosphate backbone.
54. The method of any one of embodiments 52-53, wherein the circular or circularizable probe or probe set hybridizes to a nucleic acid sequence of the analyte.
55. The method of any one of embodiments 52-53, wherein the circular or circularizable probe or probe set hybridizes to a reporter oligonucleotide of a labelling agent, wherein the labelling agent further comprises a binding moiety that directly or indirectly binds to the analyte, wherein the reporter oligonucleotide corresponds to the binding moiety and/or the analyte.
56. The method of any one of embodiments 51-55, wherein the intermediate probe comprises a sequence of nucleobases on a synthetic backbone which hybridizes to the target sequence, optionally wherein there are more molecules of the intermediate probe hybridized in the interior of the RCA product than a reference intermediate probe having the same sequence of nucleobases on a sugar-phosphate backbone.
57. The method of any one of embodiments 51-56, wherein the fluorescently labeled detection probe and/or the intermediate probe are morpholino.
58. The method of any one of embodiments 51-57, wherein the RCA product has a diameter of less than about 1.5 µm.
59. The method of any one of embodiments 51-58, wherein in the contacting step, the number of molecules of the fluorescently labeled detection probe is no more than 5, no more than 4, no more than 3, no more than 2, or no more than 1.5 times the number of copies of the target sequence in the RCA product.
60. The method of any one of embodiments 51-59, wherein at least about 10%, at least about 25%, at least about 50%, at least about 75%, or at least about 90% of the copies of the target sequence in the RCA product are bound directly or indirectly to one or more molecules of the intermediate probe and/or the fluorescently labeled detection probe.
61. The method of any one of embodiments 51-60, wherein the biological sample is contacted with multiple molecules of the reference detection probe, and more molecules of the fluorescently labeled detection probe than the reference detection probe bind to the RCA product in the biological sample.
62. The method of any one of embodiments 51-61, further comprising:
   removing molecules of the fluorescently labeled detection probe and the intermediate probe bound to the RCA product in the biological sample.
63. The method of any one of embodiments 51-62, wherein the fluorescently labeled detection probe is a first fluorescently labeled detection probe and the intermediate probe is a first intermediate probe, and the method further comprises contacting the biological sample with a second fluorescently labeled detection probe, wherein:
   the second fluorescently labeled detection probe comprises a sequence of nucleobases on a synthetic backbone which hybridizes to a second intermediate probe that hybridizes to the target sequence, and
   molecules of the second fluorescently labeled detection probe bound to the RCA product in the biological sample are detected.
64. The method of embodiment 63, wherein the second intermediate probe comprises a sequence of nucleobases on a synthetic backbone which hybridizes to the target sequence, optionally wherein there are more molecules of the second intermediate probe hybridized in the interior of the RCA product than a reference intermediate probe having the same sequence of nucleobases on a sugar-phosphate backbone.
65. A composition, comprising:
   a DNA concatemer comprising multiple copies of a target sequence,
   a plurality of intermediate probes that hybridize to the target sequence, and
   a plurality of fluorescently labeled detection probes each comprising a sequence of nucleobases on a synthetic backbone,
   wherein the DNA concatemer, at least one molecule of the intermediate probe and at least one molecule of the fluorescently labeled detection probe form a hybridization complex, optionally wherein the hybridization complex comprises multiple molecules of the intermediate probe and multiple molecules of the fluorescently labeled detection probe.
66. The composition of embodiment 65, wherein the DNA concatemer is a rolling circle amplification (RCA) product.
67. The composition of embodiment 66, wherein the RCA product is generated *in situ* in a biological sample, using a circular or circularizable probe or probe set that directly or indirectly binds to an analyte in the biological sample as template.
68. The composition of any one of embodiments 65-67, wherein the fluorescently labeled detection probe is uncharged or less electrostatically negative than a reference detection probe having the same fluorescent label and sequence of nucleobases on a sugar-phosphate backbone.
69. The composition of any one of embodiments 65-68, wherein the intermediate probe comprises a sequence of nucleobases on a synthetic backbone which hybridizes to the target sequence, optionally wherein the intermediate probe is uncharged or less electrostatically negative than a reference intermediate probe having the same sequence of nucleobases on a sugar-phosphate backbone.
70. The composition of any one of embodiments 65-69, wherein at least a portion of the fluorescently labeled detection probe hybridizes to the intermediate probe.
71. The composition of any one of embodiments 65-70, wherein the fluorescently labeled detection probe and/or the intermediate probe are morpholino.
72. The composition of any one of embodiments 65-71, wherein the DNA concatemer has a diameter of less than about 1.5 µm.
73. The composition of any one of embodiments 65-72, wherein at least about 10%, at least about 25%, at least about 50%, at least about 75%, or at least about 90% of the copies of the target sequence in the DNA concatemer are bound directly or indirectly to one or more molecules of the intermediate probe and/or the fluorescently labeled detection probe.
74. A kit, comprising:
   a fluorescently labeled detection probe comprising a sequence of nucleobases on a synthetic backbone, wherein at least a portion of the fluorescently labeled detection probe hybridizes to an intermediate probe, and
   the intermediate probe which comprises a sequence of nucleobases on a synthetic backbone that hybridizes to a target sequence.
75. The kit of embodiment 74, wherein the fluorescently labeled detection probe and the intermediate probe are morpholino.
76. The kit of embodiment 74 or 75, further comprising a circular or circularizable probe or probe set comprising the target sequence or a complement thereof, wherein the probe or probe set is capable of directly or indirectly binding to an analyte in a biological sample and generating a rolling circle amplification product using the probe or probe set as template.
78. The method of any one of embodiments 1-64, wherein the binding of the plurality of detection probes occurs in the presence of a hybridization buffer.
79. The method of embodiment 78, wherein the hybridization buffer comprises a reagent that increases the stability of A:T base pairs to approximately that of G:C pairs.
80. The method of embodiment 79, wherein the reagent comprises ammonium ions.
81. The method of embodiment 80, wherein the reagent is tetramethylammonium chloride.

## Claims

1. A method of analyzing a biological sample, comprising:
binding a plurality of detection probes directly to a DNA concatemer in the biological sample, wherein:
a detection probe of the plurality of detection probes comprises a sequence of nucleobases on a synthetic backbone;
the detection probe comprises morpholine rings in the synthetic backbone; and
the detection probe directly binds to a target sequence in the DNA concatemer; and
detecting signals from said plurality of bound detection probes, wherein the signal generated is greater than a plurality of reference detection probes having the same sequence of nucleobases on a sugar-phosphate backbone.

2. The method of claim 1, wherein the detection probe is less electrostatically negative than the reference detection probe.

3. The method of claim 2 or 3, wherein the sequence of nucleobases on the synthetic backbone is a first probe sequence, and the detection probe further comprises a second probe sequence comprising nucleobases on a sugar-phosphate backbone.

4. The method of any one of claims 1-3, wherein the detection probe is a morpholino.

5. The method of any one of claims 1-4, wherein the DNA concatemer comprises one or more barcode sequences, optionally wherein the target sequence comprises a barcode sequence corresponding to an analyte in the biological sample.

6. The method of any one of claims 1-5, wherein the DNA concatemer is a rolling circle amplification (RCA) product of a circular or circularizable probe or probe set that hybridizes to a DNA or RNA molecule in the biological sample.

7. The method of claim 6, wherein DNA concatemers generated from a plurality of different mRNA and/or cDNA molecules are analyzed.

8. The method of any one of claims 1-7, wherein the DNA concatemer is generated *in situ.*

9. The method of any one of claims 1-8, further comprising imaging the biological sample to detect a complex comprising multiple molecules of the detection probe *in situ* in the biological sample.

10. The method of any one of claims 1-9, wherein the biological sample is a cell or tissue sample.

11. The method of any one of claims 1-10, wherein the binding of the plurality of detection probes occurs in the presence of a hybridization buffer, wherein the hybridization buffer comprises a reagent that increases the stability of A:T base pairs to approximately that of G:C pairs.

12. The method of claim 11, wherein the reagent comprises ammonium ions.

13. The method of claim 12, wherein the reagent is tetramethylammonium chloride (TMAC).

14. The method of any one of claims 11-13, wherein the hybridization buffer comprises between about 0.1 M and about 2 M TMAC.

15. The method of claim 14, wherein the hybridization buffer comprises 1 M TMAC.
